(19) Europäisches Patentamt
European Patent Office
Office européen des brevets

(11) EP 0 633 259 B1

(12) **EUROPÄISCHE PATENTSCHRIFT**

(45) Veröffentlichungstag und Bekanntmachung des Hinweises auf die Patenterteilung: **13.01.1999 Patentblatt 1999/02**

(21) Anmeldenummer: **94109257.9**

(22) Anmeldetag: **16.06.1994**

(51) Int. Cl.[6]: **C07D 401/12**, C07D 405/14, C07D 405/12, C07D 409/14, C07D 403/12, C07D 413/12, C07D 239/52, A61K 31/505

(54) **Sulfonylaminopyrimidine, ihre Herstellung und Verwendung als Heilmittel**

Sulfonylaminopyrimidines, their preparation and their use as medicine

Sulfonylaminopyrimidines, leur préparation et leur usage comme médicament

(84) Benannte Vertragsstaaten:
**AT BE CH DE DK ES FR GB GR IE IT LI LU MC NL PT SE**
Benannte Erstreckungsstaaten:
**SI**

(30) Priorität: **28.06.1993 CH 1924/93**

(43) Veröffentlichungstag der Anmeldung: **11.01.1995 Patentblatt 1995/02**

(73) Patentinhaber:
**F. HOFFMANN-LA ROCHE AG**
**4002 Basel (CH)**

(72) Erfinder:
- **Breu, Volker**
  **D-79418 Schliengen (DE)**
- **Burri, Kaspar**
  **CH-4102 Binningen (CH)**
- **Cassal, Jean-Marie**
  **F-68100 Mulhouse (FR)**
- **Clozel, Martine**
  **F-68300 St. Louis (FR)**
- **Hirth, Georges**
  **F-68330 Huningue (FR)**
- **Löffler, Bernd-Michael**
  **D-79206 Oberrimsingen (DE)**
- **Müller, Marcel**
  **CH-4402 Frenkendorf (CH)**
- **Neidhart, Werner**
  **F-68870 Bartenheim (FR)**
- **Ramuz, Henri**
  **CH-4127 Birsfelden (CH)**

(74) Vertreter: **Mahé, Jean et al**
**F.Hoffmann-La Roche AG**
**Patent Department (PLP),**
**124 Grenzacherstrasse**
**4070 Basel (CH)**

(56) Entgegenhaltungen:
**EP-A- 0 510 526** **EP-A- 0 526 708**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 633 259 B1

Printed by Xerox (UK) Business Services
2.16.7/3.6

## Beschreibung

Die vorliegende Erfindung betrifft neue Sulfonylaminopyrimidine und deren Verwendung als Heilmittel. Insbesondere betrifft die Erfindung neue Sulfonylaminopyrimidine der Formel

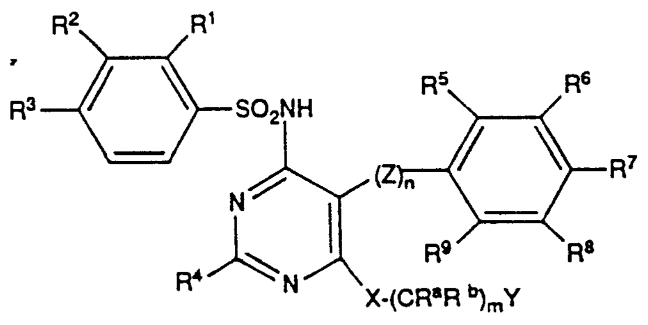

I

worin

| | |
|---|---|
| $R^1$-$R^3$ | unabhängig voneinander Wasserstoff, nieder-Alkyl, nieder-Alkoxy, nieder-Alkylthio, nieder-Alkenyl, Halogen, Trifluormethyl, Hydroxy-nieder-alkoxy, Halogen-nieder-alkoxy, Cyclo-nieder-alkyl, Hydroxyniederalkyl, Aminoniederalkyl, Aminoniederalkoxy, Alkanoylaminoniederalkoxy, Alkanoylaminoniederalkyl, Carboxyniederalkoxy, Carboxyniederalkyl, Niederalkoxycarbonylniederalkyl, Niederalkoxycarbonylniederalkoxy, Alkanoyloxyniederalkoxy, Alkanoyloxyniederalkyl, Alkoxycarbonyl, Carboxy, Amino, mono- oder di-(nieder-Alkyl)amino oder einen Rest $(R^c,R^d)N-C(O)(CH_2)_{0-4}O-$ oder $(R^c,R^d)N-C(O)(CH_2)_{0-4}-$; |
| $R^2$ und $R^3$ | zusammen Butadienyl, Methylendioxy, Aethylendioxy oder Isopropylidendioxy; |
| $R^4$ | Wasserstoff, nieder-Alkyl, Cyclo-nieder-alkyl, Trifluormethyl, nieder-Alkoxy, nieder-Alkinyloxy, nieder-Alkylthio, nieder-Alkylthio-nieder-alkyl, nieder-Alkylthio-nieder-alkoxy, Hydroxy-nieder-alkyl, Hydroxy-nieder-alkoxy, Dihydroxy-nieder-alkoxy, nieder-Alkoxy-nieder-alkyl, Hydroxy-nieder-alkoxy-nieder-alkyl, nieder-Alkoxy-nieder-alkoxy, Di-(nieder-alkoxy)-alkoxy, Hydroxy-nieder-alkoxy-nieder-alkoxy, nieder-Alkylsulfinyl, nieder-Alkylsulfinyl-nieder-alkoxy, nieder-Alkylsulfonyl, 2-Methoxy-3-hydroxypropoxy, 2-Hydroxy-3-phenylpropyl, Amino-nieder-alkyl, nieder-Alkylamino-nieder-alkyl, Di-nieder-alkylamino-nieder-alkyl, Amino, nieder-Alkylamino, Di-nieder-alkylamino, Arylamino, Aryl, Arylthio, Aryloxy, Aryl-nieder-alkyl, Aryl-nieder-alkoxy-nieder-alkyl, Aryl-nieder-alkyl-nieder-alkoxy, Heterocyclyl, Heterocyclyl-nieder-alkyl oder Heterocyclylalkoxy; |
| $R^5$ bis $R^9$ | unabhängig voneinander Wasserstoff, Halogen, Trifluormethyl, nieder-Alkyl, nieder-Alkoxy, nieder-Alkylthio, nieder-Alkylsulfinyl oder nieder-Alkylsulfonyl; |
| $R^6$ | zusammen mit $R^5$ oder $R^7$ Butadienyl, Methylendioxy, Aethylendioxy oder Isopropylidendioxy; |
| $R^a$ und $R^b$ | unabhängig voneinander Wasserstoff, nieder-Alkyl, nieder-Alkoxy oder nieder-Alkylthio; |
| $R^c$ und $R^d$ | unabhängig voneinander Wasserstoff, nieder-Alkyl oder Aryl; oder $R^c$ und $R^d$ zusammen mit dem daran gebundenen N-Atom einen 5-7-gliedrigen heterocyclischen Rest; |
| Y | einen Rest $-OC(O)NR^{10},R^{11}$, $-NHC(O)NR^{10}R^{11}$, $-OC(O)OR^{10}$ oder $-NHC(O)OR^{10}$; |
| $R^{10}$ | nieder-Alkyl, Cyclo-nieder-alkyl, Hydroxy-nieder-alkyl, Carboxy-nieder-alkyl, nieder-Alkoxycarbonyl-nieder-alkyl, nieder-Alkanoyloxy-nieder-alkyl, Aryl, Aryl-nieder-alkyl, Arylcarbamoyl--nieder-alkyl, Heterocyclyl, Heterocyclyl-nieder-alkyl, oder einen Rest $(R^c,R^d)N-C(O)(CH_2)_{1-4}-$; |
| $R^{11}$ | Wasserstoff oder ein Rest $R^{10}$; |
| $R^{10}$ und $R^{11}$ | zusammen mit dem davon gebundenen N-Atom einen 5-7 gliedrigen heterocyclischen Rest; |
| Z | -O-, -S- oder $-CH_2-$; |
| X | -O-, -S- oder -NH-; |
| n | 0 oder 1; und |
| m | 1, 2 oder 3 |

bedeuten,
und pharmazeutisch anwendbare Salze davon.

Der hier verwendete Ausdruck "nieder" bezeichnet Gruppen mit 1-7 C-Atomen, vorzugsweise 1-4 C-Atomen. Alkyl-, Alkoxy- und Alkylthiogruppen sowie Alkylgruppen als Bestandteile von Alkanoylgruppen können geradkettig oder verzweigt sein. Beispiele solcher Alkylgruppen sind Methyl, Aethyl, Propyl, Isopropyl, Butyl, sek. und tert.Butyl. Halogen

bezeichnet Fluor, Chlor, Brom und Jod, wobei Chlor bevorzugt ist. Cycloalkylreste enthalten 3 bis 8 C-Atome wie Cyclopropyl, Cyclobutyl, Cyclopentyl oder Cyclohexyl. Beispiele von Arylresten sind Phenyl und substituierte Phenylreste, wobei als Substituenten insb. Halogen, nieder-Alkyl, nieder-Alkoxy, nieder-Alkylendioxy, Carboxyl und Trifluormethyl in Betracht kommen. Beispiele von Heterocyclylresten sind Reste von mono- oder bicyclischen, 5-, 6- und 7-gliedrigen Heterocyclen mit Sauerstoff, Stickstoff oder Schwefel als Heteroatom, wie 2- und 3-Furyl, 2-, 4- und 5-Pyrimidinyl, 2-, 3- und 4-Pyridyl und Pyridyl-N-oxid, 1,2- und 1,4-Diazinyl, Morpholino, Thiomorpholino, Thiomorpholino-4,4-dioxid, 2,2-Dimethyl-1,3-dioxolanyl, 2-und 3-Thienyl, Isoxazolyl, Oxazolyl, Thiazolyl, Imidazolyl, Pyrrolyl, Pyrrolidinyl, Piperidinyl, Azepanyl, Benzofuranyl, Benzothienyl, Indolyl, Purinyl, Chinolyl, Isochinolyl und Chinazolyl, welche Reste substituiert sein können, z.B. durch 1 oder 2 nieder-Alkyl- oder nieder-Alkoxygruppen oder Halogenatome.

Eine bevorzugte Gruppe von Verbindungen der Formel I sind die, in denen n = 1 ist und von diesen sind solche bevorzugt, in denen Z = -O- ist. X ist vorzugsweise -O-. Y ist vorzugsweise $-OC(O)NR^{10}R^{11}$. m ist vorzugsweise 2. Bevorzugte Reste $R^1$-$R^3$ sind Wasserstoff, nieder-Alkyl, Cyclo-nieder-alkyl, nieder-Alkylthio, nieder-Alkoxy, nieder-Alkenyl, Alkanoyloxy-nieder-alkoxy, Hydroxy-nieder-alkoxy, nieder-Alkoxy-carbonyl-nieder-alkoxy, Halogen-nieder-alkoxy, di-(nieder-Alkyl)amino, oder die Reste $(R^c,R^d)N-C(O)(CH_2)_{0-4}O-$ und $(R^c,R^d)N-C(O)(CH_2)_{0-4}-$, in denen $R^c$ und $R^d$ zusammen einen Piperidino- oder Morpholinorest bilden, darstellen. Weiterhin bevorzugt sind solche Verbindungen, in denen $R^2$ und $R^3$ zusammen Methylendioxy darstellen.

Bevorzugte Reste $R^4$ sind Wasserstoff, nieder-Alkyl, nieder-Alkylthio, Phenyl-nieder-alkoxy-nieder-alkyl, Cyclo-nieder-alkyl, nieder-Alkoxy-nieder-alkyl; Phenyl, nieder-Alkoxyphenyl, Thienyl, Pyrimidinyl und Morpholino.

Bevorzugte Reste $R^5$-$R^9$ sind Wasserstoff, Halogen und nieder-Alkoxy. Besonders bevorzugt sind Verbindungen mit $R^5$ = Halogen, $R^6$, $R^7$ und $R^9$ = Wasserstoff, und $R^8$ = nieder-Alkoxy. Bevorzugte Reste $R^{10}$ sind nieder-Alkyl, Hydroxy-nieder-alkyl, Carboxy-nieder-alkyl, nieder-Alkoxycarbonyl-nieder-alkyl, nieder-Alkanoyloxy-nieder-alkyl, Phenyl, das durch Halogen, Hydroxy, nieder-Alkyl, nieder-Alkoxy, nieder-Alkoxycarbonyl, nieder-Alkanoyloxy, Trifluormethyl oder Methylendioxy substituiert sein kann; Phenyl-carbamoyl-nieder-alkyl, Cyclohexyl; heterocyclische Reste wie Pyridyl, Pyridyl-N-oxid, N-nieder-Alkylpyridyl, Thienyl, Furyl, N-nieder-Alkyl-pyrrolyl, 1,4-Diazinyl; Picolyl, Picolyl-N-oxid, Furylmethyl, Chinolyl, Morpholino-carbonyl, Morpholino-carbonyl-nieder-alkyl, und Pyrrolidino-carbonyl-nieder-alkyl. $R^{11}$ ist vorzugsweise Wasserstoff.

Die Verbindungen der oben angegebenen Formel I sind Hemmstoffe für Endothelin-Rezeptoren. Sie können daher zur Behandlung von Erkrankungen, die mit Endothelin-Aktivitäten assoziiert sind, insbesondere Kreislauferkrankungen, wie Hypertonie, Ischämie, Vasospasmen und Angina pectoris verwendet werden.

Die Erfindung umfasst daher die Verwendung der Verbindungen der Formel I und ihrer Salze zur Behandlung der oben erwähnten Erkrankungen, ihre Verwendung als Wirkstoffe bei der Herstellung von Heilmitteln für die erwähnten Indikationen, sowie pharmazeutische Präparate, die Verbindungen der Formel I oder deren Salze enthalten.

Die Verbindungen der Formel I können erfindungsgemäss dadurch hergestellt werden, dass man eine Verbindung der Formel

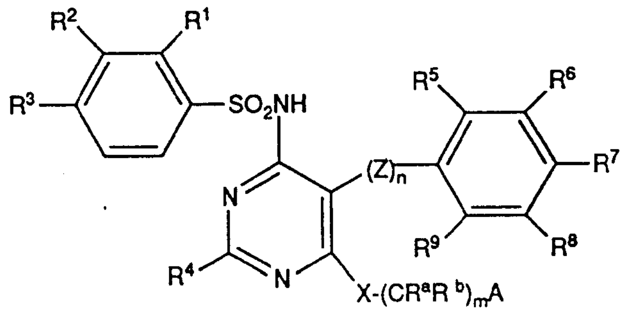

II

worin $R^1$-$R^9$, $R^a$, $R^b$, X, Z, m und n die oben angegebene Bedeutung haben und A Hydroxy oder Amino ist,

a) mit einem Isocyanat der Formel $R^{10}NCO$ oder einem Carbamoylchlorid der Formel $(R^{10}R^{11})NCOCl$ umsetzt, worin $R^{10}$ und $R^{11}$ die in Anspruch 1 angegebene Bedeutung haben, oder

b) mit Phosgen und danach mit einem Alkohol der Formel $R^{10}OH$; oder mit einem Chlorameisensäureester der Formel $R^{10}OC(O)Cl$ umsetzt,

und gewünschtenfalls in der so erhaltenen Verbindung der Formel I anwesende Substituenten abwandelt.

Die Umsetzung gemäss Verfahrensvariante a) kann in für die Herstellung von Carbamaten und Harnstoffen aus Alkoholen bzw. Aminen an sich bekannter Weise erfolgen. So kann eine Verbindung der Formel II, in der A Hydroxy ist,

mit einem Isocyanat der Formel $R^{10}NCO$ in einem geeigneten wasserfreien organischen Lösungsmittel, z.B. einem Kohlenwasserstoff wie Toluol, zweckmässig unter Erwärmen, zu einer Verbindung der Formel I, in der Y $-OC(O)NHR^{10}$ ist, umgesetzt werden. Das Isocyanat kann dabei in situ, z.B. aus einem Azid der Formel $R^{10}CON_3$ durch thermische Zersetzung, erzeugt werden. Entsprechend können unter Verwendung von Verbindungen der Formel II, in denen A Amino ist, Verbindungen der Formel I mit Y = $-NHC(O)NR^{10}$ erhalten werden. Alternativ können Verbindungen der Formel II, in der A Hydroxy ist, mit Verbindungen der Formel $R^{10}R^{11}NC(O)Cl$ unter analogen Reaktionsbedingungen zu Verbindungen der Formel I, in denen Y ein Rest $-OC(O)NR^{10}R^{11}$ ist, und Verbindungen der Formel II, in der A Amino ist, zu Verbindungen der Formel I, in der Y ein Rest $-NHC(O)NR^{10}R^{11}$ ist, umgesetzt werden.

Nach Verfahrensvariante b) kann eine Verbindung der Formel II, in der A Hydroxy ist, mit Phosgen und danach mit einem Alkohol der Formel $R^{10}OH$ zu einer Verbindung der Formel I, in der Y ein Rest $-OC(O)OR^{10}$ ist, umgesetzt werden. Anstelle von Phosgen kann ein Phosgensalz, wie Diphosgen ($Cl-COOCCl_3$) oder Triphosgen ($CO(OCCl_3)_2$ eingesetzt werden. Analog erhält man ausgehend von Verbindungen der Formel II mit A = Amino Verbindungen der Formel I mit Y = $NHC(O)OR^{10}$. Das Phosgen wird dabei zweckmässig als Lösung in einem inerten wasserfreien organischen Lösungsmittel, z.B. einem Kohlenwasserstoff wie Toluol, eingesetzt. Die

Umsetzung mit Phosgen kann bei Raumtemperatur vorgenommen werden. Das intermediär erhaltene Säurechlorid wird unmittelbar mit dem Alkohol $R^{10}OH$, zweckmässig unter Erwärmen, umgesetzt.

In den so erhaltenen Verbindungen der Formel I können darin anwesende Substituenten abgewandelt werden. Beispielsweise können Estergruppen verseift oder zur Alkoholgruppe reduziert werden; N-heterocyclische Reste, wie Pyridylreste, können zu N-Oxiden oxidiert oder N-alkyliert werden; Carbonsäuregruppen können in Ester oder Amide überführt werden. Schliesslich können die Verbindungen der Formel I in Salze, z.B. Alkalisalze, wie Na- und K-Salze, oder Erdalkalimetallsalze, wie Ca- oder Mg-Salze überführt werden. Alle diese Operationen können in an sich bekannter Weise vorgenommen werden.

Die als Ausgangsmaterial eingesetzten Verbindungen sind generell bekannt, z.B. aus den europäischen Patentpublikationen EP-A-0526708 und EP-A-510526 oder können, soweit sie nicht im einzelnen bekannt oder ihre Herstellung nachstehend beschrieben ist, in Analogie zu bekannten oder den nachstehend beschriebenen Methoden hergestellt werden.In der EP-A-0 526 708 werden Sulfonylaminopyrimidine beschrieben, die Hemmstoffe für Endothelinrezeptoren sind.

Die Hemmwirkung der Verbindungen der Formel I auf Endothelinrezeptoren kann mit der nachstehend beschriebenen Versuchsanordnungen gezeigt werden:

I: Hemmung der Endothelin-Bindung am recombinanten $ET_A$-Rezeptor

Eine cDNA, die für humanen $ET_A$-Rezeptor von menschlicher Plazenta kodiert, wurde kloniert (M. Adachi, Y.-Y. Yang, Y. Furuichi und C-Miyamoto, BBRC 180, 1265-1272) und im Baculovirus-Insektenzellen-System exprimiert. Baculovirus-infizierte Insektenzellen aus einem 23 l Fermenter werden 60 Stunden nach der Infektion abzentrifugiert (3 000 x g, 15 Minuten, 4°C), in Tris-Puffer (5 mM, pH 7.4, 1 mM $MgCl_2$) resuspendiert und erneut zentrifugiert. Nach erneuter Resuspension und Zentrifugation werden die Zellen in 800 ml des gleichen Puffers suspendiert und bei -120°C eingefroren. Der Aufbruch der Zellen erfolgt beim Auftauen der Suspension in diesem hypotonen Puffergemisch. Nach wiederholtem Gefrier/Auftau-Zyklus wird die

Suspension homogenisiert und zentrifugiert (25 000 x g, 15 Minuten, 4°C). Nach Suspension in Tris-Puffer (75 mM, pH 7.4, 25mM $MgCl_2$, 250 mM Saccharose) werden 1 ml Aliquots (Proteingehalt ca. 3.5 mg/ml) bei -85°C aufbewahrt.

Für den Bindungs-Assay werden die eingefrorenen Membranpräparate aufgetaut und nach 10 Minuten Zentrifugieren mit 25000 g bei 20°C in Assay-Puffer (50 mM Tris-Puffer pH 7.4, enthaltend 25 mM $MnCl_2$, 1 mM EDTA und 0,5% Rinderserumalbumin) resuspendiert. 100 µl dieser Membransuspension, enthaltend 70 µg Protein werden mit 50 µl $^{125}$I-Endothelin (spezif. Aktivität 2200 Ci/mMol) in Assay-Puffer (25000 cpm, Endkonzentration 20 pM) und 100 µl Assay-Puffer, der variierende Konzentrationen der Testverbindung enthält, inkubiert. Die Inkubation wird 2 Stunden bei 20°C oder 24 Stunden bei 4°C durchgeführt. Die Trennung von freiem und Membran-gebundenen Radioliganden wird durch Filtration über Glasfaserfilter vorgenommen.

In der Tabelle 1 ist die in dieser Versuchsanordnung ermittelte Hemmwirkung von Verbindungen der Formel I als $IC_{50}$ angegeben, d.h., als Konzentration [µM] die erforderlich ist, 50% der spezifischen Bindung von $^{125}$I-Endothelin zu hemmen.

Tabelle 1

| Verbindung von Beispiel | $IC_{50}$ [µM] |
|---|---|
| 10 | 0,001 |

Tabelle 1 (fortgesetzt)

| Verbindung von Beispiel | $IC_{50}$ [µM] |
|---|---|
| 70 | 0,006 |
| 75 | 0,003 |
| 120 | 0,007 |
| 134 | 0,044 |
| 140 | 0,009 |

II. Hemmung Endothelin-induzierter Kontraktionen an isolierten Aortenringen der Ratte

Aus der Thoraxaorta von erwachsenen Wistar-Kyoto-Ratten wurden Ringe von 5 mm Länge herausgeschnitten. Das Endothel wurde durch leichtes Reiben der Innenfläche entfernt. Jeder Ring wurde bei 37°C in 10 ml Krebs-Henseleit-Lösung unter Begasung mit 95% $O_2$ und 5% $CO_2$ in ein isoliertes Bad eingetaucht. Die isometrische Spannung der Ringe wurde gemessen. Die Ringe wurden auf eine Vorspannung von 3 g gedehnt. Nach 10 Minuten Inkubation mit der Testverbindung oder Vehikel wurden kumulative Dosen von Endothelin-1 zugegeben. Die Aktivität der Testverbindung wurde ermittelt durch die zu beobachtende Rechtsverschiebung der Dosis-Wirkungskurve von Endothelin-1 in gegenwart verschiedener Konzentrationen an Antagonist. Diese Rechtsverschiebung (oder auch "dose ratio", DR) entspricht dabei dem Quotienten aus den $EC_{50}$-Werten von Endothelin-1 in Gegenwart und in Abwesenheit von Antagonist, wobei der $EC_{50}$-Wert die für eine halbmaximale Kontraktion erforderliche Endothelin-Konzentration bezeichnet.

Aus der "dose ratio" DR wurde mittels eines Computerprogramms für jede einzelne Dosis-Wirkungskurve der entsprechende $PA_2$-Wert nach untenstehender Gleichung berechnet, welcher ein Mass für die Aktivität der Testverbindung darstellt.

$$pA_2 = \log(DR-1)-\log(\text{Antagonist-Konzentration})$$

Die $EC_{50}$ von Endothelin in Abwesenheit von Testverbindungen ist 0,3 nM.

Die mit Verbindungen der Formel I so erhaltenen Werte für $pA_2$ sind in Tabelle 2 angegeben.

Tabelle 2

| Verbindung von Beispiel | Dosisverhältnis (Rechtsverschiebung) |
|---|---|
| 10 | 8,2 |
| 70 | 9,0 |
| 75 | 7,3 |
| 120 | 7,2 |
| 134 | 7,3 |
| 140 | 7,0 |

III. Die in vivo Aktivität der Verbindungen der Formel I kann in einem pathophysiologisch relevanten Modell in der Ratte wie folgt gezeigt werden:

In "stroke-prone", spontan-hypertensive Ratten wurde unter Anästhesie ein Telemetrie-System (PA-C40 Implantate) implantiert, um kontinuierlich den arteriellen Blutdruck und die Herzfrequenz messen zu können. Nach der Operation liess man den Tieren 2 Wochen Zeit, sich zu erholen.

Telemetrische Messungen zeigten, dass der mittlere arterielle Blutdruck der Tiere erhöht war und etwa 190 mm Hg betrug. Die entsprechende Testverbindung (30 mg/kg in Gummi arabicum) wurde schliesslich mittels einer Sonde verabreicht und der Blutdruck kontinuierlich registriert. Es zeigte sich dabei, dass Verbindungen der Formel I einen Blutdruckabfall verursachten, wobei die Verabreichung von Gummi arabicum allein (Placebo-Kontrolle) keinen signifikanten Effekt auf den Blutdruck hatte.

Der mit einer Verbindung der Formel I erhaltenen Messwert ist in Tabelle 3 angegeben.

Tabelle 3

| Verbindung von Beispiel | % Abnahme der mittleren arteriellen Blutdrucks (mm Hg) |
|---|---|
| 70 | 30 |

Die Verbindungen der Formel I können aufgrund ihrer Fähigkeit, die Endothelinbindung zu hemmen, als Mittel zur Behandlung von Erkrankungen verwendet werden, die mit die Vasokonstriktion erhöhenden Vorgängen assoziiert sind. Beispiele solcher Erkrankungen sind Bluthochdruck, Koronarerkrankungen, Herzinsuffizienz, renale und myocardiale Ischämie, Niereninsüffizienz, Dialyse, cerebrale Ischämie, Hirninfarkt, Migräne, subarachnoidale Hämorrhagie, Raynaud-Syndrom und pulmonärer Hochdruck. Sie können ebenfalls bei Atherosklerose, Verhinderung der Restenosierung nach Ballon-induzierter Gefässdilatation, Entzündungen, Magen- und Duodenalulcera, Ulcus cruris, Gram-negativer Sepsis, Schock, Glomerulonephritis, Nierenkolik, Glaukom, Asthma, bei der Therapie und Prophylaxe diabetischer Komplikationen und Komplikationen bei der Verabreichung von Cyclosporin, sowie anderen, mit Endothelin-Aktivitäten assoziierten Erkrankungen Anwendung finden.

Die Verbindungen der Formel I können oral, rectal, parenteral, z.B. intravenös, intramuskulär, subcutan, intrathecal oder transdermal; oder sublingual oder als ophthalmologische Zubereitung, oder als Aerosol verabreicht werden. Beispiele von Applikationsformen sind Kapseln, Tabletten, oral verabreichbare Suspensionen oder Lösungen, Suppositorien, Injektionslösungen, Augentropfen, Salben oder Spraylösungen.

Eine bevorzugte Anwendungsform ist die intravenöse, intramuskuläre oder orale Applikation. Die Dosierung, in denen die Verbindungen der Formel I in wirksamen Mengen verabreicht werden, hängen von der Art des spezifischen Wirkstoffs, dem Alter und den Bedürfnissen des Patienten und der Applikationsweise ab. Im allgemeinen kommen Dosierungen von etwa 0,1-100 mg/kg Körpergewicht pro Tag in Betracht. Die die Verbindungen der Formel I enthaltenden Präparate können inerte oder auch pharmakodynamisch aktive Zusätze enthalten. Tabletten oder Granulate z.B. können eine Reihe von Bindemitteln, Füllstoffen, Trägersubstanzen oder Verdünnungsmitteln enthalten. Flüssige Präparate können beispielsweise in Form einer sterilen, mit Wasser mischbaren Lösung vorliegen. Kapseln können neben dem Wirkstoff zusätzlich ein Füllmaterial oder Verdickungsmittel enthalten. Des weiteren können geschmacksverbessernde Zusätze, sowie die üblicherweise als Konservierungs-, Stabilisierungs-, Feuchthalte- und Emulgiermittel verwendeten Stoffe, ferner auch Salze zur Veränderung des osmotischen Druckes, Puffer und andere Zusätze vorhanden sein.

Die vorstehend erwähnten Trägersubstanzen und Verdünnungsmittel können aus organischen oder anorganischen Stoffen, z.B. aus Wasser, Gelatine, Milchzucker, Stärke, Magnesiumstearat, Talkum, Gummi arabicum, Polyalkylenglykolen und dergleichen bestehen. Voraussetzung ist, dass alle bei der Herstellung der Präparate verwendeten Hilfsstoffe untoxisch sind.

Die nachstehenden Beispiele erläutern die Erfindung weiter. Von den darin verwendeten Abkürzungen bedeutet THF Tetrahydrofuran; DMSO Dimethylsulfoxid; MeOH Methanol; Sdp. Siedepunkt; und Smp. Schmelzpunkt.

Beispiel 1

a) Zu 121,5 mg N-[5-(2-Chloro-5-methoxy-phenoxy)-6-(2-hydroxy-ethoxy)-pyrimidin-4-yl]-2,3-dihydro-1,4-benzodioxin-6-sulfonamid, in 1 ml trockenem Toluol gelöst, gab man 57 mg 3-Pyridyl-isocyanat. Das Reaktionsgemisch wurde während 1 Stunde bei 100°C gerührt. Dann wurde die Lösung auf eine Kieselgelsäule gegeben und es wurde mit EtOAc eluiert. Man erhielt Pyridin-3-ylcarbaminsäure 2-[5-(2-chloro-5-methoxy-phenoxy]-6-(2,3-dihydro-1,4-benzodioxin-6-ylsulfonylamino)-pyrimidin-4-yloxy]-ethylester, weisses Pulver, MS: m/e = 630,4 $(M+H)^+$.

Herstellung der Ausgangsverbindung

b) 3-Methoxyphenol wurde nach der Vorschrift von M. Julia und I. de Rosnay, Chimie Thérapeutique 5 (1969), 334 in 2-Chlor-5-methoxyphenol mit Sulfurylchlorid umgewandelt.

c) 18,2 g 2-Chlor-5-methoxyphenol wurden in 150 ml trockenem Methanol gelöst. Man gab 9,3 g MeONa zu, gefolgt von 25 g Chlormalonsäuredimethylester. Das Reaktionsgemisch wurde während 2 Stunden bei 50°C gerührt. Nach Abdestillieren des Lösungsmittels wurde der Rückstand im Scheidetrichter zwischen Toluol und $H_2O$ verteilt und neutral gewaschen. Nach Kristallisation in Ethanol erhielt man (2-Chlor-5-methoxy)phenoxy-dimethylmalonat, weisse Kristalle mit Smp. 68-69°C.

d) 1,43 g Na wurden in 70 ml MeOH gelöst. Dann wurden 5,8 g (2-Chlor-5-methoxy)phenoxy-dimethylmalonat und 2,29 g Formamidin-acetat zugegeben; das Reaktionsgemisch wurde während 1,5 Stunden unter Rückfluss gerührt. Dann wurde das Lösungsmittel abdestilliert, der Rückstand in $H_2O$ aufgenommen, die wässrige Phase mit Essigester extrahiert, die organische Phase verworfen und die wässrige Phase mit Essigsäure auf pH 4 angesäuert, wobei das 5-(2-Chlor-5-methoxy)phenoxy-4,6(1H,5H)-pyrimidindion als weisses Pulver ausfiel. MS: m/e = 268 (M).

e) Ein Gemisch von 3,75 g 5-(2-Chlor-5-methoxy)phenoxy-4,6(1H,5H)-pyrimidindion, 5,4 g N-Ethyldiisopropylamin, 12,5 ml $POCl_3$ in 20 ml Dioxan wurde unter Rückfluss während 18 Stunden gerührt. Nach Abdestillieren der flüchtigen Komponenten, wurde der Rückstand zwischen Essigester und $H_2O$ verteilt und neutral gewaschen. Nach Abdestillieren des Lösungsmittels wurde die Verbindung an Kieselgel mit $CH_2Cl_2$ als Fliessmittel gereinigt. Nach Kristallisation aus EtOH erhielt man 4,6-Dichlor-5-(2-chlor-5-methoxy-phenoxy)-pyrimidin als weisse Kristalle mit Smp. 88-89°C.

f) Ein Vilsmeier-Reagens wurde hergestellt, indem man unter Eiskühlung 29 g Sulfurylchlorid zu 15,7 g DMF tropfte. Anschliessend wurden 27,3 g 1,2-Ethylendioxy-benzol zugetropft. Nach Ende der Zugabe wurde das Reaktionsgemisch während einer Stunde bei 100°C gerührt. Dann wurde die Lösung auf Eis gegossen und das gebildete Sulfochlorid mit $CH_2Cl_2$ extrahiert. Nach Abdestillieren des Dichlormethans wurde der Rückstand in THF aufgenommen. Man gab 10 ml 25%ige $NH_4OH$-Lösung zu und rührte während 0,5 Stunden. Das gebildete 1,4-Benzodioxan-6-sulfonamid wurde aus Essigester kristallisiert. Man erhielt leicht gelbe Kristalle. Das Sulfonamid wurde in das K-Salz durch Zugabe der stöchiometrischen Menge KOH übergeführt.

g) 917 mg 4,6-Dichlor-5-(2-chlor-5-methoxy-phenoxy)-pyrimidin und 1,29 g des 1,4-Benzodioxan-6-sulfonamid-Salzes aus 1f) wurden in 4 ml DMSO gelöst. Nach 1 Stunde bei 100°C war die Reaktion beendet. Nach Abdestillieren des Lösungsmittels wurde der Rückstand zwischen 1N HCl und Essigester verteilt. Nach Isolieren des Reaktionsproduktes wurde das N-[6-Chlor-5-(2-chlor-5-methoxy-phenoxy)-pyrimidin-4-yl]-2,3-dihydro-1,4-benzodioxan-6-sulfonamid aus Propanol kristallisiert. Smp. 185-186°C.

h) 485 mg N-[6-Chlor-5-(2-chlor-5-methoxy-phenoxy)-pyrimidin-4-yl]-2,3-dihydro-1,4-benzodioxan-6-sulfonamid wurden zu einer Na-Glykolat-Lösung (115 mg Na in 2 g Ethylenglykol) gegeben. Man liess während 1 Stunde bei 100°C reagieren. Nach Isolieren des Reaktionsproduktes wurde an Kieselgel mit Dichlormethan-Diäthyläther (1:1 in Vol.) als Fliessmittel chromatographiert. Man kristallisierte aus Diisopropyläther und erhielt N-[5-(2-Chlor-5-methoxy-phenoxy)-6-(2-hydroxy-ethoxy)-pyrimidin-4-yl]-2,3-dihydro-1,4-benzodioxan-6-sulfonamid als weisse Kristalle mit Smp. 180-181°C.

Beispiel 2

a) Eine Lösung von 100 mg 4-Pyridylcarbonsäureazid in 2 ml absolutem Toluol wurde während 1 Stunde bei 100°C gehalten, wobei das entsprechende Isocyanat gebildet wurde. Man gab dann 100 mg N-[5-(2-Chlor-5-methoxy-phenoxy)-6-(2-hydroxy-ethoxy)-pyrimidin-4-yl]-1,3-benzodioxol-5-sulfonamid zu und liess 2 Stunden bei dieser Temperatur reagieren. Dann wurde die Reaktionslösung auf eine Kieselgelsäule gegeben und es wurde mit Essigester eluiert. Der erhaltene Pyridin-4-ylcarbamylsäure 2-[6-(1,3-benzodioxol-5-ylsulfonylamino)-5-(2-chlor-5-methoxy-phenoxy)-pyrimidin-4-yloxy]-ethylester wurde aus $CHCl_3$ kristallisiert. Smp. 230°C (Zers.).

Herstellung der Ausgangsverbindung

b) Das (1,3-Benzodioxol-5-sulfonamid)-K wurde nach dem Verfahren des Beispiels 1, Abschnitt f) aus 1,3-Benzodioxol hergestellt.

c) 458 mg 4,6-Dichlor-5-(2-chlor-5-methoxy-phenoxy)-pyrimidin und 574 mg (1,3-Benzodioxol-5-sulfonamid)-K wurden zu N-[6-Chlor-5-(2-chlor-5-methoxy-phenoxy)-pyrimidin-4-yl]-1,3-benzodioxol-5-sulfonamid umgesetzt. Weisse Kristalle (aus MeOH). Smp. 230°C.

d) 470,3 mg der Verbindung aus 2c) wurden mit Na-Glykolat in Ethylenglykol in N-[5-(2-Chlor-5-methoxy-phenoxy)-6-(2-hydroxy-ethoxy)-pyrimidin-4-yl]-1,3-benzodioxol-5-sulfonamid überführt. Weisse Kristalle, Smp. 164°C.

e) 6,15 g Isonicotinsäure und 5,55 g Triäthylamin wurden in 50 ml Aceton gelöst. Zu dieser auf -10°C gekühlten Lösung wurden 6 g Chlorameisensäure-äthylester getropft. Nach 2 Stunden wurde bei 0°C eine Lösung von 5 g $NaN_3$ in 20 ml $H_2O$ gelöst zugetropft. Nach 1 Stunde bei Raumtemperatur wurde der Feststoff abfiltriert und verworfen. Die Lösung wurde bei Raumtemperatur abdestilliert und der Rückstand mit Dichlormethan extrahiert. Nach Trocknen über $MgSO_4$ wurde das Lösungsmittel abdestilliert und die zurückbleibende Flüssigkeit über Kieselgel mit Dichlormethan als Fliessmittel filtriert. Man erhielt das 4-Pyridylcarbonsäureazid als farblose Flüssigkeit, die im Tiefkühler (-18°C) kristallisierte. IR: 2141, 2190 $cm^{-1}$ ($N_3$).

Beispiel 3

a) In Analogie zu Beispiel 2 erhielt man aus 100 mg N-[5-(2-Chlor-5-methoxy-phenoxy)-6-(2-hydroxy-ethoxy)-pyrimidin-4-yl]-1,3-benzodioxol-5-sulfonamid und 100 mg 3-Pyridyl-carbonsäureazid den Pyridin-3-carbamylsäure 2-[6-(1,3-benzodioxol-5-ylsulfonylamino)-5-(2-chlor-5-methoxy-phenoxy)-pyrimidin-4-yloxy)-ethylester. Smp. 114-115°C (Zers.).

Herstellung der Ausgangsverbindung

b) Das 3-Pyridyl-carbonsäureazid wurde nach dem Verfahren von Beispiel 2, Abschnitt c) aus Nicotinsäure hergestellt. IR: 2137, 2179 $cm^{-1}$ ($N_3$).

Beispiel 4

a) In Analogie zu Beispiel 2 erhielt man aus 250 mg N-[5-(2-Chlor-5-methoxy-phenoxy)-6-(2-hydroxy-ethoxy)-pyrimidin-4-yl)-1,3-benzodioxol-5-sulfonamid und 225 mg 2-Pyridyl-carbonsäureazid den Pyridin-2-carbamylsäure 2-[6-(1,3-benzodioxol-5-ylsulfonylamino)-5-(2-chlor-5-methoxy-phenoxy)-pyrimidin-4-yloxy]-ethylester. Smp. 206°C (Zers.).

Herstellung der Ausgangsverbindung

b) Das 2-Pyridyl-carbonsäureazid wurde nach dem Verfahren von Beispiel 2, Abschnitt e) aus Picolinsäure hergestellt.

Beispiel 5

a) In Analogie zu Beispiel 2 erhielt man aus 100 mg 4-tert-Butyl-N-[5-(2-chlor-5-methoxy-phenoxy)-6-(2-hydroxy-ethoxy)-2-methoxymethyl]-benzolsulfonamid und 100 mg 3-Pyridyl-carbonsäureazid den Pyridin-3-carbamylsäure 2-[6-(4-tert-butyl-phenylsulfonylamino)-5-(2-chlor-5-methoxy-phenoxy)-2-methoxymethyl-pyrimidin-4-yloxy]-ethylester. MS: m/e = 672,6 ($M+H^+$).

Herstellung der Ausgangsverbindung

b) 16 g Methoxyacetonitril und 11,45 g Ethanol wurden in 50 ml Diäthyläther gelöst. Die Lösung wurde bei 0°C mit trockenem HCl gesättigt. Man liess die Lösung während 3 Tagen bei Raumtemperatur stehen. Dann wurde das Lösungsmittel abdestilliert, wobei Kristallisation eintrat. Smp. 122°C (Zers.).

c) 15,3 g des Imidoesterhydrochlorids aus b) wurden in 25 ml Methanol gelöst. 15,4 ml einer 6,5N $NH_3$-Lösung in Methanol wurden in einem Guss zugegeben, wobei sich ein Niederschlag bildete, der sich rasch auflöste. Man liess die Lösung während 24 Stunden bei Raumtemperatur stehen. Dann wurde das Lösungsmittel abdestilliert, wobei allmählich Kristallisation eintrat.

d) In Analogie zu Beispiel 1, Abschnitt d) erhielt man aus 5,1 g Methoxymethyl-amidin-hydrochlorid und 11,2 g (2-Chlor-5-methoxy-phenoxy)-dimethylmalonat das 2-Methoxymethyl-5-(2-chlor-5-methoxy-phenoxy-4,6(1H,5H)-pyrimidindion als weisses Pulver. MS: 312 (M).

e) In Analogie zu Beispiel 1, Abschnitt e) erhielt man das 4,6-Dichlor-2-methoxymethyl-5-(2-chlor-5-methoxy-phenoxy)-pyrimidin, Smp. 105°C (aus EtOH).

f) In Analogie zu Beispiel 1, Abschnitt g) erhielt man aus dem Dichlorid aus e) und (4-tert-Butyl-benzolsulfonamid)-K das 4-tert-Butyl-N-[6-chlor-5-(2-chlor-5-methoxy-phenoxy)-2-methoxymethyl-pyrimidin-4-yl]-benzolsulfonamid. MS: 526 ($M+H^+$).

g) In Analogie zu Beispiel 1, Abschnitt h) erhielt man aus 0,3 g der Verbindung aus Beispiel 5, Abschnitt f) das 4-tert-Butyl-N-[5-(2-chlor-5-methoxy-phenoxy)-6-(2-hydroxy-ethoxy)-2-methoxymethyl-pyrimidin-4-yl]-benzolsulfonamid. MS: 551 (M).

Beispiel 6

In Analogie zu Beispiel 2 erhielt man aus 100 mg 4-tert-Butyl-N-[5-(2-chlor-5-methoxy-phenoxy)-6-(2-hydroxy-ethoxy)-2-methoxymethyl-pyrimidin-4-yl]-benzolsulfonamid und 100 mg 2-Pyridylcarbonsäureazid den Pyridin-2-carbamylsäure 2-[6-(4-tert-butyl-phenylsulfonylamino)-5-(2-chlor-5-methoxy-phenoxy)-2-methoxymethyl-pyrimidin-4-yloxy]-ethylester. Smp. 174-175°C (aus EtOH).

Beispiel 7

a) In Analogie zu Beispiel 2 erhielt man aus 200 mg N-[5-(2-Chlor-5-methoxy-phenoxy)-6-(2-hydroxy-ethoxy)-2-methoxymethyl-pyrimidin-4-yl]-1,3-benzodioxol-5-sulfonamid und 165 mg 2-Pyridyl-carbonsäureazid den Pyridin-2-carbamylsäure 2-[6-(1,3-benzodioxol-5-ylsulfonylamino)-5-(2-chlor-5-methoxy-phenoxy)-2-methoxymethyl-pyrimidin-4-yloxy]-ethylester. Smp. 160-162°C.

Herstellung der Ausgangsverbindung

b) In Analogie zu Beispiel 1, Abschnitt g) erhielt man aus 700 mg 4,6-Dichlor-2-methoxymethyl-5-(2-chlor-5-methoxy-phenoxy)-pyrimidin und 766 mg (1,3-Benzodioxol-5-sulfonamid)-K das N-[6-Chlor-5-(2-chlor-5-methoxy-phenoxy)-2-methoxymethyl-pyrimidin-4-yl]-1,3-benzodioxol-5-sulfonamid. Smp. 114-116°C (aus EtOH).

c) In Analogie zu Beispiel 1, Abschnitt h) wurde diese Verbindung in das N-[5-(2-Chlor-5-methoxy-phenoxy)-6-(2-hydroxy-ethoxy)-2-methoxymethyl-pyrimidin-4-yl]-1,3-benzodioxol-5-sulfonamid übergeführt.

Beispiel 8

In Analogie zu Beispiel 2 erhielt man aus 125 mg N-[5-(2-Chlor-5-methoxy-phenoxy)-6-(2-hydroxy-ethoxy)-pyrimidin-4-yl]-1,3-benzodioxol-5-sulfonamid und 90 mg 1-Methyl-pyrrol-2-carbonsäureazid den 1-Methyl-pyrrol-2-carbamylsäure 2-[6-(1,3-benzodioxol-5-ylsulfonylamino)-5-(2-chlor-5-methoxy-phenoxy)-pyrimidin-4-yloxy]-ethylester. MS: 618,3 ($M+H^+$).

Das 1-Methyl-pyrrol-2-carbonsäureazid wurde aus 1-Methyl-pyrrol-2-carbonsäure mittels des Verfahrens von Beispiel 2e) als farblose Flüssigkeit erhalten. IR: 2132 $cm^{-1}$ ($N_3$).

Beispiel 9

In Analogie zu Beispiel 2 erhielt man aus 95 mg 4-tert-Butyl-N-[5-(2-chlor-5-methoxy-phenoxy)-6-(2-hydroxy-ethoxy)-pyrimidin-4-yl]-benzolsulfonamid und 90 mg 1,3-Benzodioxol-5-carbonsäureazid den 1,3-Benzodioxol-5-ylcarbaminsäure 2-[6-(4-tert-butyl-phenylsulfonylamino)-5-(2-chlor-5-methoxy-phenoxy)-pyrimidin-4-yloxy]-ethylester. MS: 671,4 ($M+H^+$).

Das 1,3-Benzodioxol-5-carbonsäureazid wurde ausgehend von der entsprechenden Carbonsäure nach dem Verfahren des Beispiels 2e) erhalten. IR: 2144 $cm^{-1}$ ($N_3$).

Beispiel 10

a) In Analogie zu Beispiel 2 erhielt man aus 100 mg N-[5-(2-Chlor-5-methoxy-phenoxy)-6-(2-hydroxy-ethoxy)-2-(2-methoxyethyl)-pyrimidin-4-yl]-1,3-benzodioxol-5-sulfonamid und 80 mg 2-Pyridyl-carbonsäureazid den Pyridin-2-ylcarbaminsäure 2-[6-(1,3-benzodioxol-5-ylsulfonylamino)-5-(2-chlor-5-methoxy-phenoxy)-2-(2-methoxyethyl)-pyrimidin-4-yloxy]-ethylester. Smp. 156°C (aus EtOH).

Herstellung der Ausgangsverbindung

b) In Analogie zu Beispiel 5, Abschnitt b) erhielt man aus Methoxypropionitril, HCl und Ethanol das Methoxyethyl-imidoethylester-hydrochlorid. MS: 116 ($M-CH_3$).

c) In Analogie zu Beispiel 5, Abschnitt c) erhielt man das Methoxyethyl-amidin-hydrochlorid.

d) In Analogie zu Beispiel 1, Abschnitt d) erhielt man aus 4,16 g Amidin-hydrochlorid aus 10c) und 8,66 g (2-Chlor-5-methoxy-phenoxy)-dimethylmalonat das (2-Chlor-5-methoxy-phenoxy)-2-methoxyethyl-4,6(1H,5H)-pyrimidindion. MS: 326 (M).

e) Nach dem Verfahren des Beispiels 1, Abschnitt e) wurde das Pyrimidindion in das 4,6-Dichlor-5-(2-chlor-5-methoxy-phenoxy)-2-methoxyethyl-pyrimidin überführt. Smp. 66,5-67,5°C (aus EtOH).

f) Aus 727 mg des Dichlorids aus 10e) und 718 mg (1,3-Benzodioxol-5-sulfonamid)-K erhielt man das N-[6-Chlor-5-(2-chlor-5-methoxy-phenoxy)-2-(2-methoxy-ethyl)-pyrimidin-4-yl]-1,3-benzodioxol-5-sulfonamid. Smp. 154-155°C (aus MeOH).

g) In Analogie zu Beispiel 1, Abschnitt h) wurde die Verbindung aus 10f) in das N-[5-(2-Chlor-5-methoxy-phenoxy)-6-(2-hydroxy-ethoxy)-2-(2-methoxyethyl)-pyrimidin-4-yl]-1,3-benzodioxol-5-sulfonamid überführt. Smp. 135-136°C (aus Diethylether).

Beispiel 11

In Analogie zu Beispiel 2 erhielt man aus 100 mg 4-tert-Butyl-N-[(2-chlor-5-methoxy-phenoxy)-6-(2-hydroxy-ethoxy)-pyrimidin-4-yl]-benzolsulfonamid und 122 mg Thiophen-3-carbonsäureazid den Thiophen-3-ylcarbaminsäure 2-[6-(4-tert-butyl-phenylsulfonylamino)-5-(2-chlor-5-methoxy-phenoxy)-pyrimidin-4-yloxy]-ethylester. MS: 633,2 $(M+H^+)$.

Das Thiophen-3-carbonsäureazid wurde aus Thiophen-3-carbonsäure nach der Vorschrift des Beispiels 2, Abschnitt e) als farblose Flüssigkeit, die im Tiefkühler (-18°C) kristallisierte, erhalten. IR: 2139, 2273 $cm^{-1}$ $(N_3)$.

Beispiel 12

In Analogie zu Beispiel 2 erhielt man aus 100 mg N-[(2-Chlor-5-methoxy-phenoxy)-6-(2-hydroxy-ethoxy)-pyrimidin-4-yl]-1,3-benzodioxol-5-sulfonamid und 122 mg Thiophen-3-carbonsäureazid den Thiophen-3-ylcarbamylsäure 2-[6-(1,3-benzodioxol-5-ylsulfonylamino)-5-(2-chlor-5-methoxy-phenoxy)-pyrimidin-4-yloxy]-ethylester. MS: 621,2 $(M+H^+)$.

Beispiel 13

a) In Analogie zu Beispiel 2 erhielt man aus 100 mg N-[5-(2-Chlor-5-methoxy-phenoxy)-6-(2-hydroxy-ethoxy)-2-methylsulfanyl-pyrimidin-4-yl]-1,3-benzodioxol-5-sulfonamid und 82 mg 2-Pyridyl-carbonsäureazid den Pyridin-2-ylcarbaminsäure 2-[6-(1,3-benzodioxol-5-ylsulfonylamino)-5-(2-chlor-5-methoxy-phenoxy)-2-methylsulfanyl-pyrimidin-4-yloxy]-ethylester. Weisse Kristalle. Smp. 173-174°C (aus EtOH).

b) Eine Lösung von 230 mg Na-Methylat in 15 ml Methanol, 1,45 g (2-Chlor-5-methoxy-phenoxy)-dimethylmalonat und 381 mg Thioharnstoff wurde während 5 Stunden am Rückfluss gehalten. Dann wurde das Lösungsmittel abdestilliert und der Rückstand in 10 ml $H_2O$ gelöst. Zu dieser Lösung gab man 630 mg Dimethylsulfat und es wurde während 2 Stunden bei Raumtemperatur gerührt. Dann wurden 300 mg Essigsäure zugefügt, wobei das gebildete 5-(2-Chlor-5-methoxy-phenoxy)-4,6-dihydroxy-2-methylsulfanyl-pyrimidin ausfiel. MS: 314 (M).

c) Dieses 4,6-Dihydroxypyrimidin wurde in Analogie zu Beispiel 1e) in die 4,6-Dichlor-Verbindung umgewandelt. Smp. 113-114°C.

d) In Analogie zu Beispiel 1, Abschnitt g) erhielt man aus 500 mg der Dichlor-Verbindung und 547 mg (1,3-Benzodioxol-5-sulfonamid)-K das N-[6-Chlor-5-(2-chlor-5-methoxy-phenoxy)-2-methylsulfanyl-pyrimidin-4-yl]-1,3-benzodioxol-5-sulfonamid. Smp. 110-112°C (aus EtOH).

e) In Analogie zu Beispiel 1, Abschnitt h) erhielt man aus 350 mg der Verbindung aus 13d) und Na-Glykolat in Ethylenglykol das N-[5-(2-Chlor-5-methoxy-phenoxy)-6-(2-hydroxy-ethoxy)-2-methylsulfanyl-pyrimidin-4-yl]-1,3-benzodioxol-5-sulfonamid. MS: 542 (M).

Beispiel 14

In Analogie zu Beispiel 2 erhielt man aus 90 mg N-[(2-Chlor-5-methoxy-phenoxy)-6-(2-hydroxy-ethoxy)-2-methoxymethyl-pyrimidin-4-yl]-1,3-benzodioxol-5-sulfonamid und 89 mg Thiophen-3-carbonsäureazid den Thiophen-3-ylcarbaminsäure 2-[6-(1,3-benzodioxol-5-ylsulfonylamino)-5-(2-chlor-5-methoxy-phenoxy)-2-methoxymethyl-pyrimidin-4-yloxy]-ethylester. MS: 665,1 $(M+H^+)$.

Beispiel 15

a) In Analogie zu Beispiel 2 erhielt man aus 150 mg N-[(2-Chlor-5-methoxy-phenoxy)-6-(2-hydroxy-ethoxy)-pyrimidin-4-yl]-1,3-benzodioxol-5-sulfonamid und 139 mg Thiophen-2-carbonsäureazid den Thiophen-2-ylcarbamylsäure 2-[6-(1,3-benzodioxol-5-ylsulfonylamino)-5-(2-chlor-5-methoxy-phenoxy)-pyrimidin-4-yloxy]-ethylester. MS: 621,3 $(M+H^+)$, Smp. 175°C (Zers.).

b) Das Thiophen-2-carbonsäureazid wurde nach dem Verfahren des Beispiels 2, Abschnitt e) aus Thiophen-2-carbonsäure hergestellt.

Beispiel 16

In Analogie zu Beispiel 2 erhielt man aus 125 mg N-[(2-Chlor-5-methoxy-phenoxy)-6-(2-hydroxy-ethoxy)-2-(methoxyethyl)-pyrimidin-4-yl]-1,3-benzodioxol-5-sulfonamid und 103 mg Thiophen-3-carbonsäureazid den Thiophen-3-ylcarbaminsäure 2-[6-(1,3-benzodioxol-5-ylsulfonylamino)-5-(2-chlor-5-methoxy-phenoxy)-2-(2-methoxy-ethyl)-pyrimidin-4-yloxy]-ethylester. MS: 679,3 $(M+H^+)$.

Beispiel 17

a) In Analogie zu Beispiel 2 erhielt man aus 200 mg N-[6-(2-Hydroxy-ethoxy)-5-(2-methoxy-phenoxy)-2-(4-methoxy-phenyl)-pyrimidin-4-yl]-1,3-benzodioxol-5-sulfonamid und 195 mg 2-Pyridyl-carbonsäureazid den Pyridin-2-ylcarbaminsäure 2-[6-(1,3-benzodioxol-5-ylsulfonylamino)-5-(2-methoxy-phenoxy)-2-(4-methoxy-phenyl)-pyrimidin-4-yloxy]-ethylester. Smp. 217-218°C (aus EtOH).

Herstellung der Ausgangsverbindung

b) In Analogie zu Beispiel 5, Abschnitt b) erhielt man aus 4-Methoxy-phenylcyanid, Methanol und HCl das 4-Methoxyphenyl-methylimidoester-hydrochlorid. MS: 165 (M).
c) Diese Verbindung wurde mit $NH_3$ zum 4-Methoxy-phenylamidin-hydrochlorid umgesetzt. MS: 150 (M).
d) In Analogie zu Beispiel 1, Abschnitt d) erhielt man aus 7,63 g (2-Methoxy-phenoxy)-dimethylmalonat und 5,6 g (4-Methoxy-phenyl)-amidin-hydrochlorid das 2-(4-Methoxyphenyl)-5-(2-methoxy-phenoxy)-4,6(1H,5H)-pyrimidindion. MS: 340 (M).
e) In Analogie zu Beispiel 1, Abschnitt c) erhielt man das 4,6-Dichlor-2-(4-methoxy-phenyl)-5-(2-methoxy-phenoxy)-pyrimidin. Smp. 113-114°C (aus EtOH).
f) In Analogie zu Beispiel 1, Abschnitt g) erhielt man aus 755 mg der Verbindung aus 17e) und 765 mg (1,3-Benzodioxol-5-sulfonamid)-K das N-[6-Chlor-5-(2-methoxy-phenoxy)-2-(4-methoxy-phenyl)-pyrimidin-4-yl]-1,3-benzodioxol-5-sulfonamid. Smp. 187-188°C (aus EtOH).
g) In Analogie zu Beispiel 1, Abschnitt h) erhielt man aus 542 mg der Verbindung aus 17f) und Na-Glykolat das N-[6-(2-Hydroxy-ethoxy)-5-(2-methoxy-phenoxy)-2-(4-methoxy-phenyl)-pyrimidin-4-yl]-1,3-benzodioxol-5-sulfonamid. MS: 567 (M).

Beispiel 18

a) In Analogie zu Beispiel 2 erhielt man aus 200 mg N-[6-(2-Hydroxy-ethoxy)-5-(2-methoxy-phenoxy)-2-phenyl-pyrimidin-4-yl]-1,3-benzodioxol-5-sulfonamid und 165 mg 2-Pyridyl-carbonsäureazid den Pyridin-2-ylcarbaminsäure 2-[6-(1,3-benzodioxol-5-ylsulfonylamino)-5-(2-methoxy-phenoxy)-2-phenyl-pyrimidin-4-yloxy]-ethylester. Smp. 187°C (aus EtOH).

Herstellung der Ausgangsverbindung

b) In Analogie zu Beispiel 1, Abschnitt g) erhielt man aus 1,4 g 4,6-Dichlor-5-(2-methoxy-phenoxy)-2-phenyl-pyrimidin und 1,9 g (1,3-Benzodioxol-5-sulfonamid)-K das N-[6-Chlor-5-(2-methoxy-phenoxy)-2-phenyl-pyrimidin-4-yl]-1,3-benzodioxol-5-sulfonamid. Smp. 185-186°C.
c) In Analogie zu Beispiel 1, Abschnitt h) erhielt man aus 1,5 der Verbindung aus 18b) und Na-Glykolat das N-[6-(2-Hydroxy-ethoxy)-5-(2-methoxy-phenoxy)-2-phenyl-pyrimidin-4-yl]-1,3-benzodioxol-5-sulfonamid. Smp. 159,5-160°C.

Beispiel 19

a) In Analogie zu Beispiel 2 erhielt man aus 125 mg N-[6-(2-Hydroxy-ethoxy)-5-(3,5-dimethoxy-phenoxy)-pyrimidin-4-yl]-1,3-benzodioxol-5-sulfonamid und 75 mg 2-Pyridyl-carbonsäureazid den Pyridin-2-ylcarbamylsäure 2-[6-(1,3-benzodioxol-5-ylsulfonylamino)-5-(3,5-dimethoxy-phenoxy)-pyrimidin-4-yloxy]-ethylester. MS: 612,3 ($M+H^+$), Smp. 212°C (Zers.).

Herstellung der Ausgangsverbindung

b) In Analogie zu Beispiel 1, Abschnitt g) erhielt man aus 1,2 g 4,6-Dichlor-5-(3,5-dimethoxy-phenoxy)-pyrimidin und 1,45 g (1,3-Benzodioxol-5-sulfonamid)-K das N-[6-Chlor-5-(3,5-dimethoxy-phenoxy)-pyrimidin-4-yl]-1,3-benzodioxol-5-sulfonamid. Smp. 167-168°C.
c) In Analogie zu Beispiel 1, Abschnitt h) erhielt man aus 0,31 g der Verbindung aus 19b) das N-[6-(2-Hydroxy-ethoxy)-5-(3,5-dimethoxy-phenoxy)-pyrimidin-4-yl]-1,3-benzodioxol-5-sulfonamid. Smp. 182°C (aus Essigester).

Beispiel 20

a) In Analogie zu Beispiel 1, Abschnitt a) erhielt man aus 0,044 ml 3-Fluorphenylisocyanat und 100 mg p-tert-Butyl-N-[5-(2-chloro-5-methoxy-phenoxy)-6-(2-hydroxyethoxy)-4-pyrimidinyl]benzolsulfonamid den 3-Fluoro-phenylcarbaminsäure 2-[6-(4-tert-butylphenylsulfonylamino)-5-(2-chloro-5-methoxy-phenoxy)-pyrimidin-4-yloxy]ethylester. MS: 644 (M).

Herstellung der Ausgangsverbindungen

b) In Analogie zu Beispiel 1g) erhielt man aus 4 g 4,6-Dichloro-5-(2-chlor-5-methoxy-phenoxy)pyrimidin aus Beispiel 1e) und 7,1 g (4-tert-Butyl-benzolsulfonamid)-K das p-tert-Butyl-N-[(6-chloro-5-(2-chloro-5-methoxy-phenoxy)-4-pyrimidinyl]benzolsulfonamid. Smp. 152-153°C.

c) In Analogie zu Beispiel 1h) erhielt man aus 2,55 g des Chlorids von Beispiel 20b) und 0,85 g Natrium in 30 ml Ethylenglykol das p-tert-Butyl-N-[5-(2-chloro-5-methoxyphenoxy)-6-(2-hydroxyethoxy)-4-pyrimidinyl]benzolsulfonamid. Smp. 140°C.

Beispiel 21

In Analogie zu Beispiel 1, Abschnitt a) erhielt man aus 0,044 ml 2-Fluorophenylisocyanat und 100 mg p-tert-Butyl-N-[5-(2-chloro-5-methoxyphenoxy)-6-(2-hydroxyethoxy)-4-pyrimidinyl]benzolsulfonamid den 2-Fluorophenylcarbaminsäure 2-[(6-(4-tert-butyl-phenylsulfonylamino)-5-(2-chloro-5-methoxy-phenoxy)-pyrimidin-4-yloxy]ethylester. MS: 644 (M).

Beispiel 22

In Analogie zu Beispiel 1, Abschnitt a) erhielt man aus 0,042 ml Phenylisocyanat und 100 mg p-tert-Butyl-N-[5-(2-chloro-5-methoxyphenoxy)-6-(2-hydroxyethoxy)-4-pyrimidinyl]benzolsulfonamid den Phenylcarbaminsäure 2-[6-(4-tert-butyl-phenylsulfonylamino)-5-(2-chloro-5-methoxyphenoxy)pyrimidin-4-yloxy]ethylester. MS: 627,4 (M+H).

Beispiel 23

In Analogie zu Beispiel 1, Abschnitt a) erhielt man aus 60 mg 4-Chlor-phenylisocyanat und 100 mg p-tert-Butyl-N-[5-(2-chloro-5-methoxyphenoxy)-6-(2-hydroxyethoxy)-4-pyrimidinyl]benzolsulfonamid den 4-Chloro-phenylcarbaminsäure 2-[6-(4-tert-butyl-phenylsulfanoylamino)-5-(2-chloro-5-methoxy-phenoxy)-pyrimidin-4-yloxy]ethylester. MS: 661,3 (M+H).

Beispiel 24

In Analogie zu Beispiel 1, Abschnitt a) erhielt man aus 50 mg 3-Methoxyphenylisocyanat und 100 mg p-tert-Butyl-N-[5-(2-chloro-5-methoxyphenoxy)-6-(2-hydroxyethoxy)-4-pyrimidinyl]benzolsulfonamid den 3-Methoxy-phenylcarbaminsäure 2-[6-(4-tert-butyl-phenylsulfonylamino)-5-(2-chloro-5-methoxy-phenoxy)-pyrimidin-4-yloxy]ethylester. MS: 657,3 (M+H).

Beispiel 25

In Analogie zu Beispiel 1, Abschnitt a) erhielt man aus 0,056 ml 4-Trifluoromethylphenylisocyanat und 100 mg p-tert-Butyl-N-[5-(2-chloro-5-methoxyphenoxy)-6-(2-hydroxyethoxy)-4-pyrimidinyl]benzolsulfonamid den 4-Trifluoromethyl-phenylcarbaminsäure 2-[6-(4-tert-butyl-phenylsulfonylamino)-5-(2-chloro-5-methoxy-phenoxy)-pyrimidin-4-yloxy]ethylester. MS: 695,3 (M+H).

Beispiel 26

In Analogie zu Beispiel 1, Abschnitt a) erhielt man aus 69 mg 2-Carbomethoxyphenylisocyanat und 100 mg p-tert-Butyl-N-[5-(2-chloro-5-methoxy-phenoxy)-6-(2-hydroxyethoxy)-4-pyrimidinyl]benzolsulfonamid den 2-[2-[6-(4-tert-Butyl-phenylsulfonylamino)-5-(2-chloro-5-methoxy)-pyrimidin-4-yloxy]-ethoxycarbonylamino]-benzoesäure-methylester. MS: 685,3 (M+H).

Beispiel 27

In Analogie zu Beispiel 1, Abschnitt a) erhielt man aus 0,05 ml 3-Methylphenylisocyanat und 100 mg p-tert-Butyl-N-[5-(2-chloro-5-methoxy-phenoxy)-6-(2-hydroxyethoxy)-4-pyrimidinyl]benzolsulfonamid den 3-Tolylcarbaminsäure 2-(6-(4-tert-butyl-phenylsulfonylamino)-5-(2-chloro-5-methoxy-phenoxy)-pyrimidin-4-yloxy]ethylester. MS: 640 (M).

Beispiel 28

In Analogie zu Beispiel 2, Abschnitt a) erhielt man aus 58 mg 2-Pyridylcarbonsäureazid und 100 mg p-tert-Butyl-N-[5-(2-chloro-5-methoxyphenoxy)-6-(2-hydroxyethoxy)-4-pyrimidinyl]benzolsulfonamid den Pyridin-2-ylcarbaminsäure 2-[6-(4-tert-butylphenylsulfonylamino)-5-(2-chloro-5-methoxy-phenoxy)-pyrimidin-4-yloxy]ethylester. MS: 628,4 (M+H).

Beispiel 29

In Analogie zu Beispiel 2, Abschnitt a) erhielt man aus 59 mg 2-Pyrazincarbonsäureazid und 100 mg p-tert-Butyl-N-[5-(2-chloro-5-methoxy-phenoxy)-6-(2-hydroxyethoxy)-4-pyrimidinyl]benzolsulfonamid den Pyrazin-2-ylcarbaminsäure 2-[6-(4-tert-butylphenylsulfonylamino)-5-(2-chloro-5-methoxy-phenoxy)-pyrimidin-4-yloxy]ethylester. MS: 408 ($M-C_5H_4N_3O_3SCl$).

Beispiel 30

In Analogie zu Beispiel 2, Abschnitt a) erhielt man aus 58 mg 3-Pyridylcarbonsäureazid und 100 mg p-tert-Butyl-N-[5-(2-chloro-5-methoxyphenoxy)-6-(2-hydroxyethoxy)-4-pyrimidinyl]benzolsulfonamid den Pyridin-3-ylcarbaminsäure 2-[6-(4-tert-butylphenylsulfonylamino)-5-(2-chloro-5-methoxy-phenoxy)-pyrimidin-4-yloxy]ethylester. MS: 628,4 (M+H).

Beispiel 31

In Analogie zu Beispiel 2, Abschnitt a) erhielt man aus 58 mg 4-Pyridylcarbonsäureazid und 100 mg p-tert-Butyl-N-[5-(2-chloro-5-methoxyphenoxy)-6-(2-hydroxyethoxy)-4-pyrimidinyl]benzolsulfonamid den Pyridin-4-ylcarbaminsäure 2-[6-(4-tert-butylphenylsulfonylamino)-5-(2-chloro-5-methoxy-phenoxy)-pyrimidin-4-yloxy]ethylester.

Beispiel 32

In Analogie zu Beispiel 1, Abschnitt a) erhielt man aus 0,052 ml 2-Methoxyphenylisocyanat und 100 mg p-tert-Butyl-N-[5-(2-chloro-5-methoxy-phenoxy)-6-(2-hydroxyethoxy)-4-pyrimidinyl]benzolsulfonamid den 2-Methoxy-phenoxycarbaminsäure 2-[6-(4-tert-butylphenylsulfonylamino)-5-(2-chloro-5-methoxy-phenoxy)-pyrimidin-4-yloxy]ethylester. MS: 656 (M).

Beispiel 33

In Analogie zu Beispiel 2, Abschnitt a) erhielt man aus 2-Acetoxy-phenylcarbonsäureazid und 100 mg p-tert-Butyl-N-[5-(2-chloro-5-methoxy-phenoxy)-6-(2-hydroxyethoxy)-4-pyrimidinyl]benzolsulfonamid den Essigsäure 2-[2-[(6-tert-butyl-phenylsulfonylamino)-5-(2-chloro-5-methoxy)-pyrimidin-4-yloxy]-ethoxycarbonylamino]-phenylester. MS: 642 ($M-C_2H_2O$).

Beispiel 34

90 mg der Verbindung aus Beispiel 33 wurden in 5 ml Ethanol gelöst, mit 1 ml 6N HCl versetzt und 6 Stunden am Rückfluss erhitzt. Das Lösungsmittel wurde abgezogen, das Rohprodukt zwischen Essigester und Wasser verteilt. Die organische Phase wurde getrocknet, das Lösungsmittel am Rotationsverdampfer entfernt und der Rückstand an Kieselgel chromatographiert mit $CH_2Cl_2$/Essigester (6:1) als Laufmittel. Man erhielt so den 2-Hydroxy-phenylcarbaminsäure 2-[6-(4-tert-butylphenylsulfonylamino)-5-(2-chloro-5-methoxy-phenoxy)-pyrimidin-4-yloxy]ethylester. MS: 428 ($M-C_7H_5NO_4SCl$).

Beispiel 35

In Analogie zu Beispiel 1, Abschnitt a) erhielt man aus 0,048 ml Benzylisocyanat und 100 mg p-tert-Butyl-N-[5-(2-chloro-5-methoxyphenoxy)-6-(2-hydroxyethoxy)-4-pyrimidinyl]benzolsulfonamid den Benzylcarbaminsäure 2-[6-(4-tert-butylphenylsulfonylamino)-5-(2-chloro-5-methoxy-phenoxy)pyrimidin-4-yloxy]ethylester. MS: 641 (M+H).

Beispiel 36

In Analogie zu Beispiel 1, Abschnitt a) erhielt man aus 0,038 ml Isopropylisocyanat und 100 mg p-tert-Butyl-N-[5-(2-chloro-5-methoxy-phenoxy)-6-(2-hydroxyethoxy)-4-pyrimidinyl]benzolsulfonamid den Isopropylcarbaminsäure 2-[6-(4-tert-butyl-phenylsulfonylamino)-5-(2-chloro-5-methoxy-phenoxy)-pyrimidin-4-yloxy]ethylester. MS: 592 (M).

Beispiel 37

In Analogie zu Beispiel 1, Abschnitt a) erhielt man aus 0,025 ml Ethylsocyanat und 75 mg p-tert-Butyl-N-[5-(2-chloro-5-methoxyphenoxy)-6-(2-hydroxyethoxy)-4-pyrimidinyl]benzolsulfonamid den Ethylcarbaminsäure 2-[6-(4-tert-butyl-phenylsulfonylamino)-5-(2-chloro-5-methoxy-phenoxy)-pyrimidin-4-yloxy]-ethylester. MS: 579 (M).

Beispiel 38

100 mg der Verbindung von Beispiel 28 löste man in 15 ml $CH_2Cl_2$, versetzte mit 60 mg 3-Chlorperbenzoesäure und erhitzte anschliessend 12 Stunden am Rückfluss. Anschliessend wurde auf Wasser gegossen und mit $CH_2Cl_2$ extrahiert. Die organische Phase wurde getrocknet und schliesslich am Rotationsverdampfer entfernt. Den Rückstand chromatographierte man an Kieselgel mit $CH_2Cl_2$/MeOH (20:1) als Laufmittel. Man erhielt so den 1-Oxy-pyridin-2-ylcarbaminsäure 2-[6-(4-tert-butyl-phenylsulfonylamino)-5-(2-chloro-5-methoxy-phenoxy)-pyrimidin-4-yloxy]-ethylester. MS: 644,4 (M+H).

Beispiel 39

In Analogie zu Beispiel 38 erhielt man aus 60 mg der in Beispiel 31 hergestellten Verbindung und 33 mg 3-Chlorperbenzoesäure den 1-Oxy-pyridin-4-ylcarbaminsäure 2-[6-(4-tert-butyl-phenylsulfonylamino)-5-(2-chloro-5-methoxy-phenoxy)-pyrimidin-4-yloxy]-ethylester. MS: 644,5 (M+H).

Beispiel 40

Durch Alkylierung der Verbindung aus Beispiel 1, Abschnitt a) mit Methyljodid erhielt man das 3-[2-[5-(2-Chloro-5-methoxy-phenoxy)-6-(2,3-dihydro-1,4-benzodioxin-6-ylsulfonylamino)-pyrimidin-4-yloxy]-ethoxycarbonylamino]-1-methyl-pyridiniumjodid.

Beispiel 41

Durch Deprotonierung der Verbindung in Beispiel 40 mit Natriummethylat als Base wurde das N-[5-(2-Chloro-5-methoxy-phenoxy)-6-[2-(1-methyl-pyridin-3-yliocarbamoyloxy)-ethoxy]-pyrimidin-4-yl]-1,3-benzodioxol- 5-sulfonamid erhalten.

Beispiel 42

In Analogie zu Beispiel 1, Abschnitt a) erhielt man aus 0,05 ml Phenylisocyanat und 100 mg p-tert-Butyl-N-[6-(2-hydroxyethoxy)-5-(m-methoxy-phenoxy)-4-pyrimidinyl]benzolsulfonamid (vgl. EP-A-526708) den Phenylcarbaminsäure 2-[6-(4-tert-butyl-phenylsulfonylamino)-5-(3-methoxy-phenoxy)-pyrimidin-4-yloxy]-ethylester. MS: 593,4 (M+H).

Beispiel 43

In Analogie zu Beispiel 2, Abschnitt a) erhielt man aus 62 mg 2-Pyridylcarbonsäureazid und 100 mg p-tert-Butyl-N-[6-(2-hydroxyethoxy)-5-(m-methoxyphenoxy)-4-pyrimidinyl]benzolsulfonamid den Pyridin-2-ylcarbaminsäure 2-[6-(4-tert-butyl-phenylsulfonylamino)-5-(3-methoxy-phenoxy)-pyrimidin-4-yloxy]-ethylester. MS: 594,5 (M+H).

Beispiel 44

In Analogie zu Beispiel 2, Abschnitt a) erhielt man aus 62 mg 4-Pyridylcarbonsäureazid und 100 mg p-tert-Butyl-N-[6-(2-hydroxyethoxy)-5-(m-methoxyphenoxy)-4-pyrimidinyl]benzolsulfonamid den Pyridin-4-ylcarbaminsäure 2-[6-(4-tert-butyl-phenylsulfonylamino)-5-(3-methoxy-phenoxy)-pyrimidin-4-yloxy]-ethylester. MS: 594,5 (M+H).

Beispiel 45

In Analogie zu Beispiel 2, Abschnitt a) erhielt man aus 84 mg Chinaldinsäureazid und 100 mg p-tert-Butyl-N-[6-(2-hydroxyethoxy)-5-(m-methoxyphenoxy)-4-pyrimidinyl]benzolsulfonamid den Chinolin-2-ylcarbaminsäure-2-[6-(4-tert-butyl-phenylsulfonylamino)-5-(3-methoxy-phenoxy)-pyrimidin-4-yloxy]-ethylester. MS: 644,3 (M+H).

Beispiel 46

In Analogie zu Beispiel 1, Abschnitt a) erhielt man aus 0,062 ml (R)-1-Phenyl-ethylisocyanat und 100 mg p-tert-Butyl-N-[6-(2-hydroxyethoxy)-5-(m-methoxyphenoxy)-4-pyrimidinyl]benzolsulfonamid den (R)-1-Phenyl-ethylcarbaminsäure-2-[6-(4-tert-butyl-phenylsulfonylamino)-5-(3-methoxy-phenoxy)-pyrimidin-4-yloxy]-ethylester. MS: 621 (M+H).

Beispiel 47

In Analogie zu Beispiel 1, Abschnitt a) erhielt man aus 0,05 ml Cyclohexylisocyanat und 100 mg p-tert-Butyl-N-[6-(2-hydroxyethoxy)-5-(m-methoxyphenoxy)-4-pyrimidinyl]benzolsulfonamid den Cyclohexylcarbaminsäure 2-[6-(4-tert-butyl-phenylsulfonylamino)-5-(3-methoxy-phenoxy)-pyrimidin-4-yloxy]-ethylester. MS: 599,4 (M+H).

Beispiel 48

In Analogie zu Beispiel 1, Abschnitt a) erhielt man aus 0,24 ml Isocyanatoessigsäureethylester und 500 mg p-tert-Butyl-N-[6-(2-hydroxy-ethoxy)-5-(m-methoxyphenoxy)-4-pyrimidinyl]benzolsulfonamid den [2-[6-(4-tert-Butyl-phenylsulfonylamino)-5-(3-methoxy-phenoxy)-pyrimidin-4-yloxy]-ethoxycarbonylamino]-essigsäureethylester. MS: 603,5 (M+H).

Beispiel 49

410 mg [2-[6-(4-tert-Butyl-phenylsulfonylamino)-5-(3-methoxy-phenoxy)-pyrimidin-4-yloxy]-ethoxycarbonylamino]-essigsäureethylester in 20 ml Ethanol versetzte man mit 1,36 ml 1N NaOH und erhitzte die Reaktionslösung 2 Stunden bei 60°C. Anschliessend versetzte man mit 1,36 ml 1N HCl und engte am Rotationsverdampfer ein. Der Rückstand wurde an Kieselgel chromatographiert mit $CH_2Cl_2$/MeOH (10:1) als Laufmittel. Man erhielt so 300 mg 2-[6-(4-tert-Butyl-phenylsulfonylamino)-5-(3-methoxy-phenoxy)-pyrimidin-4-yloxy]-ethoxycarbonylamino-essigsäure. MS: 573,3 (M-H).

Beispiel 50

100 mg [2-[6-(4-tert-Butyl-phenylsulfonylamino)-5-(3-methoxy-phenoxy)-pyrimidin-4-yloxy]-ethoxycarbonylamino]-essigsäureethylester löste man in 3 ml Ethanol, versetzte mit 3 ml THF und gab anschliessend 36 mg $CaCl_2$ und 25 mg $NaBH_4$ bei Raumtemperatur zu. Man liess 2 Stunden bei Raumtemperatur rühren, engte am Rotationsverdampfer ein und verteilte den Rückstand zwischen verdünnter Zitronensäure und Essigester. Die organische Phase wurde getrocknet, das Lösungsmittel entfernt und der Rückstand an Kieselgel mit $CH_2Cl_2$ (20:1) als Laufmittel chromatographiert. Man erhielt so 78 mg 2-Hydroxy-ethylcarbaminsäure 2-[6-(4-tert-butyl-phenylsulfonylamino)-5-(3-methoxy-phenoxy)-pyrimidin-4-yloxy]-ethylester. MS: 561 (M+H).

Beispiel 51

In Analogie zu Beispiel 1, Abschnitt a) erhielt man aus 0,15 ml Morpholin-4-carbonylchlorid und 200 mg p-tert-Butyl-N-[6-(2-hydroxy-ethoxy)-5-(m-methoxyphenoxy)-4-pyrimidinyl]benzolsulfonamid, unter Zusatz von 154 mg Dimethylaminopyridin bei einer Reaktionszeit von 12 Stunden, den Morpholin-4-carbonsäure 2-[6-(4-tert-butyl-phenylsulfonylamino)-5-(3-methoxy-phenoxy)-pyrimidin-4-yloxy]-ethylester. MS: 587,4 (M+H).

Beispiel 52

100 mg 2-[6-(4-tert-Butyl-phenylsulfonylamino)-5-(3-methoxy-phenoxy)-pyrimidin-4-yloxy]-ethoxycarbonylamino-essigsäure in 15 ml $CH_2Cl_2$ versetzte man mit 0,015 ml Morpholin, 22 mg Dimethylaminopyridin, 33 mg N-Aethyl-N'-(3-dimethylaminopropyl)-carbodiimid-hydrochlorid und rührte die Lösung über Nacht bei Raumtemperatur. Anschliessend engte man am Rotationsverdampfer ein, verteilte den Rückstand zwischen Essigester und $H_2O$. Nach Trocknen der organischen Phase entfernte man das Lösungsmittel am Rotationsverdampfer und chromatographierte den Rückstand an Kieselgel mit $CH_2Cl_2$/MeOH (40:1) als Laufmittel. Man erhielt so 80 mg 2-Morpholin-4-yl-2-oxo-ethylcarbaminsäure

2-[6-(4-tert-butyl-phenylsulfonylamino)-5-(3-methoxy-phenoxy)-pyrimidin-4-yloxy]-ethylester. MS: 644,5 (M+H).

Beispiel 53

In Analogie zu Beispiel 52 erhielt man aus 100 mg 2-[6-(4-tert-Butyl-phenylsulfonylamino)-5-(3-methoxy-phenoxy)-pyrimidin-4-yloxy]-ethoxy-carbonylamino-essigsäure und 0,014 ml Pyrrolidin den 2-Oxo-2-pyrrolidin-1-yl-ethylcarbaminsäure 2-[6-(4-tert-butyl-phenylsulfonylamino)-5-(3-methoxy-phenoxy)-pyrimidin-4-yloxy]ethylester. MS: 628,5 (M+H).

Beispiel 54

In Analogie zu Beispiel 52 erhielt man aus 100 mg 2-[6-(4-tert-Butyl-phenylsulfonylamino)-5-(3-methoxy-phenoxy)-pyrimidin-4-yloxy]-ethoxy-carbonylamino-essigsäure und 0,016 ml Anilin den Phenylcarbamoylmethylcarbaminsäure 2-[6-(4-tert-butyl-phenylsulfonylamino)-5-(3-methoxy-phenoxy)-pyrimidin-4-yloxy]ethylester. MS: 650,5 (M+H).

Beispiel 55

In Analogie zu Beispiel 1, Abschnitt a) erhielt man aus 100 mg p-tert-Butyl-N-[6-(2-hydroxyethoxy)-5-(m-methoxy-phenoxy)-4-pyrimidinyl]benzolsulfonamid und 78 mg Ethyl-2-isocyanato-4-methyl-valerat den 2-[2-[6-(4-tert-Butyl-phenylsulfonylamino)-5-(3-methoxy-phenoxy)-pyrimidin-4-yloxy]-ethoxycarbonylamino]-4-methyl-pentansäureethylester. MS: 659 (M+H).

Beispiel 56

Durch Alkylierung von Pyridin-4-ylcarbaminsäure 2-[6-(4-tert-butylphenylsulfonylamino)-5-(2-chloro-5-methoxy-phenoxy)-pyrimidin-4-yloxy]ethylester mit Methyljodid erhielt man das 4-[2-[6-(4-tert-Butyl-phenylsulfonylamino)-5-(2-chloro-5-methoxy-phenoxy-pyrimidin-4-yloxy]-ethoxycarbonylamino]-1-methyl-pyridiniumjodid.

Beispiel 57

Analog zu Beispiel 2, Abschnitt a) erhielt man aus 3-Furylisocyanat und N-[5-(2-Chlor-5-methoxy-phenoxy)-6-(2-hydroxy-ethoxy)-pyrimidin-4-yl]-1,3-benzodioxol-5-sulfonamid den Furan-3-ylcarbaminsäure 2-[6-(1,3-benzodioxol-5-ylsulfonylamino)-5-(2-chloro-5-methoxy-phenoxy)-pyrimidin-4-yloxy]-ethylester.

Beispiel 58

In Analogie zu Beispiel 2, Abschnitt a) erhielt man aus 2-Pyridylcarbonsäureazid und 4-tert-Butyl-N-[6-(2-hydroxyethoxy)-2-isopropyl-5-(2-methoxy-phenoxy)-pyrimidin-4-yl]-benzolsulfonamid (vgl. EP-A-526708) den Pyridin-2-ylcarbaminsäure 2-[6-(4-tert-butyl-phenylsulfonylamino)-2-isopropyl-5-(2-methoxy-phenoxy)-pyrimidin-4-yloxy]-ethylester. Smp. 119-120°C.

Beispiel 59

In Analogie zu Beispiel 2, Abschnitt a) erhielt man aus 4-Pyridylcarbonsäureazid und der Ausgangsverbindung von Beispiel 58 den Pyridin-4-ylcarbaminsäure 2-[6-(4-tert-butyl-phenylsulfonylamino)-2-isopropyl-5-(2-methoxy-phenoxy)-pyrimidin-4-yloxy]-ethylester.

Beispiel 60

In Analogie zu Beispiel 2, Abschnitt a) erhielt man aus 2-Pyridylcarbonsäureazid und p-tert-Butyl-N-[6-(2-hydroxyethoxy)-5-(o-methoxy-phenoxy)-2-propyl-4-pyrimidinyl]benzolsulfonamid (vgl. EP-A-526708) den Pyridin-2-ylcarbaminsäure 2-[6-(4-tert-butyl-phenylsulfonylamino)-5-(2-methoxy-phenoxy)-2-propyl-pyrimidin-4-yloxy]-ethylester. Smp. 147-149°C.

Beispiel 61

In Analogie zu Beispiel 2, Abschnitt a) erhielt man aus 4-Pyridylcarbonsäureazid und der Ausgangsverbindung von Beispiel 60 den Pyridin-4-ylcarbaminsäure 2-[6-(4-tert-butyl-phenylsulfonylamino)-5-(2-methoxy-phenoxy)-2-propyl-pyrimidin-4-yloxy]-ethylester.

Beispiel 62

In Analogie zu Beispiel 2, Abschnitt a) erhielt man aus 2-Pyridylcarbonsäureazid und p-tert-Butyl-N-[2-tert-butyl-6-(2-hydroxyethoxy)-5-(o-methoxyphenoxy)-4-pyrimidinyl]benzolsulfonamid (vgl. EP-A-526708) den Pyridin-2-ylcarbaminsäure 2-[2-tert-butyl-6-(4-tert-butyl-phenylsulfonylamino)-5-(2-methoxy-phenoxy)-pyrimidin-4-yloxy]-ethylester.

Beispiel 63

In Analogie zu Beispiel 2, Abschnitt a) erhielt man aus 4-Pyridylcarbonsäureazid und der Ausgangsverbindung von Beispiel 62 den Pyridin-4-ylcarbaminsäure 2-[2-tert-butyl-6-(4-tert-butylphenylsulfonylamino)-5-(2-methoxy-phenoxy)-pyrimidin-4-yloxy]-ethylester.

Beispiel 64

In Analogie zu Beispiel 2, Abschnitt a) erhielt man aus 2-Pyridylcarbonsäureazid und 4-tert-Butyl-N-[2-cyclopropyl-6-(2-hydroxy-ethoxy)-5-(2-methoxy-phenoxy)-pyrimidin-4-yl]benzolsulfonamid (vgl. EP-A-526708) den Pyridin-2-ylcarbaminsäure 2-[6-(4-tert-butyl-phenylsulfonylamino)-2-cyclopropyl-5-(2-methoxy-phenoxy)-pyrimidin-4-yloxy]-ethylester.

Beispiel 65

In Analogie zu Beispiel 2, Abschnitt a) erhielt man aus 4-Pyridylcarbonsäureazid und der Ausgangsverbindung von Beispiel 64 den Pyridin-4-ylcarbaminsäure 2-[6-(4-tert-butyl-phenylsulfonylamino)-2-cyclopropyl-5-(2-methoxy-phenoxy)-pyrimidin-4-yloxy]-ethylester.

Beispiel 66

In Analogie zu Beispiel 2, Abschnitt a) erhielt man aus 2-Pyridylcarbonsäureazid und 4-tert-Butyl-N-[6-(2-hydroxy-ethoxy)-5-(2-methoxy-phenoxy)-2-(thiophen-2-yl)-pyrimidin-4-yl]benzolsulfonamid (vgl. EP-A-526708) den Pyridin-2-ylcarbaminsäure 2-[6-(4-tert-butyl-phenylsulfonylamino)-5-(2-methoxy-phenoxy)-2-thiophen-2-yl-pyrimidin-4-yloxy]-ethylester. Smp. 175-180°C.

Beispiel 67

In Analogie zu Beispiel 2, Abschnitt a) erhielt man aus 4-Pyridylcarbonsäureazid und der Ausgangsverbindung von Beispiel 66 den Pyrimidin-4-ylcarbaminsäure 2-[6-(4-tert-butyl-phenylsulfonylamino)-5-(2-methoxy-phenoxy)-2-thiophen-2-yl-pyrimidin-4-yloxy]-ethylester. Smp. 188-192°C.

Beispiel 68

In Analogie zu Beispiel 2, Abschnitt a) erhielt man aus 2-Pyridylcarbonsäureazid und p-tert-Butyl-N-[6-(2-hydroxyethoxy)-5-(o-methoxy-phenoxy)-2-methyl-4-pyrimidinyl]benzolsulfonamid (vgl. EP-A-526708) den Pyridin-2-ylcarbaminsäure 2-[6-(4-tert-butyl-phenylsulfonylamino)-5-(2-methoxy-phenoxy)-2-methyl-pyrimidin-4-yloxy]-ethylester. Smp. 169-170°C.

Beispiel 69

In Analogie zu Beispiel 2, Abschnitt a) erhielt man aus 4-Pyridylcarbonsäureazid und der Ausgangsverbindung von Beispiel 68 den Pyridin-4-ylcarbaminsäure 2-[6-(4-tert-butyl-phenylsulfonylamino)-5-(2-methoxy-phenoxy)-2-methyl-pyrimidin-4-yloxy]-ethylester. Smp. 155-158°C.

Beispiel 70

a) In Analogie zu Beispiel 2, Abschnitt a) erhielt man aus 2-Pyridylcarbonsäureazid und 4-tert-Butyl-N-[6-(2-hydroxy-ethoxy)-5-(2-methoxy-phenoxy)-2,2'-bipyrimidin-4-yl]-benzolsulfonamid (vgl. EP-A-526708) den Pyridin-2-ylcarbaminsäure-2-[6-(4-tert-butyl-phenylsulfonylamino)-5-(2-methoxy-phenoxy)-2,2'-bipyrimidin-4-yloxy]-ethylester. Smp. 203-204°C.

Herstellung des Natriumsalzes

b) 414 mg der obigen Verbindung löste man in Dioxan. Anschliessend versetzte man mit der stöchiometrischen Menge an Natriummethylat in 2 ml Methanol, wobei das entsprechende Natriumsalz ausfiel. Man nutschte anschliessend das Salz ab und trocknete es sorgfältig bei 60°C.

| | | | | |
|---|---|---|---|---|
| Ber. | C 57,14 | H 4,65 | N 14,13 | S 4,62 |
| Gef. | C 56,85 | H 4,85 | N 13,94 | S 4,60 |

Herstellung des Hydrochlorids

c) 414 mg der in Beispiel 70a) hergestellten Verbindung löste man in absolutem Dioxan und versetzte mit einem Ueberschuss an Chlorwasserstoff in absolutem Dioxan. Es bildete sich ein amorpher Niederschlag, der abgenutscht wurde und bis zur Gewichtskonstanz bei 30°C im Wasserstrahlvakuum getrocknet wurde.

Beispiel 71

In Analogie zu Beispiel 2, Abschnitt a) erhielt man aus 4-Pyridylcarbonsäureazid und der Ausgangsverbindung von Beispiel 70, Abschnitt a) den Pyridin-4-ylcarbaminsäure 2-[6-(4-tert-butyl-phenylsulfonylamino)-5-(2-methoxy-phenoxy)-2,2'-bipyrimidin-4-yloxy]-ethylester. Smp. 138-141°C.

Beispiel 72

a) In Analogie zu Beispiel 2, Abschnitt a) erhielt man aus 2-Pyridincarbonsäureazid und 4-tert-Butyl-N-[6-(2-hydroxy-ethoxy)-5-(2-methoxy-phenoxy)-2-(morpholin-4-yl)-pyrimidin-4-yl]-benzolsulfonamid den Pyridin-2-ylcarbaminsäure-2-[6-(2-tert-butyl-phenylsulfonylamino)-5-(2-methoxy-phenoxy)-2-morpholin-4-yl-pyrimidin-4-yloxy]-ethylester. MS: 679,3 (M+H); IR: 1735 (KBr, Carbamat).

Herstellung der Ausgangsverbindung

b) In Analogie zu Beispiel 1, Abschnitt d) wurde aus (2-Methoxy-phenoxy)-malonsäuredimethylester (EP-A-526708) und Morpholin-formamidin das 5-(2-Methoxy-phenoxy)-2-(morpholin-4-yl)-4,6(1H,5H)-pyrimidindion hergestellt. Daraus wurde mit $POCl_3$ das 4,6-Dichloro-5-(2-methoxy-phenoxy)-2-(morpholin-4-yl)pyrimidin, daraus mit (4-tert-Butylbenzolsulfonamid)-K das [4-tert-Butyl-N-[6-chloro-5-(2-methoxyphenoxy)-2-(morpholin-4-yl)-pyrimidin-4-yl]-benzolsulfonamid] und schliesslich mit Na-Aethylenglykolat das 4-tert-Butyl-N-[6-(2-hydroxy-ethoxy)-5-(2-methoxy-phenoxy)-2-(morpholin-4-yl]-benzolsulfonamid erhalten.

Beispiel 73

In Analogie zu Beispiel 2, Abschnitt a) erhielt man aus 4-Pyridylcarbonsäureazid und 4-tert-Butyl-N-[6-(2-hydroxy-ethoxy)-5-(2-methoxy-phenoxy)-2-(morpholin-4-yl-pyrimidin-4-yl]-benzolsulfonamid den Pyridin-4-ylcarbaminsäure 2-[6-(2-tert-butyl-phenylsulfonylamino)-5-(2-methoxy-phenoxy)-2-morpholin-4-yl-pyrimidin-4-yloxy]-ethylester. MS: 679,5 (M+H); IR: 1739 (KBr, Carbamat).

Beispiel 74

In Analogie zu Beispiel 2, Abschnitt a) erhielt man aus 3-Pyridylcarbonsäureazid und 4-tert-Butyl-N-[6-(2-hydroxy-ethoxy)-5-(2-methoxy-phenoxy)-2-(morpholin-4-yl-pyrimidin-4-yl]-benzolsulfonamid den Pyridin-3-ylcarbaminsäure 2-[6-(2-tert-butyl-phenylsulfonylamino)-5-(2-methoxy-phenoxy)-2-morpholin-4-yl-pyrimidin-4-yloxy]-ethylester. MS: 679,7 (M+H); IR: 1735 (KBr, Carbamat).

Beispiel 75

In Analogie zu Beispiel 2, Abschnitt a) erhielt man aus 2-Furancarbonsäureazid und 4-tert-Butyl-N-[6-(2-hydroxy-ethoxy)-5-(2-methoxy-phenoxy-2-(morpholin-4-yl-pyrimidin-4-yl]-benzolsulfonamid den Furan-2-ylcarbaminsäure 2-[6-(4-tert-butyl-phenylsulfonylamino)-5-(2-methoxy-phenoxy)-2-morpholin-4-yl-pyrimidin-4-yloxy]-ethylester. MS: 668,2 (M+H); IR: 1718 (KBr, Carbamat).

Beispiel 76

In Analogie zu Beispiel 2, Abschnitt a) erhielt man aus 3-Methyl-isoxazol-5-yl-carbonsäureazid und 4-tert-Butyl-N-[6-(2-hydroxy-ethoxy)-5-(2-methoxy-phenoxy)-2-(morpholin-4-yl-pyrimidinyl-4-yl]-benzolsulfonamid den 3-Methyl-isoxazol-5-yl-carbaminsäure 2-[6-(4-tert-butyl-phenylsulfonylamino)-5-(2-methoxy-phenoxy)-2-morpholin-4-yl-pyrimidin-4-yloxy]-ethylester. Smp. 103-107°C.

Das eingesetzte Carbonsäureazid erhielt man aus 3-Methyl-isoxazol-5-yl-carbonsäure (Tetrahedron Letters, 327, 1967) und $NaN_3$ in Analogie zu Beispiel 2, Abschnitt e).

Beispiel 77

a) In Analogie zu Beispiel 2, Abschnitt a) erhielt man aus 2-Pyridylcarbonsäureazid und 4-Cyclopropyl-N-[6-(2-hydroxy-ethoxy)-5-(2-methoxy-phenoxy)-2,2'-bipyrimidin-4-yl]-benzolsulfonamid den Pyridin-2-yl-carbaminsäure 2-[6-(4-cyclopropyl-phenylsulfonylamino)-5-(2-methoxy-phenoxy)-2,2'-bipyrimidin-4-yloxy]-ethylester. MS: 656 (M+H); IR: 1732 (Carbamat).

Herstellung der Ausgangsverbindung:

b) 4-Cyclopropylanilin (Bull. Soc. Chim. Belg. 1975, 84, 1197) wurde diazotiert und mit $SO_2/Cu_2Cl_2$ (3 Stunden, Raumtemperatur) zu dem entsprechenden 4-Cyclopropylbenzolsulfonylchlorid umgesetzt. Anschliessende Aminolyse mit Ammoniumhydroxid lieferte das entsprechende 4-Cyclopropylbenzolsulfonamid, Smp. 158-168°C, welches schliesslich mit KOH in EtOH in das Kaliumsalz überführt wurde.

Aus (4-Cyclopropylbenzolsulfonamid)-K und 4,6-Dichloro-5-(2-methoxy-phenoxy)-2,2'-bipyrimidin (vgl. EP-A-526708) wurde 4-Cyclopropyl-N-[6-chlor-5-(2-methoxy-phenoxy)-2-(pyrimidin-2-yl)-pyrimidin-4-yl]-benzolsulfonamid das schliesslich mit Natriumäthylenglykolat umgesetzt wurde.

Beispiel 78

a) In Analogie zu Beispiel 2, Abschnitt a) erhielt man aus 2-Pyridylcarbonylazid und 4-Methylsulfanyl-N-[6-(2-hydroxy-ethoxy)-5-(2-methoxy-phenoxy)-2,2'-bipyrimidin-4-yl]-benzolsulfonamid den Pyridin-2-ylcarbaminsäure 2-[5-(2-methoxy-phenoxy)-6-(4-methylsulsulfanyl-phenylsulfonylamino)-2,2'-bipyrimidin-4-yloxy]-ethylester. Smp. 175-178°C.

Herstellung der Ausgangsverbindung:

b) In Analogie zu Beispiel 1, Abschnitt g) wurde aus (p-Methylthiobenzolsulfonamid)-K und 4,6-Dichloro-5-(2-methoxy-phenoxy)-2,2'-bipyrimidin das 4-Methylsulfanyl-N-[6-chlor-5-(2-methoxy-phenoxy)-2-(pyrimidin-2-yl)-pyrimidin-4-yl]-benzolsulfonamid und daraus mit Natriumäthylenglykolat das 4-Methylsulfonyl-N-[6-(2-hydroxy-ethoxy)-5-(2-methoxy-phenoxy)-2,2'-bipyrimidin-4-yl]-benzolsulfonamid erhalten. Smp. 194-195°C, erhalten.

Beispiel 79

In Analogie zu Beispiel 2, Abschnitt a) erhielt man aus 4-Methylsulfanyl-N-[6-(2-hydroxy-ethoxy)-5-(2-methoxy-phenoxy)-2-(pyrimidin-2-yl)-pyrimidin-4-yl]-benzolsulfonamid und 3-Methyl-isoxazol-5-yl-carbonsäureazid den 3-Methyl-isoxazol-5-ylcarbaminsäure 2-[5-(2-methoxy-phenoxy)-6-(4-methylsulfanyl-phenylsulfonylamino)-2,2'-bipyrimidin-4-yloxy-ethylester. Smp. 187-189°C.

Beispiel 80

a) In Analogie zu Beispiel 2, Abschnitt a) erhielt man aus 2-Pyridylcarbonsäureazid und 4-Vinyl-N-[6-(2-hydroxy-ethoxy)-5-(2-methoxy-phenoxy)-2,2'-bipyrimidin-4-yl]-benzolsulfonamid den Pyridin-2-ylcarbaminsäure 2-[5-(2-methoxy-phenoxy)-6-(4-vinyl-phenylsulfonylamino)-2,2'-bipyridin-4-yloxy]-ethylester. Smp. 166-173°C.

Die Ausgangsverbindung erhielt man durch Umsetzung von (p-Vinylbenzosulfonamid)-K und 4,6-Dichloro-5-(2-methoxy-phenoxy)-2,2'-bipyrimidin zu 4-Vinyl-N-[6-chlor-5-(2-methoxy-phenoxy-2-pyrimidin-2-yl)-pyrimidin-4-yl]-benzolsulfonamid und Umsetzung dieser Verbindung mit Natriumäthylenglycolat. Smp. 182-184°C.

Beispiel 81

a) In Analogie zu Beispiel 2, Abschnitt a) erhielt man aus 4-tert-Butyl-N-[6-(2-hydroxyethoxy)-5-(2-chloro-5-methoxybenzyl)-2-(morpholin-4-yl)-pyrimidin-4-yl]-benzolsulfonamid und 3-Methyl-isoxazol-5-yl-carbonsäureazid den 3-Methyl-isoxazol-5-ylcarbaminsäure 2-[6-(4-tert-butyl-phenylsulfonylamino)-5-(2-chloro-5-methoxy-benzyl)-2-morpholin-4-yl-pyrimidin-4-yloxy]-ethylester. MS: 715,5 (M).

Die Ausgangsverbindung wurde wie folgt synthetisiert:

b) 4-Chloro-m-kresol wurde mit Dimethylsulfat methyliert zu 1-Chloro-4-methoxy-2-methylbenzol. Dieses wurde mit N-Bromosuccinimid zu 2-Bromomethyl-1-chloro-4-methoxybenzol umgesetzt. Anschliessende Alkylierung mit Diethylmalonat lieferte (2-Chloro-5-methoxy-benzyl)-malonsäurediethylester. Dieser wurde in Analogie zu Beispiel 1, Abschnitt d) mit Morpholinformamidin zu 5-(2-Chloro-5-methoxy-benzyl)-2-(morpholin-4-yl)-4,6(1H,5H)-pyrimidindion kondensiert. Anschliessende Chlorierung mit $POCl_3$, analog zu Beispiel 1e), ergab das 4,6-Dichloro-5-(2-chloro-5-methoxy-benzyl)-2-(morpholin-4-yl)-pyrimidin, welches weiter mit (4-tert-Butylbenzolsulfonamid)-K, analog zu Beispiel 1g), zu 4-tert-Butyl-N-[6-chloro-5-(2-chloro-5-methoxy-benzyl)-2-(morpholin-4-yl)-pyrimidin-4-yl]benzolsulfonamid umgesetzt wurde. Anschliessende Umsetzung, analog zu Beispiel 1h), mit Natriumäthylenglykolat ergab schliesslich das 4-tert-Butyl-N-[6-(2-hydroxy-ethoxy)-5-(2-chloro-5-methoxybenzyl)-2-(morpholin-4-yl)pyrimidin-4-yl]-benzolsulfonamid. Smp. 159-162°C.

Beispiel 82

0,1 g 4-tert-Butyl-N-[6-(2-hydroxy-ethoxy)-5-(2-methoxyphenylsulfanyl)-pyrimidin-4-yl]-benzolsulfonamid und 0,1 g 2-Pyridylcarbonsäureazid wurden in 20 ml Toluol während 3 Stunden bei 110°C gerührt und eingeengt. Der Rückstand wurde zwischen Wasser und Chloroform verteilt, die organische Phase getrocknet und eingeengt. Der Rückstand wurde über Kieselgel mit Chloroform gereinigt, und aus Chloroform-Aether umkristallisiert. Man erhielt 0,09 g Pyridin-2-ylcarbaminsäure 2-[6-(4-tert-butyl-phenylsulfonylamino)-5-(2-methoxy-phenylsulfanyl)-pyrimidin-4-yloxy)-ethylester. Smp. 193°C, MS: M = 609.

Das Ausgangsmaterial wurde wie folgt hergestellt:

a) Zu einer Natriummethylatlösung aus 40 ml MeOH und 0,54 g Natrium wurde 0,86 g Formamidinacetat und 2 g (2-Methoxy-phenylsulfanyl)-malonsäuredimethylester (J. Org. Chem. 55, 33-38 [1990]) gegeben. Das Reaktionsgemisch wurde 4 Stunden bei 80°C gerührt und eingeengt. Der Rückstand wurde zwischen Toluol und Wasser verteilt, und die wässrige Phase auf pH 3 gestellt. Der Niederschlag wurde abgenutscht, mit Aether gewaschen und getrocknet. Man erhielt 0,4 g 6-Hydroxy-5-(2-methoxy-phenylsulfanyl)-3,4-dihydro-pyrimidin-4-on. Smp. 291°C.

b) 0,35 g 6-Hydroxy-5-(2-methoxy-phenylsulfanyl)-3,4-dihydro-pyrimidin-4-on wurden in 10 ml Dioxan mit 0,7 ml Hünigs Base und 0,65 ml $POCl_3$ versetzt. Das orange Reaktionsgemisch wurde 20 Stunden bei 80°C gerührt, danach das überschüssige Reagens und Dioxan abdestilliert. Der Rückstand wurde in Dichlormethan aufgenommen und mit Wasser, gesättigte $NaHCO_3$ und Wasser gewaschen. Die organische Phase wurde getrocknet, eingeengt und der Rückstand über Kieselgel mit Dichlormethan gereinigt. Man erhielt 0,27 g 4,6-Dichloro-5-(2-methoxy-phenylsulfanyl)-pyrimidin. Smp. 103°C.

c) 0,24 g 4,6-Dichloro-5-(2-methoxy-phenylsulfanyl)-pyrimidin und 0,415 g (p-t-Butyl-sulfonamid)-Kalium in 5 ml trockenem Dimethylsulfoxid wurden unter Argon 1 Stunde auf 120°C erwärmt. Danach wurde das Reaktionsgemisch mit 50 ml Wasser versetzt und auf pH 1 gestellt. Der Niederschlag wurde abgenutscht, mit Wasser gewaschen und zwischen Essigester und Wasser verteilt. Die organische Phase wurde getrocknet, das Lösungsmittel abgedampft und der Rückstand über Kieselgel mit Dichlormethan und Chloroform gereinigt. Man erhielt 0,26 g 4-tert-Butyl-N-[6-chloro-5-(2-methoxy-phenylsulfanyl)-pyrimidin-4yl]-benzolsulfonamid. Smp. 186°C.

d) Zu einer Natriumglykolatlösung aus 0,015 g Natrium und 0,326 g Aethylenglykol wurden 0,1 g 4-tert-Butyl-N-[6-chloro-5-(2-methoxy-phenylsulfanyl)-pyrimidin-4-yl-benzolsulfonamid gegeben. Das Reaktionsgemisch wurde unter Argon 2 Stunden bei 60°C gerührt, und zwischen 1N Salzsäure und Essigester verteilt. Die organische Phase wurde mit Wasser gewaschen, über Natriumsulfat getrocknet und das Lösungsmittel abdestilliert. Der Rückstand wurde über Kieselgel mit Chloroform chromatographiert. Man erhielt 0,077 g 4-tert-Butyl-N-[6-(2-hydroxy-ethoxy)-5-(2-methoxy-phenylsulfanyl)-pyrimidin-4-yl]-benzolsulfonamid als Schaum. MS: 489 (M).

Beispiel 83

In Analogie zu Beispiel 82 wurde aus 4-tert-Butyl-N-[6-(2-hydroxy-ethoxy)-5-(2-methoxy-phenylsulfanyl)-2,2'-bipyrimidin-4-yl]-benzolsulfonamid das Pyridin-3-ylcarbaminsäure 2-[6-tert-butyl-phenylsulfonylamino)-5-(2-methoxy-phenylsulfanyl)-2,2'-bipyrimidin-4-yloxy]-ethylester erhalten. Smp. 115°C.

Das Ausgangsmaterial wurde wie folgt hergestellt:

a) In Analogie zu Beispiel 82, Abschnitt a) wurde aus (2-Methoxy-phenylsulfanyl)-malonsäuredimethylester und 2-Amidinpyrimidin das 6-Hydroxy-5-(2-methoxy-phenylsulfanyl)-3,4-dihydro-2,2'-bipyrimidin-4-on als Oel erhalten, welches in Analogie zu Beispiel 82, Abschnitt b) mit $POCl_3$ umgesetzt wurde. Man erhielt das 4,6-Dichloro-5-(2-methoxy-phenylsulfanyl)-2,2'-dipyrimidin, welches in Analogie zu Beispiel 82, Abschnitt c) mit (p-t-Butyl-benzolsulfonamid)-Kalium umgesetzt wurde. Man erhielt das 4-tert-Butyl-N-[6-chloro-5-(2-methoxy-phenylsulfanyl)-2,2'-bipyrimidin-4-yl]-benzol- sulfonamid, welches in Analogie zu Beispiel 82, Abschnitt d) mit Natriumglykolat zu 4-tert-Butyl-N-[6-(2-hydroxy-ethoxy)-5-(2-methoxy-phenylsulfanyl)-2,2'-bipyrimidin-4-yl]-benzolsulfonamid umgesetzt wurde.

Beispiel 84

a) In Analogie zu Beispiel 82 erhielt man aus 4-tert-Butyl-N-[6-(2-hydroxy-ethoxy)-5-(2-methoxy-phenylsulfanyl)-2-methyl-pyrimidin-4-yl]-benzolsulfonamid und 2-Pyridylcarbonsäureazid den Pyridin-4-ylcarbaminsäure 2-[6-(4-tert-butyl-phenylsulfonylamino)-5-(2-methoxy-phenylsulfanyl)-2-methyl-pyrimidin-4-yloxy]-ethylester. Smp. 157°C.

b) Das oben eingesetzte Sulfonamid wurde gemäss der Synthesesequenz von Beispiel 82a)-d) synthetisiert, wobei Formamidinacetat in Abschnitt a) durch Acetamidinhydrochlorid ersetzt wurde.

Beispiel 85

a) In Analogie zu Beispiel 2, Abschnitt a) erhielt man aus p-tert-Butyl-N-[5-(2-bromo-5-methoxyphenoxy)-6-(2-hydroxy-ethoxy)-4-pyrimidinyl]-benzolsulfonamid und 2-Pyridylcarbonsäureazid den Pyridyl-2-ylcarbaminsäure 2-[5-(2-bromo-5-methoxy-phenoxy)-6-(4-tert-butyl-phenylsulfonylamino)-pyrimidin-4-yloxy]-ethylester.

Herstellung der Ausgangsverbindung:

b) Durch Bromierung von 4,6-Dichloro-5-(3-methoxyphenoxy)-pyrimidin mit N-Bromsuccinimid in Essigsäure/Acetanhydrid bei 100°C erhielt man das 4,6-Dichloro-5-(2-bromo-5-methoxyphenoxy)pyrimidin; daraus mit p-tert-Benzolsulfonamid-K das N-[5-(2-Bromo-5-methoxyphenoxy)-6-chloropyrimidin-4-yl]-4-tert-butyl-benzolsulfonamid und daraus mit Na-Aethylenglykolat das p-tert-Butyl-N-[5-(2-bromo-5-methoxyphenoxy)-6-(2-hydroxyethoxy)-4-pyrimidinyl]-benzolsulfonamid.

Beispiel 86

a) In Analogie zu Beispiel 2, Abschnitt a) erhielt man aus p-tert-Butyl-N-[5-(3,4-dimethoxy-phenoxy)-6-(2-hydroxyethoxy)-4-pyrimidinyl]-benzolsulfonamid den Pyridin-4-ylcarbaminsäure 2-[6-(4-tert-butyl-phenylsulfonylamino)-5-(3,4-dimethoxy-phenoxy)-pyrimidin-4-yloxy]-ethylester.

Herstellung der Ausgangsverbindung:

b) Durch Kondensation von 2-(3,4-Dimethoxy-phenoxy)-propan-1,3-dionsäure mit Formamidinacetat erhielt man das 5-(3,4-Dimethoxy-phenoxy)-pyrimidin-4,6-diol, daraus mit $POCl_3$ das 4,6-Dichloro-5-(3,4-dimethoxy-phenoxy)-pyrimidin, daraus mit p-tert-Butylbenzolsulfonamid-K das 4-tert-Butyl-N-[6-chloro-5-(3,4-dimethoxy-phenoxy)-pyrimidin-4-yl]-benzolsulfonamid und daraus mit Na-Aethylenglykolat das p-tert-Butyl-N-[5-(3,4-dimethoxy-phenoxy)-6-(2-hydroxyethoxy)-4-pyrimidinyl]-benzolsulfonamid.

Beispiel 87

In Analogie zu Beispiel 2, Abschnitt a) erhielt man aus 2-Pyridylcarbonsäureazid und 4-tert-Butyl-N-[5-(3,4-dimethoxyphenoxy)-6-(2-hydroxyethoxy)-4-pyrimidinyl]-benzolsulfonamid den Pyridin-2-ylcarbaminsäure 2-[6-(4-tert-butyl-phenylsulfonylamino)-5-(3,4-dimethoxy-phenoxy)-pyrimidin-4-yloxy]-ethylester. MS: 624,2 (M+H).

Beispiel 88

a) In Analogie zu Beispiel 2, Abschnitt a) erhielt man aus 2-Pyridylcarbonsäureazid und 3,4-Dimethoxy-N-[5-(2-chloro-5-methoxyphenoxy)-6-(2-hydroxy-ethoxy)-4-pyrimidinyl]-benzolsulfonamid den Pyridin-2-ylcarbaminsäure 2-[5-(2-chloro-5-methoxy-phenoxy)-6-(3,4-dimethoxy-phenylsulfonylamino)-pyrimidin-4-yloxy]-ethylester. MS: 632,5 (M+H).
b) Die Ausgangsverbindung erhielt man ausgehend von der Verbindung aus Beispiel 1e) analog dem Verfahren von Beispiel 1g) und 1h) mit (3,4-Dimethoxybenzolsulfonamid)-K als Komponente. MS: 412 (M-$SO_2Cl$).

Beispiel 89

a) In Analogie zu Beispiel 1, Abschnitt a) erhielt man aus Phenylisocyanat und Essigsäure 2-[4-[6-(2-hydroxy-ethoxy)-5-(2-methoxy-phenoxy)-pyrimidin-4-ylaminosulfonyl]-phenoxy]-ethylester den Essigsäure 2-[4-[5-(2-methoxy-phenoxy)-6-(2-phenylcarbamoyloxy-ethoxy)-pyrimidin-4-ylsulfamoyl]-phenoxy]-ethylester. MS: 639,4 (M+H).

Herstellung der Ausgangsverbindung:

b) 9 g Essigsäure-2-phenoxy-ethylester löste man in 75 ml Methylenchlorid und tropfte die Lösung anschliessend in 11 ml eiskalte Chlorsulfonsäure ein. Man liess noch 1 Stunde bei Raumtemperatur nachreagieren, verteilte zwischen Eiswasser und $CH_2Cl_2$, trocknete die organische Phase und entfernte das Lösungsmittel schliesslich am Rotationsverdampfer. Den Rückstand kristallisierte man aus Essigester-Hexan. Man erhielt so 4,3 g Essigsäure 2-(4-chlorsulfonylphenoxy)ethylester.
c) Eine Lösung von 0,783 g 6-[2-(t-Butyl-dimethylsilyloxy)ethoxy]-5-(2-methoxy-phenoxy)pyrimidin-4-ylamin (beschrieben in EP 526708) in 30 ml Tetrahydrofuran wurde mit 0,436 g NaH (60%ig) versetzt und 1 Stunde bei Raumtemperatur gerührt. Danach wurden 0,7 g Essigsäure 2-(4-chloro-sulfonyl-phenoxy)-ethylester zugesetzt. Das Reaktionsgemisch wurde 3,5 Stunden bei Raumtemperatur gerührt, auf Eis gegossen, mit Aethylacetat extrahiert und die organische Phase getrocknet. Nach Entfernen des Lösungsmittels und Chromatographie an Kieselgel mit $CH_2Cl_2$/Essigester (10:1) wurden 460 mg des noch silylgeschützten Produkts erhalten, als weisser Schaum. Dieser wurde in 20 ml Acetonitril bei 0°C langsam mit 3 ml 40%iger HF versetzt. Das Reaktionsgemisch wurde 30 Minuten bei 0°C und 90 Minuten bei Raumtemperatur gerührt, danach auf Eis/2N $KHCO_3$-Lösung gegossen, mit $CH_2Cl_2$ extrahiert und die organische Phase getrocknet. Nach Entfernen des Lösungsmittels wurde der Rückstand an Kieselgel mit $CH_2Cl_2$/MeOH (30:1) als Laufmittel chromatographiert. Man erhielt so 319 mg des gewünschten Produkts. MS: 520 (M+H).

Beispiel 90

In Analogie zu Beispiel 2, Abschnitt a) erhielt man aus 2-Pyridylcarbonsäureazid und Essigsäure 2-[4-[6-(2-hydroxyethoxy)-5-(2-methoxyphenoxy)-pyrimidin-4-ylaminosulfonyl]phenoxy]ethylester den Essigsäure 2-[4-5-(2-methoxy-phenoxy)-6-(2-pyridin-2-ylcarbamoyloxy-ethoxy)-pyrimidin-4-ylsulfamoyl]-phenoxy]-ethylester. MS: 640,5 (M+H).

Beispiel 91

In Analogie zu Beispiel 2, Abschnitt a) erhielt man aus 4-Pyridylcarbonsäureazid und Essigsäure 2-[4-[6-(2-hydroxyethoxy)-5-(2-methoxy-phenoxy)pyrimidin-4-ylaminosulfonyl]phenoxy]ethylester den Essigsäure 2-[4-5-(2-methoxy-phenoxy)-6-(2-pyridin-4-ylcarbamoyloxy-ethoxy)-pyrimidin-4-ylsulfamoyl]-phenoxy]-ethylester. MS: 640,5 (M+H).

Beispiel 92

In Analogie zu Beispiel 2, Abschnitt a) erhielt man aus 3-Pyridylcarbonsäureazid und Essigsäure 2-[4-[6-(2-hydroxyethoxy)-5-(2-methoxy-phenoxy)pyrimidin-4-ylaminosulfonyl]phenoxy]ethylester den Essigsäure 2-[4-[5-(2-methoxy-phenoxy)-6-(2-pyridin-4-ylcarbamoyloxy-ethoxy)-pyrimidin-4-ylsulfamoyl]-phenoxy]-ethylester. MS: 640,4 (M+H).

Beispiel 93

In Analogie zu Beispiel 1, Abschnitt a) erhielt man aus 2-Fluorphenylisocyanat und Essigsäure 2-[4-[6-(2-hydroxyethoxy)-5-(2-methoxyphenoxy)-pyrimidin-4-ylaminosulfonyl]phenoxy]ethylester den Essigsäure 2-[4-[6-[2-(2-fluoro-phenylcarbamoyloxy)-ethoxy]-5-(2-methoxy-phenoxy)-pyrimidin-4-ylsulfamoyl]-phenoxy]-ethylester. MS: 657,4 (M+H).

Beispiel 94

Durch basische Verseifung der Verbindung von Beispiel 89 erhielt man Phenylcarbaminsäure 2-[6-[4-(2-hydroxy-ethoxy)-phenylsulfonylamino]-5-(2-methoxy-phenoxy)-pyrimidln-4-yloxy]-ethylester. MS: 597,3 (M+H).

Beispiel 95

Durch basische Verseifung der in Beispiel 90 hergestellten Verbindung erhielt man den Pyridin-2-ylcarbaminsäure 2-[6-[4-(2-hydroxy-ethoxy)-phenylsulfonylamino]-5-(2-methoxy-phenoxy)-pyrimidin-4-yloxy]-ethylester. MS: 598,6 (M+H).

Beispiel 96

Durch basische Verseifung der in Beispiel 91 hergestellten Verbindung erhielt man den Pyridin-4-ylcarbaminsäure 2-[6-[4-(2-hydroxy-ethoxy)-phenylsulfonylamino]-5-(2-methoxy-phenoxy)-pyrimidin-4-yloxy]-ethylester. MS: 596,6 (M-H).

Beispiel 97

Durch basische Verseifung der in Beispiel 92 hergestellten Verbindung erhielt man den Pyridin-3-ylcarbaminsäure 2-[6-[4-(2-hydroxy-ethoxy)-phenylsulfonylamino]-5-(2-methoxy-phenoxy)-pyrimidin-4-yloxy]-ethylester. MS: 598,4 (M+H).

Beispiel 98

Durch basische Verseifung der in Beispiel 93 hergestellten Verbindung erhielt man den 2-Fluoro-phenylcarbaminsäure 2-[6-[4-(2-hydroxy-ethoxy)-phenylsulfonylamino]-5-(2-methoxy-phenoxy)-pyrimidin-4-yloxy]-ethylester. MS: 615,4 (M+H).

Beispiel 99

In Analogie zu Beispiel 1, Abschnitt a) erhielt man aus N-[6-(2-Hydroxy-ethoxy)-5-(2-methoxy-phenoxy)-pyrimidin-4-yl]-4-methoxy-3-(2-morpholin-4-yl-2-oxo-ethoxy)-benzolsulfonamid und 2-Fluorisocyanat den 2-Fluoro-phenylcarbaminsäure 2-[6-[4-methoxy-3-(2-morpholin-4-yl-2-oxo-ethoxy)-phenylsulfonylamino]-5-(2-methoxy-phenoxy)-pyrimidin-4-yloxy]-ethylester. MS: 728,5 (M+H).

Zur Herstellung der Ausgangsverbindung wurde 2-(2-Methoxy-phenoxy)-1-(morpholin-4-yl)-ethan mit Chlorsulfonsäure zu 4-Methoxy-3-(2-morpholin-4-yl-2-oxo-ethoxy)-benzolsulfonylchlorid und dieses mit dem Amin aus Beispiel 89c) in analoger Weise umgesetzt. MS: 591 (M+H).

Beispiel 100

In Analogie zu Beispiel 2, Abschnitt a) erhielt man aus 2-Pyridylcarbonsäureazid und N-[6-(2-Hydroxy-ethoxy)-5-(2-methoxy-phenoxy)-pyrimidin-4-yl]-4-methoxy-3-(2-morpholin-4-yl-2-oxo-ethoxy)-benzolsulfonamid den Pyridin-2-ylcarbaminsäure 2-[6-[4-methoxy-3-[2-morpholin-4-yl-2-oxo-ethoxy)-phenylsulfonylamino]-5-(2-methoxy-phenoxy)-pyrimidin-4-yloxy]-ethylester. MS: 709,2 (M-H).

Beispiel 101

In Analogie zu Beispiel 2, Abschnitt a) erhielt man aus 4-Pyridylcarbonsäureazid und N-[6-(2-Hydroxy-ethoxy)-5-(2-methoxy-phenoxy)-pyrimidin-4-yl]-4-methoxy-3-(2-morpholin-4-yl-2-oxo-ethoxy)-benzolsulfonamid den Pyridin-4-ylcarbaminsäure 2-[6-[4-methoxy-3-[2-morpholin-4-yl-2-oxo-ethoxy)-phenylsulfonylamino]-5-(2-methoxy-phenoxy)-pyrimidin-4-yloxy]-ethylester. MS: 711,4 (M+H).

Beispiel 102

In Analogie zu Beispiel 2, Abschnitt a) erhielt man aus Pyrazincarbonsäureazid und N-[6-(2-Hydroxy-ethoxy)-5-(2-methoxy-phenoxy)-pyrimidin-4-yl]-4-methoxy-3-(2-morpholin-4-yl-2-oxo-ethoxy)-benzolsulfonamid den Pyrazin-2-ylcarbaminsäure 2-[6-[4-methoxy-3-[2-morpholin-4-yl-2-oxo-ethoxy)-phenylsulfonylamino]-5-(2-methoxy-phenoxy)-pyrimidin-4-yloxy]-ethylester. MS: 712,5 (M+H).

Beispiel 103

a) In Analogie zu Beispiel 1, Abschnitt a) erhielt man aus 2-Fluorisocyanat und Essigsäure 2-[4-[5-(2-chloro-5-methoxy-phenoxy)-6-(2-hydroxy-ethoxy)-pyrimidin-4-ylaminosulfonyl]-phenoxy]-ethylester den Essigsäure 2-[4-[5-(2-chloro-5-methoxy-phenoxy)-6-[2-(2-fluoro-phenylcarbamoyloxy)-ethoxy]-pyrimidin-4-ylsulfamoyl]-phenoxy]-ethylester. MS: 691,6 (M+H).

Herstellung der Ausgangsverbindungen

b) In Analogie zu Beispiel 89, Abschnitt c) erhielt man aus 6-[2-(tert-Butyl-dimethyl-silanyloxy)-ethoxy]-5-(2-chloro-5-methoxy-phenoxy)-pyrimidin-4-ylamin und dem Sulfonylchlorid aus Beispiel 89, Abschnitt b) die oben eingesetzte Verbindung. MS: 554,3 (M+H).

c) In eine Lösung von 9,9 g 4,6-Dichloro-5-(2-chloro-5-methoxy-phenoxy)-pyrimidin aus Beispiel 1e) in 400 ml Ethanol wurden bei -78°C ca. 500 ml $NH_3$ eingeleitet. Danach wurde das Reaktionsgemisch 15 Stunden bei -78°C und 50 Stunden bei Raumtemperatur gerührt und schliesslich eingedampft. Der Rückstand wurde zwischen Aethylacetat und Wasser verteilt und die organische Phase aufgearbeitet. Man erhielt so 8,53 g 6-Chloro-5-(2-chloro-5-methoxy-phenoxy)-pyrimidin-4-ylamin als gelbe Kristalle. MS: 285 (M).

d) 8,53 g der vorstehend erhaltenen Verbindung wurden zu einer Lösung von 0,82 g Natrium in 100 ml Aethylenglykol bei 50°C gegeben. Die Lösung wurde 20 Stunden auf 100°C erwärmt, danach zwischen halbgesättigter $NH_4Cl$-Lösung und $CH_2Cl_2$ verteilt und aufgearbeitet. Man erhielt 8,3 g 2-[6-Amino-5-(2-chloro-5-methoxy-phenoxy)-4-pyrimidin-4-yloxy]-1-ethanol als weissen Feststoff, der ohne weitere Reinigung silyliert wurde. Dazu wurde obiges Material (8,3 g) in 300 ml Methylenchlorid gelöst, mit 8,15 g Dimethylaminopyridin versetzt und schliesslich bei Raumtemperatur mit 10,05 g t-Butyldimethylchlorsilan. Die Reaktionslösung wurde 5 Stunden bei Raumtemperatur gerührt. Dann wurde filtriert, die Lösung eingeengt, der Eindampfrückstand zwischen halbgesättigter $NH_4Cl$-Lösung und Aethylacetat verteilt und die organische Phase aufgearbeitet. Anschliessende Kristallisation aus Methylenchlorid/Hexan lieferte 7 g 6-[2-(tert-Butyl-dimethyl-silanyloxy)-ethoxy]-5-(2-chloro-5-methoxy-phenoxy)-pyrimidin-4-ylamin. MS: 410 ($M-CH_3$).

Beispiel 104

Durch basische Verseifung der in Beispiel 103a) hergestellten Verbindung erhielt man 2-Fluoro-phenylcarbaminsäure 2-[5-(2-chloro-5-methoxy-phenoxy)-6-[4-(2-hydroxy-ethoxy)-phenylsulfonylamino]-pyrimidin-4-yloxy]-ethylester.

Beispiel 105

In Analogie zu Beispiel 2, Abschnitt a) erhielt man aus Essigsäure 2-[4-[5-(2-chloro-5-methoxy-phenoxy)-6-(2-hydroxy-ethoxy)-pyrimidin-4-ylaminosulfonyl]-phenoxy]-ethylester und 2-Pyridylcarbonsäureazid den Essigsäure 2-[4-[5-(2-chloro-5-methoxy-phenoxy)-6-(2-pyridin-2-ylcarbamoyloxy-ethoxy)-pyrimidin-4-ylsulfamoyl]-phenoxy]-ethylester. MS: 674,5 (M+H).

Beispiel 106

Durch basische Verseifung der in Beispiel 105 hergestellten Verbindung erhielt man den Pyridin-2-ylcarbaminsäure 2-[5-(2-chloro-5-methoxy-phenoxy)-6-[4-(2-hydroxy-ethoxy)-phenylsulfonylamino]-pyrimidin-4-yloxy]-ethylester. MS: 632,4 (M+H).

Beispiel 107

a) In Analogie zu Beispiel 1, Abschnitt a) erhielt man aus N-[5-(2-chloro-5-methoxy-phenoxy)-6-(2-hydroxy-ethoxy)-pyrimidin-4-yl]-4-methoxy-3-(2-morpholin-4-yl-2-oxo-ethoxy)-benzolsulfonamid und 2-Fluorisocyanat den 2-Fluoro-phenylcarbaminsäure 2-[5-(2-chloro-5-methoxy-phenoxy)-6-[4-methoxy-3-(2-morpholin-4-yl-2-oxo-ethoxy)-phenylsulfonylamino]-pyrimidin-4-yloxy]-ethylester. MS: 762,5 (M+H).

Herstellung der Ausgangsverbindungen

b) In Analogie zu Beispiel 89, Abschnitt c) erhielt man aus dem Sulfonylchlorid von Beispiel 99 und 6-[2-(tert-Butyl-dimethyl-silanyloxy)-ethoxy]-5-(2-chloro-5-methoxy-phenoxy)-pyrimidin-4-ylamin die oben eingesetzte Ausgangsverbindung. MS: 525 (M-$SO_2$Cl).

Beispiel 108

In Analogie zu Beispiel 2, Abschnitt a) erhielt man aus N-[5-(2-Chloro-5-methoxy-phenoxy)-6-(2-hydroxy-ethoxy)-pyrimidin-4-yl]-4-methoxy-3-(2-morpholin-4-yl-2-oxo-ethoxy)-benzolsulfonamid und 2-Pyridylcarbonsäureazid den Pyridin-2-ylcarbaminsäure 2-[5-(2-chloro-5-methoxy-phenoxy)-6-[4-methoxy-3-(2-morpholin-4-yl-2-oxo-ethoxy)-phenylsulfonylamino]-pyrimidin-4-yloxy]-ethylester. MS: 745,5 (M+H).

Beispiel 109

In Analogie zu Beispiel 2, Abschnitt a) erhielt man aus N-[5-(2-Chloro-5-methoxy-phenoxy)-6-(2-hydroxy-ethoxy)-pyrimidin-4-yl]-4-methoxy-3-(2-morpholin-4-yl-2-oxo-ethoxy)-benzolsulfonamid und 4-Pyridylcarbonsäureazid den Pyridin-4-ylcarbaminsäure 2-[5-(2-chloro-5-methoxy-phenoxy)-6-[4-methoxy-3-(2-morpholin-4-yl-2-oxo-ethoxy)-phenylsulfonylamino]-pyrimidin-4-yloxy]-ethylester. MS: 745,6 (M+H).

Beispiel 110

In Analogie zu Beispiel 2, Abschnitt a) erhielt man aus N-[5-(2-Chloro-5-methoxy-phenoxy)-6-(2-hydroxy-ethoxy)-pyrimidin-4-yl]-4-methoxy-3-(2-morpholin-4-yl-2-oxo-ethoxy)-benzolsulfonamid und Pyrazincarbonsäureazid den Pyrazin-2-ylcarbaminsäure 2-[5-(2-chloro-5-methoxy-phenoxy)-6-[4-methoxy-3-(2-morpholin-4-yl-2-oxo-ethoxy)-phenylsulfonylamino]-pyrimidin-4-yloxy]-ethylester. MS: 746,4 (M+H).

Beispiel 111

a) In Analogie zu Beispiel 1, Abschnitt a) erhielt man aus Phenylisocyanat und N-[6-(2-Hydroxy-ethoxy)-5-(-3-methoxy-phenoxy)-pyrimidin-4-yl]-4-methoxy-3-(2-morpholin-4-yl-2-oxo-ethoxy)-benzolsulfonamid den Phenylcarbaminsäure 2-[6-[4-methoxy-3-(2-morpholin-4-yl-2-oxo-ethoxy)-phenyl-sulfonylamino]-5-(3-methoxy-phenoxy)-pyrimidin-4-yloxy]-ethylester. MS: 710,5 (M+H).

Herstellung der Ausgangsverbindungen

b) In Analogie zu Beispiel 89, Abschnitt c) erhielt man aus dem Sulfonylchlorid von Beispiel 99 und 6-[2-(tert-Butyl-dimethyl-silanyloxy)-ethoxy]-5-(3-methoxy-phenoxy)-pyrimidin-4-ylamin die oben eingesetzte Verbindung.
c) Das in b) eingesetzte Amin wurde aus 4,6-Dichloro-5-(3-methoxy-phenoxy)-pyrimidin (beschrieben in EP 526708) gemäss dem Beispiel 103, Abschnitt c) und d) erhalten.

Beispiel 112

a) In Analogie zu Beispiel 1, Abschnitt a) erhielt man aus 2-Fluorisocyanat und 5-[N-[5-(2-Chloro-5-methoxy-phenoxy)-6-(2-hydroxy-ethoxy)-pyrimidin-4-yl]aminosulfonyl]-2-methoxy-phenoxyessigsäure-ethylester den [5-[5-(2-Chloro-5-methoxy-phenoxy)-6-[2-(2-fluorophenylcarbamoyloxy)-ethoxy]-pyrimidin-4-ylsulfamoyl]-2-methoxy-phenoxy]-essigsäure-ethylester. MS: 621 (M-$SO_2$Cl).

Herstellung der Ausgangsverbindungen

b) In Analogie zu Beispiel 89, Abschnitt c) erhielt man aus 6-[2-(tert-Butyl-dimethyl-silanyloxy)-ethoxy]-5-(2-chloro-5-methoxy-phenoxy)-pyrimidin-4-ylamin und (2-Methoxy-5-chlorosulfonyl)phenoxyessigsäure-ethylester (Herstellung beschrieben in EP 526708) die oben eingesetzte Ausgangsverbindung. MS: 484 (M-$SO_2$Cl).

Beispiel 113

Durch basische Verseifung der in Beispiel 112a) hergestellten Verbindung erhielt man 5-[5-(2-Chloro-5-methoxy-phenoxy)-6-[2-(2-fluoro-phenylcarbamoyloxy)-ethoxy]-pyrimidin-4-ylsulfamoyl]-2-methoxy-phenoxy]-essigsäure. MS: 693.4 (M+H).

Beispiel 114

a) In Analogie zu Beispiel 2, Abschnitt a) erhielt man aus 2-Pyridylcarbonsäureazid und N-[5-(2-Chloro-5-methoxy-phenoxy)-6-(2-hydroxy-ethoxy)-pyrimidin-4-yl]-4-(2-morpholin-4-yl-2-oxo-ethoxy)-benzolsulfonamid den Pyridin-2-ylcarbaminsäure 2-[5-(2-chloro-5-methoxy-phenoxy)-6-[4-(2-morpholin-4-yl-2-oxo-ethoxy]-phenylsulfonylamino]-pyrimidin-4-yloxy]-ethylester. MS: 715,3 (M+H).

Herstellung der Ausgangsverbindungen

b) In Analogie zu Beispiel 89, Abschnitt c) erhielt man aus 6-[2-(tert-Butyl-dimethyl-silanyloxy)-ethoxy]-5-(2-chloro-5-methoxy-phenoxy)-pyrimidin-4-ylamin und 4-(2-Morpholin-4-yl-2-oxo-ethoxy)-benzolsulfonylchlorid die oben eingesetzte Verbindung. MS: 495 (M-$SO_2$Cl).
c) Das oben eingesetzte Sulfonylchlorid erhielt man analog zu Beispiel 89, Abschnitt b) aus 4-(Phenoxyacetyl)-morpholin und Chlorsulfonsäure.

Beispiel 115

In Analogie zu Beispiel 2, Abschnitt a) erhielt man aus N-[5-(2-Chloro-5-methoxy-phenoxy)-6-(2-hydroxy-ethoxy)-pyrimidin-4-yl]-4-(2-morpholin-4-yl-2-oxo-ethoxy)-benzolsulfonamid und Pyrazincarbonsäureazid den Pyrazin-2-ylcarbaminsäure 2-[5-(2-chloro-5-methoxy-phenoxy)-6-[4-(2-morpholin-4-yl-2-oxo-ethoxy]-phenylsulfonylamino]-pyrimidin-4-yloxy]-ethylester. MS: 716,4 (M+H).

Beispiel 116

a) In Analogie zu Beispiel 2, Abschnitt a) erhielt man aus 2-Pyridylcarbonsäureazid und N-[6-(2-Hydroxy-ethoxy)-5-(2-methoxy-phenoxy)-pyrimidin-4-yl]-4-(2-morpholin-4-yl-2-oxo-ethoxy)-benzolsulfonamid den Pyridin-2-ylcarbaminsäure 2-[5-(2-methoxy-phenoxy)-6-[4-(2-morpholin-4-yl-2-oxo-ethoxy)-phenylsulfonylamino]-pyrimidin-4-yloxy]-ethylester. MS: 681,3 (M+H).

Herstellung der Ausgangsverbindungen

b) Obige Ausgangsverbindung wurde in Analogie zu Beispiel 89, Abschnitt c) hergestellt, wobei als Sulfonylchlorid die Verbindung aus Beispiel 114 eingesetzt wurde.

Beispiel 117

In Analogie zu Beispiel 2, Abschnitt a) erhielt man aus 4-Pyridylcarbonsäureazid und N-[6-(2-Hydroxy-ethoxy)-5-(2-methoxy-phenoxy)-pyrimidin-4-yl]-4-(2-morpholin-4-yl-2-oxo-ethoxy)-benzolsulfonamid den Pyridin-4-ylcarbaminsäure 2-[5-(2-methoxy-phenoxy)-6-[4-(2-morpholin-4-yl-2-oxo-ethoxy)-phenylsulfonylamino]-pyrimidin-4-yloxy]-ethylester. MS: 681,5 (M+H).

Beispiel 118

a) In Analogie zu Beispiel 2, Abschnitt a) erhielt man aus 2-Pyridylcarbonsäureazid und N-[6-(2-Hydroxy-ethoxy)-5-(2-methoxy-phenoxy)-pyrimidin-4-yl]-4-(3-morpholin-4-yl-3-oxo-propyl)-benzolsulfonamid den Pyridin-2-ylcarbaminsäure 2-[5-(2-methoxy-phenoxy)-6-[4-(3-morpholin-4-yl-3-oxo-propyl)-phenylsulfonylamino]-pyrimidin-4-yloxy]-ethylester. MS: 679,4 (M+H).

Herstellung der Ausgangsverbindungen

b) Obige Ausgangsverbindung erhielt man in Analogie zu Beispiel 89, Abschnitt c), wobei als Sulfonylchlorid das 4-(3-Morpholin-4-yl-3-oxo-propyl)-benzolsulfonylchlorid eingesetzt wurde. MS: 558 (M).
c) Vorstehendes Sulfochlorid wurde gemäss Beispiel 89, Abschnitt b) aus 4-(1-Oxo-3-phenylpropyl)-morpholin und Chlorsulfonsäure erhalten. MS: 317 (M+H).

Beispiel 119

a) In Analogie zu Beispiel 2, Abschnitt a) erhielt man aus 2-Pyridylcarbonsäureazid und N-[5-(2-Chloro-5-methoxy-phenoxy)-6-(2-hydroxy-ethoxy)-pyrimidin-4-yl]-4-(3-morpholin-4-yl-3-oxo-propyl)-benzolsulfonamid den Pyridin-2-ylcarbaminsäure 2-[5-(2-chloro-5-methoxy-phenoxy)-6-[4-(3-morpholin-4-yl-3-oxo-propyl)-phenylsulfonylamino]-pyrimidin-4-yloxy]-ethylester. MS: 713,6 (M+H).

Herstellung der Ausgangsverbindungen

b) In Analogie zu Beispiel 89, Abschnitt c) erhielt man aus 6-[2-(tert-Butyl-dimethyl-silanyloxy)-ethoxy]-5-(2-chloro-5-methoxy-phenoxy)-pyrimidin-4-ylamin und dem Sulfochlorid von Beispiel 118 die obige Ausgangsverbindung. MS: 557 (M-Cl).

Beispiel 120

a) In Analogie zu Beispiel 2, Abschnitt a) erhielt man aus 2-Pyridylcarbonsäureazid und N-[6-(2-Hydroxy-ethoxy)-5-(2-methoxy-phenoxy)-pyrimidin-4-yl]-4-methoxy-3-(3-morpholin-4-yl-3-oxo-propyl)-benzolsulfonamid den Pyridin-2-ylcarbaminsäure 2-[6-[4-methoxy-3-(3-morpholin-4-yl-3-oxo-propyl)-phenylsulfonylamino]-5-(2-methoxy-phenoxy)-pyrimidin-4- yloxy]-ethylester. MS: 709,5 (M+H).

Herstellung der Ausgangsverbindungen

b) In Analogie zu Beispiel 89, Abschnitt c) mit 4-Methoxy-3-(3-morpholin-4-yl-3-oxo-propyl)-benzolsulfonylchlorid als Ausgangsmaterial erhielt man vorstehende Verbindung. MS: 524 (M-$SO_2$).

c) Obiges Sulfochlorid wurde analog zu Beispiel 89b) aus 3-(2-Methoxy-phenyl)-1-morpholin-4-ylpropanon und Chlorsulfonsäure hergestellt.

Beispiel 121

a) In Analogie zu Beispiel 2, Abschnitt a) erhielt man aus 2-Pyridylcarbonsäureazid und N-[5-(2-Chloro-5-methoxy-phenoxy)-6-(2-hydroxy-ethoxy)-pyrimidin-4-yl]-4-methoxy-3-(3-morpholin-4-yl-3-oxo-propyl)-benzolsulfonamid den Pyridin-2-ylcarbaminsäure 2-[5-(2-chloro-5-methoxy-phenoxy)-6-[4-methoxy-3-(3-morpholin-4-yl-3-oxo-propyl)-phenylsulfonylamino]-pyrimidin-4-yloxy]-ethylester. MS: 743,4 (M+H).

b) Vorstehende Verbindung erhielt man in Analogie zu Beispiel 89, Abschnitt c) aus 6-[2-(tert-Butyl-dimethyl-silanyloxy)-ethoxy]-5-(2-chloro-5-methoxy-phenoxy)-pyrimidin-4-ylamin und dem Sulfochlorid von Beispiel 120b). MS: 623,6 (M+H).

Beispiel 122

In Analogie zu Beispiel 2, Abschnitt a) erhielt man aus N-[5-(2-Chloro-5-methoxy-phenoxy)-6-(2-hydroxy-ethoxy)-pyrimidin-4-yl]-4-methoxy-3-(3-morpholin-4-yl-3-oxo-propyl)-benzolsulfonamid und Pyrazincarbonsäureazid den Pyrimidin-2-ylcarbaminsäure 2-[5-(2-chloro-5-methoxy-phenoxy)-6-[4-methoxy-3-(3-morpholin-4-yl-3-oxo-propyl)-phenylsulfonylamino]-pyrimidin-4-yloxy]-ethylester. MS: 744,5 (M+H).

Beispiel 123

a) In Analogie zu Beispiel 2, Abschnitt a) erhielt man aus 2-Pyridylcarbonsäureazid und N-[6-(2-Hydroxy-ethoxy)-5-(2-methoxy-phenoxy)-pyrimidin-4-yl]-4-methoxy-3-(3-oxo-3-piperidin-1-yl-propyl)-benzolsulfonamid den Pyridin-2-ylcarbaminsäure 2-[5-(4-methoxy-phenoxy)-6-[4-methoxy-3-(3-piperidin-1-yl-3-oxo-propyl)-phenylsufonylamino]-pyrimidin-4-yloxy]-ethylester. MS: 707,5 (M+H).

Herstellung der Ausgangsverbindungen

b) In Analogie zu Beispiel 89, Abschnitt c) mit 4-Methoxy-3-(3-oxo-3-piperidin-1-yl-propyl)-benzolsulfonylchlorid als Reaktionskomponente erhielt man obige Ausgangsverbindung.

c) Vorstehendes Sulfochlorid wurde synthetisiert gemäss Beispiel 89b) aus 3-(2-Methoxy-phenyl)-1-piperidin-1-yl-propanon und Chlorsulfonsäure. MS: 345 (M).

Beispiel 124

a) In Analogie zu Beispiel 2, Abschnitt a) erhielt man aus 2-Pyridylcarbonsäureazid und N-[6-[2-Hydroxy-ethoxy)-5-(2-methoxy-phenoxy)-2,2'-bipyrimidin-4-yl]-4-methoxy-3-(3-piperidin-1-yl-3-oxo-propyl)-benzolsulfonamid den Pyridin-2-ylcarbaminsäure 2-[5-(2-methoxy-phenoxy)-6-[4-methoxy-3-(3-piperidin-1-yl-3-oxo-propyl)-phenylsulfonylamino]-2,2'-bipyrimidin-4-yloxy]-ethylester. MS: 785,5 (M+H).

Herstellung der Ausgangsverbindungen

b) In Analogie zu Beispiel 89, Abschnitt c) erhielt man aus dem Sulfochlorid von Beispiel 123b) und 6-[2-(tert-Butyl-dimethyl-silanyloxy)-ethoxy]-5-(2-methoxy-phenoxy)-2,2'-bipyrimidin-4-ylamin die oben eingesetzte Verbindung. MS: 665,5 (M+H).
c) Obige Ausgangsverbindung erhielt man aus 4,6-Dichloro-5-(2-methoxy-phenoxy)-2,2'-bipyrimidin gemäss Beispiel 103, Abschnitt c) und d). MS: 454 (M-$CH_3$).

Beispiel 125

a) In Analogie zu Beispiel 1, Abschnitt a) erhielt man aus 2-Fluorisocyanat und N-[6-(2-Hydroxy-ethoxy)-5-(2-methoxy-phenoxy)-pyrimidin-4-yl]-4-methoxy-3-(2-oxo-2-piperidin-1-yl-ethoxy)-benzolsulfonamid den 2-Fluoro-phenylcarbaminsäure 2-[6-[4-methoxy-3-(2-oxo-2-piperidin-1-yl-ethoxy)-phenylsulfonylamino]-5-(2-methoxy-phenoxy)-pyridin-4-yloxy]-ethylester. MS: 726,6 (M+H).

Herstellung der Ausgangsverbindungen

b) In Analogie zu Beispiel 89, Abschnitt c) mit 4-Methoxy-3-(2-oxo-2-piperidin-1-yl-ethoxy)-benzolsulfonylchlorid als Reaktionskomponente erhielt man obige Ausgangsverbindung. MS: 524 (M-$SO_2$).
c) Vorstehendes Sulfochlorid wurde synthetisiert gemäss Beispiel 89b) aus 2-(2-Methoxy-phenoxy)-1-piperidin-1-yl-ethanon.

Beispiel 126

a) In Analogie zu Beispiel 2, Abschnitt a) erhielt man aus 2-Pyridylcarbonsäureazid und N-[6-(2-Hydroxy-ethoxy)-5-(2-methoxy-phenoxy)-pyrimidin-4-yl]-4-methoxy-2-(3-morpholin-4-yl-3-oxo-propyl)-benzolsulfonamid den Pyridin-2-ylcarbaminsäure 2-[6-[4-methoxy-2-(3-morpholin-4-yl-3-oxo-propyl)-phenylsulfonylamino]-5-(2-methoxy-phenoxy)-pyrimidin-4-yloxy]-ethylester. MS: 709,4 (M+H).

Herstellung der Ausgangsverbindungen

b) In Analogie zu Beispiel 89, Abschnitt c) mit 4-Methoxy-2-(3-morpholin-4-yl-3-oxo-propyl)-benzolsulfonamid als Reaktionskomponente erhielt man obiges Ausgangsmaterial. MS: 524 (M-$SO_2$).
c) Das vorstehende Sulfochlorid wurde analog zu Beispiel 89b) aus 3-(3-Methoxy-phenyl)-1-morpholin-4-yl-propanon und Chlorsulfonsäure synthetisiert.

Beispiel 127

a) In Analogie zu Beispiel 2, Abschnitt a) erhielt man aus 2-Pyridylcarbonsäureazid und 4-(2-Bromo-ethoxy)-N-[6-(2-hydroxyethoxy)-5-(2-methoxy-phenoxy)-4-pyrimidinyl]-benzolsulfonamid den Pyridin-2-ylcarbaminsäure 2-[6-[4-(2-bromo-ethoxy)-phenylsulfonylamino]-5-(2-methoxy-phenoxy)-pyrimidin-4-yloxy]-ethylester. MS: 660,3 (M+H).

Herstellung der Ausgangsverbindung

b) Obige Ausgangsverbindung erhielt man gemäss Beispiel 89, Abschnitt c) mit 4-(2-Bromo-ethoxy)-benzolsulfonylchlorid als Reaktionskomponente. MS: 475 (M-$SO_2$).

Beispiel 128

a) In Analogie zu Beispiel 2, Abschnitt a) erhielt man aus 2-Pyridylcarbonsäureazid und Essigsäure 3-[4-[6-(2-hydroxy-ethoxy)-5-(2-methoxy-phenoxy)-pyrimidin-4-ylsulfamoyl]-phenoxy]-propylester den Essigsäure 3-[4-[5-(2-methoxy-phenoxy)-6-(2-pyridin-2-ylcarbamoyloxy-ethoxy)-pyrimidin-4-ylsulfamoyl]-phenoxy]-propylester. MS: 654,5 (M+H).

Herstellung der Ausgangsverbindungen

b) Die oben eingesetzte Verbindung erhielt man gemäss Beispiel 89, Abschnitt c) mit Essigsäure 3-(4-chlorsulfonylphenoxy)-propylester als Sulfonylchlorid. MS: 534,3 (M+H).

c) Das vorstehende Sulfochlorid wurde analog zu Beispiel 89b) aus 3-Phenoxy-1-propanolacetat und Chlorsulfonsäure erhalten.

Beispiel 129

Durch alkalische Verseifung der in Beispiel 128a) hergestellten Verbindung erhielt man den Pyridin-2-ylcarbaminsäure 2-[6-[4-(3-hydroxy-propoxy)-phenylsulfonylamino]-5-(2-methoxy-phenoxy)-pyrimidin-4-yloxy]-ethylester. MS: 612,4 (M+H).

Beispiel 130

a) In Analogie zu Beispiel 2, Abschnitt a) erhielt man aus N-[6-(2-Hydroxy-ethoxy)-5-(2-methoxy-phenoxy)-pyrimidin-4-yl]-4-methoxy-3-(2-morpholin-4-yl-2-oxo-ethyl)-benzolsulfonamid und 2-Pyridylcarbonsäureazid den Pyridin-2-ylcarbaminsäure 2-[6-[4-methoxy-3-(2-morpholin-4-yl-2-oxo-ethyl)-phenylsulfonylamino]-5-(2-methoxy-phenoxy)-pyrimidin-4-yloxy]-ethylester. MS: 695,6 (M+H).

Herstellung der Ausgangsverbindungen

b) In Analogie zu Beispiel 89, Abschnitt c) erhielt man aus 4-Methoxy-3-(2-morpholin-4-yl-2-oxo-ethyl)-benzolsulfonylchlorid obige Verbindung. MS: 510 (M-$SO_2$).

c) Das oben eingesetzte Sulfochlorid wurde analog zu Beispiel 89b) aus 2-(2-Methoxy-phenyl)-1-(morpholin-4-yl)-ethanon und Chlorsulfonsäure erhalten.

Beispiel 131

a) In Analogie zu Beispiel 1, Abschnitt a) erhielt man aus 4-tert-Butyl-N-[6-(2-amino-ethoxy)-5-(2-chloro-5-methoxy-phenoxy)-pyrimidin-4-yl]-benzolsulfonamid und Phenylisocyanat das 4-tert-Butyl-N-[5-(2-chloro-5-methoxy-phenoxy)-6-[2-(3-phenyl-ureido)-ethoxy]-pyrimidin-4-yl]-benzolsulfonamid. MS: 526 (M-$SO_2Cl$).

Herstellung der Ausgangsverbindung

b) In Analogie zu Beispiel 1h), wobei Ethylenglykol durch Ethanolamin ersetzt wurde, und der Verbindung aus Beispiel 20b) erhielt man obige Ausgangsverbindung. MS: 407 (M-$SO_2Cl$).

Beispiel 132

In Analogie zu Beispiel 1, Abschnitt a) erhielt man aus 4-tert-Butyl-N-[6-(2-amino-ethoxy)-5-(2-chloro-5-methoxy-phenoxy)-pyrimidin-4-yl]-benzolsulfonamid und 2-Fluorisocyanat das 4-tert-Butyl-N-[5-(2-chloro-5-methoxy-phenoxy)-6-[2-[3-(2-fluoro-phenyl)-ureido]-ethoxy]-pyrimidin-4-yl]-benzolsulfonamid. MS: 544 (M-$SO_2Cl$).

Beispiel 133

In Analogie zu Beispiel 2, Abschnitt a) erhielt man aus 2-Pyridylcarbonsäureazid und 4-tert-Butyl-N-[6-(2-amino-ethoxy)-5-(2-chloro-5-methoxy-phenoxy)-pyrimidin-4-yl]-benzolsulfonamid das 4-tert-Butyl-N-[5-(2-chloro-5-methoxy-phenoxy)-6-[2-(3-pyridin-2-yl-ureido)-ethoxy]-pyrimidin-4-yl]-benzolsulfonamid. MS: 627,4 (M+H).

Beispiel 134

In Analogie zu Beispiel 2, Abschnitt a) erhielt man aus 4-Pyridylcarbonsäureazid und 4-tert-Butyl-N-[6-(2-amino-ethoxy)-5-(2-chloro-5-methoxy-phenoxy)-pyrimidin-4-yl]-benzolsulfonamid das 4-tert-Butyl-N-[5-(2-chloro-5-methoxy-phenoxy)-6-[2-(3-pyridin-4-yl-ureido)-ethoxy]-pyrimidin-4-yl[-benzolsulfonamid. MS: 627,5 (M+H).

Beispiel 135

In Analogie zu Beispiel 2, Abschnitt a) erhielt man aus 3-Pyridylcarbonsäureazid und 4-tert-Butyl-N-[6-(2-amino-ethoxy)-5-(2-chloro-5-methoxy-phenoxy)-pyrimidin-4-yl]-benzolsulfonamid das 4-tert-Butyl-N-[5-(2-chloro-5-methoxy-phenoxy)-6-[2-(3-pyridin-3-yl-ureido)-ethoxy]-pyrimidin-4-yl]-benzolsulfonamid. MS: 407 (M-$SO_2$-$C_6N_4N_2O$).

Beispiel 136

In Analogie zu Beispiel 38 erhielt man durch Oxidation der in Beispiel 135 hergestellten Verbindung das 4-tert-Butyl-N-[5-(2-chloro-5-methoxy-phenoxy)-6-[2-(3-1-oxy-pyridin-4-yl)-ureido]-ethoxy]-pyrimidin-4-yl]-benzolsulfonamid.

Beispiel 137

a) In Analogie zu Beispiel 2, Abschnitt a) erhielt man aus 4-tert-Butyl-N-[6-(2-amino-ethoxy)-5-(2-methoxy-phenoxy)-2-methyl-pyrimidin-4-yl]-benzolsulfonamid und 2-Pyridylcarbonsäureazid das 4-tert-Butyl-N-[5-(2-methoxy-phenoxy)-2-methyl-6-[2-(3-pyridin-2-yl-ureido)-ethoxy]-pyrimidin-4-yl]-benzolsulfonamid. MS: 607,4 (M+H).

Herstellung der Ausgangsverbindungen

b) In Analogie zu Beispiel 1, Abschnitt h) mit Ethanolamin anstatt Ethylenglykol erhielt man aus 4-tert-Butyl-N-[6-chloro-5-(2-methoxy-phenoxy)-2-methyl-pyrimidin-4-yl]-benzolsulfonamid (Herstellung beschrieben in EP 510526) obige Verbindung. MS: 487,5 (M+H).

Beispiel 138

110 mg N-[6-(2-Hydroxy-ethoxy)-5-(3,5-dimethoxy-phenoxy)-pyrimidin-4-yl]-1,3-benzodioxol-5-sulfonamid wurden in 3 ml trockenem THF gelöst, dann wurden 3 ml einer 2M-Lösung $COCl_2$ in Toluol zugegeben. Man liess während 2 Tagen bei Raumtemperatur reagieren. Dann wurden die Lösungsmittel und der Ueberschuss an Reagens abdestilliert. Der Rückstand wurde in 2 ml absoluten Dioxan gelöst und 250 mg 2-Hydroxymethyl-Pyridin wurden zugegeben. Diese Lösung wurde während 2 Stunden bei 70°C gehalten. Dann wurden die flüchtigen Komponenten aodestilliert. Der zurückbleibende Rückstand wurde an Kieselgel mit Essigester als Fliessmittel chromatographiert. Man erhielt 70 mg Carbonsäure 2-[6-(1,3-benzodioxol-5-ylsulfonylamino)-5-(3,5-dimethoxy-phenoxy)-pyrimidin-4-yloxy]-ethylester-pyridin-3-ylmethylester. MS: 627,3 ($M+H^+$).

Beispiel 139

In Analogie zu Beispiel 138 erhielt man aus 250 mg N-[6-(2-Hydroxy-ethoxy)-5-(2-chlor-5-methoxy-phenoxy)-pyrimidin-4-yl]-1,3-benzodioxol-5-sulfonamid den Carbonsäure 2-[6-(1,3-benzodioxol-5-sulfonylamino)-5-(2-chlor-5-methoxy-phenoxy)-pyrimidin-4-yloxy]-ethylester-pyridin-2-ylmethylester. Smp. 148-150°C (aus EtOH).

Beispiel 140

In Analogie zu Beispiel 138 erhielt man aus 250 mg N-[6-(2-Hydroxy-ethoxy)-5-(2-chlor-5-methoxy-phenoxy)-pyrimidin-4-yl]-1,3-benzodioxol-5-sulfonamid den Carbonsäure 2-[6-(1,3-benzodioxol-5-ylsulfonylamino)-5-(2-chlor-5-methoxy-phenoxy)-pyrimidin-4-yloxy]-ethylester-pyridin 2-ylmethylester. MS: 629,2 ($M-H^-$).

Beispiel 141

In Analogie zu Beispiel 138 erhielt man aus 175 mg 4-tert-Butyl-N-[6-(2-hydroxy-ethoxy)-5-(2-chlor-5-methoxy-phenoxy)-pyrimidin-4-yl)-benzolsulfonamid und 2-Hydromethyl-pyridin den Carbonsäure 2-[6-(4-tert-butyl-benzolsulfonylamino)-5-(2-chlor-5-methoxy-phenoxy)-pyrimidin-4-yloxy]-ethylester-pyridin-2-ylmethylester. IR: 1751 $cm^{-1}$ (Ester C=O).

Beispiel 142

In Analogie zu Beispiel 138 erhielt man aus 125 mg N-[6-(2-Hydroxy-ethoxy)-5-(2-chlor-5-methoxy-phenoxy)-pyrimidin-4-yl]-1,3-benzodioxol-5-sulfonamid und 3-(Hydroxymethylfuran) den Carbonsäure 2-[6-(1,3-benzodioxol-5-ylsulfonylamino)-5-(2-chlor-5-methoxy-phenoxy)-pyrimidin-4-yloxy]-ethylester-furan-3-ylmethylester. MS: 620 ($M+H^+$).

Beispiel 143

Zu einer Lösung von 0,1 g 4-tert-Butyl-N-[5-(2-chloro-5-methoxy-phenoxy)-6-(2-hydroxy-ethoxy)-2-methyl-pyrimidin-4-yl]-benzolsulfonamid (EP-A-0526,708) in 1 ml Dichlormethan wurden 0,3 ml einer Lösung von Phosgen 20% in Toluol gegeben. Das Reaktionsgemisch wurde während 30 Minuten bei 20°C gerührt und eingeengt. Der Rückstand wurde in 5 ml Toluol mit 0,183 ml 3-Hydroxymethylpyridin versetzt und eingeengt. Der Rückstand wurde zwischen Chloroform und Wasser verteilt, die organische Phase getrocknet und eingeengt. Nach Kieselgelchromatographie mit Dichlormethan-Essigester 8:2 und Kristallisation aus Aethanol erhielt man 43 mg Carbonsäure 2-[6-(4-tert-butylphenylsulfonylamino)-5-(2-chloro-5-methoxy-phenoxy)-2-methyl-pyrimidin-4-yloxy]-ethylester-pyridin-3-ylmethylester. Smp. 118-119°C. MS: M = 657.

Beispiel 144

In Analogie zu Beispiel 143 wurde aus 4-tert-Butyl-N-[5-(2-chloro-5-methoxy-phenoxy)-6-(2-hydroxy-ethoxy)-2-methyl-pyrimidin-4-yl]-benzolsulfonamid und 2-Hydroxymethylpyridin der Carbonsäure 2-[6-(4-tert-butylphenylsulfonylamino)-5-(2-chloro-5-methoxy-phenoxy)-2-methyl-pyrimidin-4-yloxy]-ethylester-pyridin-2-ylmethylester, Smp. 122°C, MS: M = 657 erhalten.

Beispiel 145

In Analogie zu Beispiel 143 wurde aus 4-tert-Butyl-N-[5-(2-chloro-5-methoxy-phenoxy)-6-(2-hydroxy-ethoxy)-2-methyl-pyrimidin-4-yl]-benzolsulfonamid und 4-Hydroxymethylpyridin der Carbonsäure 2-[6-(4-tert-butyl-phenylsulfonylamino)-5-(2-chloro-5-methoxy-phenoxy)-2-methyl-pyrimidin-4-yloxy]-ethylester-pyrimidin-4-ylmethylester, Schaum, MS: M = 657 erhalten.

Beispiel 146

In Analogie zu Beispiel 138 erhielt man aus 4-tert-Butyl-N-[6-(2-hydroxy-ethoxy)-5-(2-methoxy-phenylsulfanyl)-2,2'-bipyrimidin-4-yl]-benzolsulfonamid und 3-Hydroxymethylpyridin den Carbonsäure 2-[6-(4-tert-Butyl-phenylsulfonylamino)-5-(2-methoxy-phenylsulfanyl)-2,2'-bipyrimidin-4-yloxy]-ethylester-pyridin-3-ylmethylester.

Beispiel 147

In Analogie zu Beispiel 138 erhielt man aus 4-tert-Butyl-N-[6-(2-hydroxy-ethoxy)-5-(2-methoxy-phenylsulfanyl)-2-methyl-pyrimidin-4-yl]-benzolsulfonamid und 3-Hydroxymethylpyridin den Carbonsäure 2-[6-(4-tert-Butyl-phenylsulfonylamino)-5-(2-methoxy-phenylsulfanyl)-2-methylpyrimidin-4-yloxy]-ethylester-pyridin-3-ylmethylester.

Beispiel 148

a) In Analogie zu Beispiel 2, Abschnitt a) erhielt man aus N-[2-(2-Benzyloxy-ethyl)-5-(2-chloro-5-methoxy-phenoxy)-6-(2-hydroxy-ethoxy)-pyrimidin-4-yl]-1,3-benzoldioxol-5-sulfonamid und 2-Pyridylcarbonsäureazid den Pyridin-2-ylcarbaminsäure 2-[6-(1,3-benzodioxol-5-ylsulfonylamino)-2-(2-benzyloxy-ethyl]-5-(2-chloro-5-methoxy-phenoxy)-pyrimidin-4-yloxy]-ethylester. Smp. 143°C.

b) Die oben eingesetzte Ausgangsverbindung erhielt man gemäss dem Verfahren von Beispiel 1, Abschnitt d) und e) und Beispiel 2, Abschnitt b), c) und d), wobei in Beispiel 1d) Formamidinacetat durch 2-Benzyloxyethylamidin-hydrochlorid ersetzt wurde.

Beispiel 149

a) In Analogie zu Beispiel 2, Abschnitt a) erhielt man aus N-[5-(2-Chloro-5-methoxy-phenoxy)-6-(2-hydroxy-ethoxy)-pyrimidin-4-yl]-3-isopropyl-4-methoxy-benzolsulfonamid und 2-Pyridylcarbonsäureazid den Pyridin-2-ylcarbaminsäure 2-[5-(2-chloro-5-methoxy-phenoxy)-6-(3-isopropyl-4-methoxy-phenylsulfonylamino)-pyrimidin-4-yloxy]-ethylester. Smp. 191-193°C.

b) Die oben eingesetzte Ausgangsverbindung erhielt man aus der Verbindung von Beispiel 1e) durch Umsetzung mit (4-Methoxy-3-isopropyl-phenylsulfonamid)-K analog zu Beispiel 1g) und anschliessende Glykolyse gemäss Beispiel 1h). Smp. 167-168°C.

Beispiel 150

a) In Analogie zu Beispiel 2, Abschnitt a) erhielt man aus N-[5-(2-Chloro-5-methoxy-phenoxy)-6-(2-hydroxy-ethoxy)-2-(3-methoxy-propyl)-pyrimidin-4-yl]-1,3-benzodioxol-5-sulfonamid und 2-Pyridylcarbonsäureazid den Pyridin-2-ylcarbaminsäure 2-[6-(1,3-benzodioxol-5-ylsulfonylamino)-5-(2-chloro-5-methoxy-phenoxy)-2-(3-methoxy-propyl)-pyrimidin-4-yloxy]-ethylester. Smp. 163-164°C.

b) Obige Ausgangsverbindung erhielt man in Analogie zu Beispiel 10, Abschnitt b)-g), wobei in 10b) Methoxybutyronitril anstatt Methoxyproprionitril eingesetzt wurde. Smp. 137°C.

Beispiel 151

In Analogie zu Beispiel 2, Abschnitt a) erhielt man aus 2-Pyridincarbonsäureazid und 4-Dimethylamino-N-[6-(2-hydroxy-ethoxy)-5-(2-methoxy-phenoxy)-2-morpholin-4-yl-pyrimidin-4-yl]-benzolsulfonamid den Pyridin-2-ylcarbaminsäure 2-[6-(4-dimethylaminophenylsulfonylamino)-5-(2-methoxy-phenoxy)-2-morpholin-4-yl-pyrimidin-4-yloxy]ethylester. MS: 664.4 (M-H).

Die oben eingesetzte Ausgangsverbindung wurde gemäss Beispiel 72, Abschnitt b) hergestellt, mit (4-Dimethylaminobenzolsulfonamid)-K als Reaktionskomponente.

Beispiel 152

In Analogie zu Beispiel 1 wurden hergestellt:

Furan-3-ylcarbaminsäure 2-[6-(1,3-benzodioxol-5-ylsulfonylamino)-5-(2-chlor-5-methoxy-phenoxy)-pyrimidin-4-yloxy]-ethylester, MS: 604,9 ($M+H^+$),
Pyridin-2-ylcarbaminsäure 2-[6-(1,3-benzodioxol-5-ylsulfonylamino)-5-(2-methoxy-phenoxy)-2-methylsulfanyl-pyrimidin-4-yloxy]-ethylester, Smp. 171-172°C (aus Ethanol),
Pyridin-2-ylcarbaminsäure 2-[6-(1,3-benzodioxol-5-ylsulfonylamino)-5-(2-chlor-5-methoxy-phenoxy)-2-(4-methoxy-phenyl)-pyrimidin-4-yloxy]-ethylester, Smp: Zersetzung bei 135°C,
Furan-3-ylcarbaminsäure 2-[6-(1,3-benzodioxol-5-ylsulfonylamino)-5-(2-chlor-5-methoxy-phenoxy)-2-(4-methoxy-phenyl)-pyrimidin-4-yloxy]-ethylester, MS: 711,2 ($M+H^{\oplus}$),
Pyridin-2-ylcarbaminsäure 2-[6-(4-tert.butyl-phenylsulfonylamino)-5-(2-methoxy-phenoxy)-2-methylsulfanyl-pyrimidin-4-yloxy]-ethylester, Smp. 155°C (aus Ethanol),
Pyridin-2-ylcarbaminsäure 2-[6-(1,3-benzodioxol-5-ylsulfonylamino)-5-(2-methoxy-phenoxy)-2-(3-methoxy-phenyl)-pyrimidin-4-yloxy]-ethylester, Smp. 199-200°C,
Pyridin-2-ylcarbaminsäure 2-[6-(1,3-benzodioxol-5-ylsulfonylamino)-5-(2-chlor-5-methoxy-phenoxy)-2-morpholin-4-yl-pyrimidin-4-yloxy]-ethylester, Smp. 199-200°C (aus Ethanol),
Pyridin-2-ylcarbaminsäure 2-[6-(4-tert.butyl-phenylsulfonylamino)-5-(2-methoxy-phenoxy)-2-(4-methoxy-phenyl)-pyrimidin-4-yloxy]-ethylester, Smp. 168°C,
Pyridin-2-ylcarbaminsäure 2-[6-(1,3-benzodioxol-5-ylsulfonylamino)-5-(2-chlor-5-methoxy-phenoxy)-2-(2-methoxy-ethoxy)-pyrimidin-4-yloxy]-ethylester, Smp. 188°C,
Pyridin-2-ylcarbaminsäure 2-[6-(4-tert.butyl-phenylsulfonylamino)-5-(2-chlor-5-methoxy-phenoxy)-2-morpholin-4-yl-pyrimidin-4-yloxy]-ethylester, Smp. 219-220°C,
Pyridin-2-ylcarbaminsäure 2-[6-(4-tert.butyl-phenylsulfonylamino)-5-(2-methoxy-phenoxy)-2-(3-methoxy-phenyl)-pyrimidin-4-yloxy]-ethylester, Smp. 178-179°C (aus Ethanol),
Pyridin-2-ylcarbaminsäure 2-[6-(1,3-benzodioxol-5-ylsulfonylamino)-5-(2-methoxy-phenoxy)-2-(3,4,5-trimethoxy-phenyl)-pyrimidin-4-yloxy]-ethylester, MS: 748,4 ($M+H^{\oplus}$),
Pyridin-2-ylcarbaminsäure 2-[5-(2-chlor-5-methoxy-phenoxy)-6-[4-methylsulfanyl-3-(morpholin-4-ylcarbonyl)-phenylsulfonylamino]-pyrimidin-4-yloxy]-ethylester, Smp: Zersetzung bei 203°C,
Pyridin-2-ylcarbaminsäure 2-[6-(4-tert.butyl-phenylsulfonylamino)-5-(2-chlor-5-methoxy-phenoxy)-2-methylsulfanyl-pyrimidin-4-yloxy]-ethylester, Smp. 168-169°C (aus Ethanol),
Pyridin-2-ylcarbaminsäure (RS)-2-[6-(1,3-benzodioxol-5-ylsulfonylamino)-5-(2-chlor-5-methoxy-phenoxy)-2-(2,3-dimethoxy-propoxy)-pyrimidin-4-yloxy]-ethylester, Smp. 173-174°C (aus EtOH),
Pyridin-2-ylcarbaminsäure 2-[6-(1,3-benzodioxol-5-ylsulfonylamino)-5-(2-chlor-5-methoxy-phenoxy)-2-(2-methylsulfanyl-ethoxy)-pyrimidin-4-yloxy]-ethylester, Smp. 158-159°C (aus Ethanol),
Pyridin-2-ylcarbaminsäure 2-[6-(4-tert.butyl-phenylsulfonylamino)-2-furan-2-ylmethoxy-5-(2-methoxy-phenoxy)-pyrimidin-4-yloxy]-ethylester, MS: 690,2 ($M+H^+$),
Pyridin-2-ylcarbaminsäure 2-[5-(2-methoxy-phenoxy)-2-(3-methoxy-phenyl)-6-(4-methylsulfanyl-3-morpholin-4-ylcarbonyl-phenylsulfonylamino)-pyrimidin-4-yloxy]-ethylester, MS: 801,4 [$(M-H)^{\oplus}$],

Pyridin-2-ylcarbaminsäure (RS)-2-[6-(1,3-benzodioxol-5-ylsulfonylamino)-5-(2-chlor-5-methoxy-phenoxy)-2-(2-methylsulfinyl-ethoxy)-pyrimidin-4-yloxy]-ethylester, Smp. 183°C (aus Ethanol),

Thiophen-3-ylcarbaminsäure 2-[6-(1,3-benzodioxol-5-ylsulfonylamino)-5-(2-chlor-5-methoxy-phenoxy)-2-(2-methoxy-ethoxy)-pyrimidin-4-yloxy]-ethylester, MS: 695,2 (M+H$^{\oplus}$),

Pyridin-2-ylcarbaminsäure 2-[6-(4-tert.butyl-phenylsulfonylamino)-5-(2-chlor-5-methoxy-phenoxy)-2-methylsulfanyl-pyrimidin-4-yloxy]-ethylester, Smp. 211-212°C (aus Ethanol),

Thiophen-3-ylcarbaminsäure (RS)-2-[6-(1,3-benzodioxol-5-ylsulfonylamino)-5-(2-chlor-5-methoxy-phenoxy)-2-(2-methylsulfinyl-ethoxy)-pyrimidin-4-yloxy]-ethylester, MS: 727,3 (M+H$^{\oplus}$),

Pyridin-2-ylcarbaminsäure 2-[6-(4-methoxy-5-phenylsulfonylamino)-5-(2-methoxy-phenoxy)-2,2'-bipyrimidin-4-yloxy]-ethylester, Smp. 188-189°C (aus Ethanol),

Thiophen-3-ylcarbaminsäure 2-[6-(4-tert.butyl-phenylsulfonylamino)-5-(2-methoxy-phenoxy)-2,2'-bipyrimidin-4-yloxy]-ethylester, MS: 677,3 (M+H$^{\oplus}$),

Thiophen-2-ylcarbaminsäure 2-[6-(4-tert.butyl-phenylsulfonylamino)-5-(2-methoxy-phenoxy)-2,2'-bipyrimidin-4-yloxy]-ethylester, MS: 675,3 [(M-H)$^{-}$],

Pyridin-2-ylcarbaminsäure 2-[6-(1,3-benzodioxol-5-ylsulfonylamino)-5-(3,5-dimethoxy-phenoxy)-2-phenyl-pyrimidin-4-yloxy]-ethylester,

Pyridin-2-ylcarbaminsäure 2-[6-(1,3-benzodioxol-5-ylsulfonylamino)-5-(2-methoxy-phenoxy)-(2-methoxy-phenyl)-pyrimidin-4-yloxy]-ethylester, Smp: Zersetzung bei 157°C,

Thiophen-3-ylcarbaminsäure 2-[6-(1,3-benzodioxol-5-sulfonylamino)-5-(2-methoxy-phenoxy)-2-(2-methoxy-phenyl)-pyrimidin-4-yloxy]-ethylester, MS: 693,1 [(M+H)$^{\oplus}$],

Pyridin-2-ylcarbaminsäure 2-[2-(1,3-benzodioxol-5-yl)-6-(1,3-benzodioxol-5-ylsulfonylamino)-5-(2-methoxy-phenoxy)-pyrimidin-4-yloxy]-ethylester, Smp. 185-186°C (aus Ethanol),

Pyrimidin-2-ylcarbaminsäure 2-[6-(1,3-benzodioxol-5-ylsulfonylamino)-5-(2-chlor-5-methoxy-phenoxy)-pyrimidin-4-yloxy]-ethylester, MS: 615,2 [(M-H)$^{\oplus}$],

Pyridin-2-ylcarbaminsäure 2-[5-(2-methoxy-phenoxy)-6-phenylsulfonylamino-2,2'-bipyrimidin-4-yloxy]-ethylester, Smp. 190°C (aus Ethanol),

Pyridin-2-ylcarbaminsäure 2-[5-(2-methoxy-phenoxy)-6-(4-methyl-phenylsulfonylamino)-2,2'-bipyrimidin-4-yloxy]-ethylester, Smp. 194°C (aus Ethanol),

Thiophen-3-ylcarbaminsäure 2-[5-(2-methoxy-phenoxy)-6-(4-methoxy-phenylsulfonylamino)-2,2'-bipyrimidin-4-yloxy]-ethylester, MS: 649,4 [M-H)$^{-}$],

Pyrimidin-2-ylcarbaminsäure 2-[6-(4-tert.butyl-phenylsulfonylamino)-5-(2-methoxy-phenoxy)-2,2'-bipyrimidin-4-yloxy]-ethylester, MS: 671,4 [(M-H)$^{-}$],

Furan-2-ylcarbaminsäure 2-[5-(2-methoxy-phenoxy)-6-(4-methoxy-phenylsulfonylamino)-2,2'-bipyrimidin-4-yloxy]-ethylester, MS: 635,4 [M+H$^{+}$],

Pyridin-2-ylcarbaminsäure 2-[6-(4-isobutyl-phenylsulfonylamino)-5-(2-methoxy-phenoxy)-2,2'-bipyrimidin-4-yloxy]-ethylester, Smp. 176-177°C,

Pyrazin-2-ylcarbaminsäure 2-[5-(2-methoxy-phenoxy)-6-(4-methoxy-phenylsulfonylamino)-2,2'-bipyrimidin-4-yloxy]-ethylester, Smp. 182-183°C,

Furan-2-ylcarbaminsäure 2-[6-(4-tert.butyl-phenylsulfonylamino)-5-(2-methoxy-phenoxy)-2,2'-bipyrimidin-4-yloxy]-ethylester, MS: 659,5 [(M-H)$^{-}$],

Pyridin-2-ylcarbaminsäure (S)-2-[6-(1,3-benzodioxol-5-ylsulfonylamino)-5-(2-chlor-5-methoxy-phenoxy)-2-(2,2-dimethyl-1,3-dioxolan-4-ylmethoxy)-pyrimidin-4-yloxy]-ethylester, Smp. 178°C (aus Ethanol),

Pyridin-2-ylcarbaminsäure 2-[6-(4-methoxy-3-morpholin-4-ylcarbonyl-phenylsulfonylamino)-5-(2-methoxy-phenoxy)-2-(2-methoxy-phenyl)-pyrimidin-4-yloxy]-ethylester, Smp: Zersetzung bei 147°C,

Pyridin-2-ylcarbaminsäure 2-[6-(1,3-benzodioxol-5-ylsulfonylamino)-5-(2-methoxy-phenoxy)-2-piperidin-1-yl-pyrimidin-4-yloxy]-ethylester, Smp. 214-215°C,

Pyridin-2-ylcarbaminsäure 2-[6-(1,3-benzodioxol-5-ylsulfonylamino)-5-(2-methoxy-phenoxy)-2-thiomorpholin-4-yl-pyrimidln-4-yloxy]-ethylester, Smp. 210°C,

Pyridin-2-ylcarbaminsäure 2-[6-(1,3-benzodioxol-5-ylsulfonylamino)-5-(2-methoxy-phenoxy)-2-prop-2-ynyloxy-pyrimidin-4-yloxy]-ethylester, Smp. 177-178°C,

Pyridin-2-ylcarbaminsäure 2-[6-(1,3-benzodioxol-5-ylsulfonylamino)-5-(2-methoxy-phenoxy)-2-pyrrolidin-1-yl-pyrimidin-4-yloxy]-ethylester, Smp: Zersetzung bei 236°C,

Pyridin-2-ylcarbaminsäure 2-[6-(1,3-benzodioxol-5-ylsulfonylamino)-5-(2-methoxy-phenoxy)-2-azepan-1-yl-pyrimidin-4-yloxy]-ethylester, Smp. 201-202°C.

Beispiel 153

Zu 125 mg N-[6-(2-Hydroxy-ethoxy)-2-(2-methoxy-phenoxy)-2,2'-bipyrimidin-4-yl]-4-isobutyl-benzosulfonamid in 2

ml Dichlormethan gelöst, tropfte man 3 ml einer 1,9 molaren $COCl_2$-Lösung in Toluol. Nach 1 Stunde Reaktion bei Raumtemperatur war das Chlorformiat gebildet. Der Ueberschuss an Reagens wurde abdestilliert und der Rückstand wurde in 5 ml THF gelöst. Dann wurden bei heftigem Rühren 2 ml 25%ige Ammoniumhydroxid-Lösung zugegeben. Nach 15 Minuten bei Raumtemperatur wurde das gebildete Carbamat isoliert. Man erhielt 100 mg Carbamylsäure 2-[6-(4-isobutyl-phenylsulfonylamino)-5-(2-methoxy-phenoxy)-2,2'-bipyrimidin-4-yloxy]-ethylester. Smp. 138-140°C (aus Diethylether).

Beispiel 154

In Analogie zu Beispiel 153 erhielt man

Morpholin-4-carbonsäure 2-[6-(4-isobutyl-phenylsulfonylamino)-5-(2-methoxy-phenoxy)-2,2'-bipyrimidin-4-yloxy]-ethylester, Smp. 178-179°C (aus Diethylether),
Imidazol-1-carbonsäure 2-[6-(4-isobutyl-phenylsulfonylamino)-5-(2-methoxy-phenoxy)-2,2'-bipyrimidin-4-yloxy]-ethylester, Smp: Zersetzung bei 153°C,
Imidazol-2-carbonsäure 2-[6-(4-tert.butyl-phenylsulfonyl)-5-(2-methoxy-phenoxy)-2-methylsulfonyl-pyrimidin-4-yloxy]-ethylester, MS: 646,4 ($M+H^{\oplus}$),
Pyridin-2-ylcarbaminsäure 2-[6-(4-tert.butyl-phenylsulfonylamino)-5-(2-methoxy-phenoxy)-2-methylsulfonyl-pyrimidin-4-yloxy]-ethylester, MS: 672,2 ($M+H^{\oplus}$),
(S)-2-[2-[6-(1,3-Benzodioxol-5-ylsulfonylamino)-5-(2-methoxy-phenoxy)-2-phenyl-pyrimidin-4-yloxy]-ethoxycarbonylamino]-3-phenyl-propionsäure-tert.butylester, MS: 785,4 ($M+H^{\oplus}$),
(S)-2-[2-[6-(1,3-Benzodioxol-5-ylsulfonylamino)-5-(2-methoxy-phenoxy)-2-phenyl-pyrimidin-4-yloxy]-ethoxycarbonylamino]-3-phenyl-propionsäureethylester, MS: 757 ($M+H^{\oplus}$),
Thiazol-2-ylcarbaminsäure 2-[5-(2-methoxy-phenoxy)-6-(4-methoxy-phenylsulfonylamino)-2,2'-bipyrimidin-4-yloxy]-ethylester, MS: 650,3 [$(M-H)^-$].

Beispiel 155

In Analogie zu Beispiel 138 erhielt man

Carbonsäure 1,3-benzodioxol-5-ylmethylester 2-[6-(1,3-benzodioxol-5-ylsulfonylamino)-5-(2-chlor-5-methoxy-phenoxy)-pyrimidin-4-yloxy]-ethylester, Smp. 154-155°C (aus Ethanol),
Carbonsäure 2-[6-(1,3-benzodioxol-5-ylsulfonylamino)-5-(2-methoxy-phenoxy)-2-(2-methoxy-phenyl)-pyrimdin-4-yloxy]-ethylester, MS: 692,4 ($M+H^{\oplus}$),
Carbonsäure 2-[6-(4-tert.butyl-phenylsulfonylamino)-5-(2-methoxy-phenoxy)-2,2'-bipyrimidin-4-yloxy]-ethylester-pyridin-3-ylmethylester, MS: 685,4 [$(M-H)^-$],
Carbonsäure 2-[6-(4-tert.butyl-phenylsulfonylamino)-5-(2-methoxy-phenoxy)-2,2'-bipyrimidin-4-yloxy]-ethylester-furan-2-ylmethylester, MS: 674,4 [$(M-H)^-$],
Carbonsäure 2-[6-(4-tert.butyl-phenylsulfonylamino)-5-(2-methoxy-phenoxy)-2,2'-bipyrimidin-4-yloxy]-ethylester-furan-3-ylmethylester, MS: 674,4 [$(M-H)^-$].

Beispiel 156

Durch Verseifung der Ethylestergruppe des [S]-2-[2-[6-(1,3-Benzodioxol-5-ylsulfonylamino)-5-(2-methoxy-phenoxy)-2-phenyl-pyrimidin-4-yloxy]-ethoxycarbonylamino]-3-phenyl-propionsäure-ethylester mit KOH erhielt man (S)-2-[2-[6-(1,3-Benzodioxol-5-ylsulfonylamino)-5-(2-methoxy-phenoxy)-2-phenyl-pyrimidin-4-yloxy]-ethoxycarbonylamino]-3-phenyl-propionsäure (weisser Feststoff). MS: 729,3 ($M+H^+$).

Beispiel 157

Durch Behandlung mit 1N HCl in Dioxan bei 80° während 1 Stunde wurde aus Pyridin-2-ylcarbaminsäure (S)-2-[6-(1,3-benzodioxol-5-ylsulfonylamino)-5-(2-chlor-5-methoxy-phenoxy)-2-(2,2-dimethyl-1,3-dioxolan-4-ylmethoxy)-pyrimidin-4-yloxy]-ethylester der Pyridin-2-ylcarbaminsäure (R)-2-[6-(1,3-benzodioxol-5-sulfonylamino)-5-(2-chlor-5-methoxy-phenoxy)-2-(2,3-dihydroxy-propoxy)-6-(2-hydroxy-ethoxy)-pyrimidin-4-yloxy]-ethylester, Smp. 142-143°C (aus Ethanol) erhalten.

Beispiel A

Tabletten enthaltend die folgenden Bestandteile können in herkömmlicher Weise hergestellt werden:

| Bestandteile | pro Tablette |
|---|---|
| Verbindung der Formel I | 10,0 - 100,0 mg |
| Lactose | 125,0 mg |
| Maisstärke | 75,0 mg |
| Talk | 4,0 mg |
| Magnesiumstearat | 1,0 mg |

Beispiel B

Kapseln enthaltend die folgenden Bestandteile können in herkömmlicher Weise hergestellt werden:

| Bestandteile | pro Kapsel |
|---|---|
| Verbindung der Formel I | 25,0 mg |
| Lactose | 150,0 mg |
| Maisstärke | 20,0 mg |
| Talk | 5,0 mg |

Beispiel C

Injektionslösungen können folgende Zusammensetzung aufweisen:

| | |
|---|---|
| Verbindung der Formel I | 3,0 mg |
| Gelatine | 150,0 mg |
| Phenol | 4,7 mg |
| Wasser für Injektionslösungen | ad 1,0 ml |

Beispiel D

In 3,5 ml Myglyol 812 und 0,08 g Benzylalkohol werden 500 mg Verbindung der Formel I suspendiert. Diese Suspension wird in einen Behälter mit Dosierventil eingefüllt. Es werden 5,0 g Freon 12 unter Druck durch das Ventil in den Behälter abgefüllt. Durch Schütteln wird das Freon in der Myglyol-Benzylalkoholmischung gelöst. Dieser Spraybehälter enthält ca. 100 Einzeldosen, die einzeln appliziert werden können.

**Patentansprüche**

**1.** Verbindungen der Formel

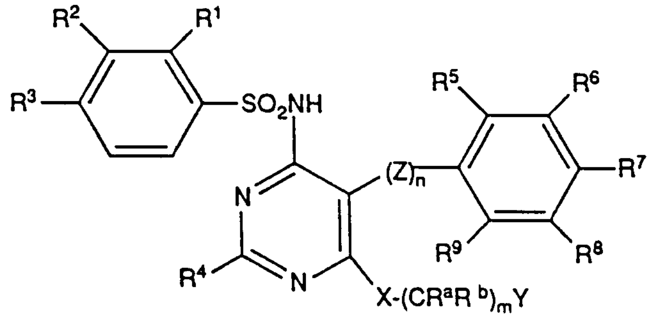

I

worin

| | |
|---|---|
| $R^1$-$R^3$ | unabhängig voneinander Wasserstoff, nieder-Alkyl, nieder-Alkoxy, nieder-Alkylthio, nieder-Alkenyl, Halogen, Trifluormethyl, Hydroxy-nieder-alkoxy, Halogen-nieder-alkoxy, Cyclo-nieder-alkyl, Hydroxyniederalkyl, Aminoniederalkyl, Aminoniederalkoxy, Alkanoylaminoniederalkoxy, Alkanoylaminoniederalkyl, Carboxyniederalkoxy, Carboxyniederalkyl, Niederalkoxycarbonylniederalkyl, Niederalkoxycarbonylniederalkoxy, Alkanoyloxyniederalkoxy, Alkanoyloxyniederalkyl, Alkoxycarbonyl, Carboxy, Amino, mono-oder di-(nieder-Alkyl)amino oder einen Rest $(R^c,R^d)N-C(O)(CH_2)_{0-4}O-$ oder $(R^c,R^d)N-C(O)(CH_2)_{0-4}-$; |
| $R^2$ und $R^3$ | zusammen Butadienyl, Methylendioxy, Aethylendioxy oder Isopropylidendioxy; |
| $R^4$ | Wasserstoff, nieder-Alkyl, Cyclo-nieder-alkyl, Trifluormethyl, nieder-Alkoxy, nieder-Alkinyloxy, nieder-Alkylthio, nieder-Alkylthio-nieder-alkyl, nieder-Alkylthio-nieder-alkoxy, Hydroxy-nieder-alkyl, Hydroxy-nieder-alkoxy, Dihydroxy-nieder-alkoxy, nieder-Alkoxy-nieder-alkyl, Hydroxy-nieder-alkoxy-nieder-alkyl, nieder-Alkoxy-nieder-alkoxy, Di-(nieder-alkoxy)-alkoxy, Hydroxy-nieder-alkoxy-nieder-alkoxy, nieder-Alkylsulfinyl, nieder-Alkylsulfinyl-nieder-alkoxy, nieder-Alkylsulfonyl, 2-Methoxy-3-hydroxypropoxy, 2-Hydroxy-3-phenylpropyl, Amino-nieder-alkyl, nieder-Alkylamino-nieder-alkyl, Di-nieder-alkylamino-nieder-alkyl, Amino, nieder-Alkylamino, Di-nieder-alkylamino, Arylamino, Aryl, Arylthio, Aryloxy, Aryl-nieder-alkyl, Aryl-nieder-alkoxy-nieder-alkyl, Aryl-nieder-alkyl-nieder-alkoxy, Heterocyclyl, Heterocyclyl-nieder-alkyl oder Heterocyclylalkoxy; |
| $R^5$ bis $R^9$ | Wasserstoff, Halogen, Trifluormethyl, nieder-Alkyl, nieder-Alkoxy, nieder-Alkylthio, nieder-Alkylsulfinyl oder nieder-Alkylsulfonyl; |
| $R^6$ | zusammen mit $R^5$ oder $R^7$ Butadienyl, Methylendioxy, Aethylendioxy oder Isopropylidendioxy; |
| $R^{10}$ | nieder-Alkyl, Cyclo-nieder-alkyl, Hydroxy-nieder-alkyl, Carboxy-nieder-alkyl, nieder-Alkoxycarbonyl-nieder-alkyl, nieder-Alkanoyloxy-nieder-alkyl, Aryl, Aryl-nieder-alkyl, Arylcarbamoyl--nieder-alkyl, Heterocyclyl, Heterocyclyl-nieder-alkyl, oder einen Rest $(R^c,R^d)N-C(O)(CH_2)_{1-4}-$; |
| $R^{11}$ | Wasserstoff oder ein Rest $R^{10}$; |
| $R^{10}$ und $R^{11}$ | zusammen mit dem davon gebundenen N-Atom einen 5-7 gliedrigen heterocyclischen Rest; |
| $R^a$ und $R^b$ | unabhängig voneinander Wasserstoff, nieder-Alkyl, nieder-Alkoxy oder nieder-Alkylthio; |
| $R^c$ und $R^d$ | unabhängig voneinander Wasserstoff, nieder-Alkyl oder Aryl; oder $R^c$ und $R^d$ zusammen mit dem daran gebundenen N-Atom einen 5-7-gliedrigen heterocyclischen Rest; |
| Y | einen Rest $-OC(O)NR^{10},R^{11}$, $-NHC(O)NR^{10}R^{11}$, $-OC(O)OR^{10}$ oder $-NHC(O)OR^{10}$; |
| Z | -O-, -S- oder $-CH_2-$; |
| X | -O-, -S- oder -NH-; |
| n | 0 oder 1; |
| m | 1, 2 oder 3 und "nieder" Reste mit 1-7 C-Atomen |

bedeuten,
und pharmazeutisch anwendbare Salze davon.

2. Verbindungen gemäss Anspruch 1, in denen n = 1 und Z = -O- ist.

3. Verbindungen gemäss Anspruch 2, in denen X = -O- ist.

4. Verbindungen gemäss den Ansprüchen 1-3, in denen $R^5$ Halogen, $R^8$ nieder-Alkoxy; und $R^6$, $R^7$ und $R^9$ Wasserstoff sind, und "nieder" Reste mit 1-7 C-Atomen bedeutet.

**5.** Verbindungen gemäss den Ansprüchen 1-4, in denen $R^3$ nieder-Alkyl und $R^1$ und $R^2$ Wasserstoff sind, und "nieder" Reste mit 1-7 C-Atomen bedeutet.

**6.** Verbindungen gemäss den Ansprüchen 1-5, in denen $R^4$ Wasserstoff, nieder-Alkyl, nieder-Alkoxy-nieder-alkyl, nieder-Alkylthio, Phenyl, nieder-Alkoxyphenyl, Phenyl-nieder-alkoxy-nieder-alkyl, Cyclo-nieder-alkyl, Pyrimidinyl, Morpholino oder Thienyl ist, und "nieder" Reste mit 1-7 C-Atomen bedeutet.

**7.** Verbindungen gemäss den Ansprüchen 1-6, in denen Y = $-OC(O)NR^{10}R^{11}$ ist.

**8.** Verbindungen gemäss Anspruch 7, in denen $R^{10}$ Heterocyclyl und $R^{11}$ Wasserstoff ist.

**9.** Verbindungen gemäss Anspruch 8, in denen der Heterocyclylrest $R^{10}$ ein Pyridylrest ist.

**10.** Die Verbindungen gemäss Anspruch 9,

Pyridin-2-ylcarbaminsäure 2-[6-(1,3-benzodioxol-5-ylsulfonylamino)-5-(2-chlor-5-methoxy-phenoxy)-pyrimidin-4-yloxy]-ethylester,
Pyridin-2-ylcarbaminsäure 2-[6-(4-tert-butyl-phenylsulfonylamino)-5-(2-chlor-5-methoxy-phenoxy)-2-methoxymethyl-pyrimidin-4-yloxy]-ethylester,
Pyridin-2-ylcarbaminsäure 2-[6-(1,3-benzodioxol-5-ylsulfonylamino)-5-(2-chlor-5-methoxy-phenoxy)-2-methoxymethyl-pyrimidin-4-yloxy]-ethylester,
Pyridin-2-ylcarbaminsäure 2-[6-(1,3-benzodioxol-5-ylsulfonylamino)-5-(2-chlor-5-methoxy-phenoxy)-2-(2-methoxyethyl)-pyrimidin-4-yloxy]-ethylester,
Pyridin-2-ylcarbaminsäure 2-[6-(1,3-benzodioxol-5-ylsulfonylamino)-5-(2-chlor-5-methoxy-phenoxy)-2-methylsulfanyl-pyrimidin-4-yloxy]-ethylester,
Pyridin-2-ylcarbaminsäure 2-[6-(1,3-benzodioxol-5-ylsulfonylamino)-5-(2-methoxy-phenoxy)-2-(4-methoxyphenyl)-pyrimidin-4-yloxy]-ethylester,
Pyridin-2-ylcarbaminsäure 2-[6-(1,3-benzodioxol-5-ylsulfonylamino)-5-(2-methoxy-phenoxy)-2-phenyl-pyrimidin-4-yloxy]-ethylester,
Pyridin-2-ylcarbaminsäure 2-[6-(1,3-benzodioxol-5-ylsulfonylamino)-5-(3,5-dimethoxy-phenoxy)-pyrimidin-4-yloxy]-ethylester,
Pyridin-2-ylcarbaminsäure 2-[6-(4-tert-butylphenylsulfonylamino)-5-(2-chloro-5-methoxy-phenoxy)-pyrimidin-4-yloxy]ethylester,
1-Oxy-pyridin-2-ylcarbaminsäure 2-[6-(4-tert-butyl-phenyl-sulfonylamino)-5-(2-chloro-5-methoxy-phenoxy)-pyrimidin-4-yloxy]-ethylester,
Pyridin-2-ylcarbaminsäure 2-[6-(tert-butyl-phenylsulfonylamino)-5-(3-methoxy-phenoxy)-pyrimidin-4-yloxy]-ethylester,
Pyridin-2-ylcarbaminsäure 2-[6-(4-tert-butyl-phenylsulfonylamino)-2-isopropyl-5-(2-methoxy-phenoxy)-pyrimidin-4-yloxy]-ethylester,
Pyridin-2-ylcarbaminsäure 2-[6-(4-tert-butyl-phenylsulfonylamino)-5-(2-methoxy-phenoxy)-2-propyl-pyrimidin-4-yloxy]-ethylester,
Pyridin-2-ylcarbaminsäure 2-[2-tert-butyl-6-(4-tert-butyl-phenylsulfonylamino)-5-(2-methoxy-phenoxy)-pyrimidin-4-yloxy]-ethylester,
Pyridin-2-ylcarbaminsäure 2-[6-(4-tert-butyl-phenylsulfonylamino)-2-cyclopropyl-5-(2-methoxy-phenoxy)-pyrimidin-4-yloxy]-ethylester,
Pyridin-2-ylcarbaminsäure 2-[6-(4-tert-butyl-phenylsulfonylamino)-5-(2-methoxy-phenoxy)-2-thiophen-2-yl-pyrimidin-4-yloxy]-ethylester,
Pyridin-2-ylcarbaminsäure 2-[6-(4-tert-butyl-phenylsulfonylamino)-5-(2-methoxy-phenoxy)-2-methyl-pyrimidin-4-yloxy]-ethylester,
Pyridin-2-ylcarbaminsäure-2-[6-(4-tert-butyl-phenylsulfonylamino)-5-(2-methoxy-phenoxy)-2,2'-bipyrimidin-4-yloxy]-ethylester,
Pyridin-2-ylcarbaminsäure-2-[6-(2-tert-butyl-phenylsulfonylamino)-5-(2-methoxyphenoxy)-2-morpholin-4-yl-pyrimidin-4-yloxy]-ethylester,
Pyridin-2-yl-carbaminsäure 2-[6-(4-cyclopropyl-phenylsulfonylamino)-5-(2-methoxy-phenoxy)-2,2'-bipyrimidin-4-yloxy]-ethylester,
Pyridin-2-ylcarbaminsäure 2-[5-(2-methoxy-phenoxy)-6-(4-methylsulsulfanyl-phenylsulfonylamino)-2,2'-bipyrimidin-4-yloxy]-ethylester,
Pyridin-2-ylcarbaminsäure 2-[5-(2-methoxy-phenoxy)-6-(4-vinyl-phenylsulfonylamino)-2,2'-bipyrimidin-4-yloxy]-ethylester,
Pyridyl-2-ylcarbaminsäure 2-[5-(2-bromo-5-methoxy-phenoxy)-6-(4-tert-butyl-phenylsulfonylamino)-pyrimidin-4-yloxy]-ethylester,
Pyridin-2-ylcarbaminsäure 2-[6-(4-tert-butyl-phenylsulfonylamino)-5-(3,4-dimethoxy-phenoxy)-pyrimidin-4-yloxy]-ethylester,
Pyridin-2-ylcarbaminsäure 2-[5-(2-chloro-5-methoxy-phenoxy)-6-(3,4-dimethoxy-phenylsulfonylamino)-pyrimidin-4-yloxy]-ethylester,
Essigsäure 2-[4-5-(2-methoxy-phenoxy)-6-(2-pyridin-2-ylcarbamoyloxyethoxy)-pyrimidin-4-ylsulfamoyl]-phenoxy]-ethylester,
Pyridin-2-ylcarbaminsäure 2-[6-[4-(2-hydroxy-ethoxy)-phenylsulfonylamino]-5-(2-methoxy-phenoxy)-pyrimidin-4-yloxy]-ethylester,
Pyridin-2-ylcarbaminsäure 2-[6-[4-methoxy-3-[2-morpholin-4-yl-2-oxo-ethoxy)-phenylsulfonylamino]-5-(2-methoxy-phenoxy)-pyrimidin-4-yloxy]-ethylester,
Essigsäure 2-[4-[5-(2-chloro-5-methoxy-phenoxy)-6-(2-pyridin-2-ylcarbamoyloxy-ethoxy)-pyrimidin-4-ylsulfamoyl]-phenoxy]-ethylester.
Pyridin-2-ylcarbaminsäure 2-[5-(2-chloro-5-methoxy-phenoxy)-6-[4-(2-hydroxy-ethoxy)-phenylsulfonylamino]-pyrimidin-4-yloxy]-ethylester,
Pyridin-2-ylcarbaminsäure 2-[5-(2-chloro-5-methoxy-phenoxy)-6-[4-methoxy-3-(2-morpholin-4-yl-2-oxo-ethoxy)-phenylsulfonylamino]-pyrimidin-4-yloxy]-ethylester,
Pyridin-2-ylcarbaminsäure 2-[5-(2-chloro-5-methoxy-phenoxy)-6-[4-(2-morpholin-4-yl-2-oxo-ethoxy]-phenylsulfonylamino]-pyrimidin-4-yloxy]-ethylester,
Pyridin-2-ylcarbaminsäure 2-[5-(2-methoxy-phenoxy)-6-[4-(2-morpholin-4-yl-2-oxo-ethoxy)-phenylsulfonylamino]-pyrimidin-4-yloxy]-ethylester,
Pyridin-2-ylcarbaminsäure 2-[5-(2-methoxy-phenoxy)-6-[4-(3-morpholin-4-yl-oxo-propyl)-phenylsulfonylamino]-pyrimidin-4-yloxy]-ethylester,
Pyridin-2-ylcarbaminsäure 2-[5-(2-chloro-5-methoxy-phenoxy)-6-[4-(3-morpholin-4-yl-3-oxo-propyl)-phenylsulfonylamino]-pyrimidin-4-yloxy]-ethylester,
Pyridin-2-ylcarbaminsäure 2-[6-[4-methoxy-3-(3-morpholin-4-yl-3-oxo-propyl)-phenylsulfonylamino]-5-(2-methoxy-phenoxy)-pyrimidin-4-yloxy]-ethylester,
Pyridin-2-ylcarbaminsäure 2-[5-(2-chloro-5-methoxy-phenoxy)-6-[4-methoxy-3-(3-morpholin-4-yl-3-oxo-propyl)-phenylsulfonylamino)-pyrimidin-4-yloxy]-ethylester,
Pyridin-2-ylcarbaminsäure 2-[5-(4-methoxy-phenoxy)-6-[4-methoxy-3-(3-piperidin-1-yl-3-oxo-propyl)-phenylsufonylamino]-pyrimidin-4-yloxy]-ethylester,
Pyridin-2-ylcarbaminsäure 2-[5-(2-methoxy-phenoxy)-6-[4-methoxy-3-(3-piperidin-1-yl-3-oxo-propyl)-phenylsulfonylamino]-2,2'-bipyrimidin-4-yloxy]-ethylester,
Pyridin-2-ylcarbaminsäure 2-[6-[4-methoxy-2-(3-morpholin-4-yl-3-oxo-propyl)-phenylsulfonylamino]-5-(2-methoxy-phenoxy)-pyrimidin-4-yloxy]-ethylester,
Pyridin-2-ylcarbaminsäure 2-[6-[4-(2-bromo-ethoxy)-phenylsulfonylamino]-5-(2-methoxy-phenoxy)-pyrimidin-4-yloxy]-ethylester,
Essigsäure 3-[4-[5-(2-methoxy-phenoxy)-6-(2-pyridin-2-ylcarbamoyloxy-ethoxy)-pyrimidin-4-ylsulfamoyl]-phenoxy]-propylester,
Pyridin-2-ylcarbaminsäure 2-[6-[4-(3-hydroxy-propoxy)-phenylsulfonylamino]-5-(2-methoxy-phenoxy)-pyrimidin-4-yloxy]-ethylester,
Pyridin-2-ylcarbaminsäure 2-[6-[4-methoxy-3-(2-morpholin-4-yl-2-oxo-ethyl)-phenylsulfonylamino]-5-(2-methoxy-phenoxy)-pyrimidin-4-yloxy]-ethylester,
Pyridin-2-ylcarbaminsäure 2-[6-(1,3-benzodioxol-5-ylsulfonylamino)-2-(2-benzyloxy-ethyl]-5-(2-chloro-5-methoxy-phenoxy)-pyrimidin-4-yloxy]-ethylester,
Pyridin-2-ylcarbaminsäure 2-[5-(2-chloro-5-methoxy-phenoxy)-6-(3-isopropyl-4-methoxy-phenylsulfonylamino)-pyrimidin-4-yloxy]-ethylester,
Pyridin-2-ylcarbaminsäure 2-[6-(1,3-benzodioxol-5-ylsulfonylamino)-5-(2-chloro-5-methoxy-phenoxy)-2-(3-methoxy-propyl)-pyrimidin-4-yloxy]-ethylester,
Pyridin-2-ylcarbaminsäure 2-[6-(4-dimethylaminophenylsulfonylamino)-5-(2-methoxy-phenoxy)-2-morpholin-4-yl-pyrimidin-4-yloxy]-ethylester.

**11.** Die Verbindungen gemäss Anspruch 9,

Pyridin-3-ylcarbaminsäure 2-[5-(2-chloro-5-methoxy-phenoxy]-6-(2,3-dihydro-1,4-benzodioxin-6-ylsulfonylamino)-pyrimidin-4-yloxy]-ethylester,

Pyridin-4-ylcarbaminsäure 2-[6-(1,3-benzodioxol-5-ylsulfonylamino)-5-(2-chlor-5-methoxy-phenoxy)-pyrimidin-4-yloxy]-ethylester,
Pyridin-3-ylcarbaminsäure 2-[6-(1,3-benzodioxol-5-ylsulfonylamino)-5-(2-chlor-5-methoxy-phenoxy)-pyrimidin-4-yloxy)-ethylester,
Pyridin-3-ylcarbaminsäure 2-[6-(4-tert-butyl-phenylsulfonylamino)-5-(2-chlor-5-methoxy-phenoxy)-2-methoxymethyl-pyrimidin-4-yloxy]-ethylester,
Pyridin-3-ylcarbaminsäure 2-[6-(4-tert-butylphenylsulfonylamino)-5-(2-chloro-5-methoxy-phenoxy)-pyrimidin-4-yloxy]ethylester,
Pyridin-4-ylcarbaminsäure 2-[6-(4-tert-butylphenylsulfonylamino)-5-(2-chloro-5-methoxy-phenoxy)-pyrimidin-4-yloxy]ethylester,
1-Oxy-pyridin-4-ylcarbaminsäure 2-[6-(4-tert-butyl-phenylsulfonylamino)-5-(2-chloro-5-methoxyphenoxy)-pyrimidin-4-yloxy]-ethylester,
3-[2-[5-(2-Chloro-5-methoxy-phenoxy)-6-(2,3-dihydro-1,4-benzodioxin-6-ylsulfonylamino)-pyrimidin-4-yloxy]-ethoxycarbonylamino]-1-methylpyridiniumjodid,
N-[5-(2-Chloro-5-methoxy-phenoxy)-6-[2-(1-methyl-pyridin-3-yliocarbamoyloxy)-ethoxy]-pyrimidin-4-yl]-1,3-benzodioxol-5-sulfonamid,
Pyridin-4-ylcarbaminsäure 2-[6-(4-tert-butyl-phenylsulfonylamino)-5-(3-methoxy-phenoxy)-pyrimidin-4-yloxy]-ethylester,
4-[2-[6-(4-tert-Butyl-phenylsulfonylamino)-5-(2-chloro-5-methoxy-phenoxy-pyrimidin-4-yloxy]-ethoxycarbonylamino]-1-methyl-pyridiniumjodid,
Pyridin-4-ylcarbaminsäure 2-[6-(4-tert-butyl-phenylsulfonylamino)-2-isopropyl-5-(2-methoxy-phenoxy)-pyrimidin-4-yloxy]-ethylester,
Pyridin-4-ylcarbaminsäure 2-[6-(4-tert-butyl-phenylsulfonylamino)-5-(2-methoxy-phenoxy)-2-propyl-pyrimidin-4-yloxy]-ethylester,
Pyridin-4-ylcarbaminsäure 2-[2-tert-butyl-6-(4-tert-butylphenylsulfonylamino)-5-(2-methoxy-phenoxy)-pyrimidin-4-yloxy]-ethylester,
Pyridin-4-ylcarbaminsäure 2-[6-(4-tert-butyl-phenylsulfonylamino)-2-cyclopropyl-5-(2-methoxy-phenoxy)-pyrimidin-4-yloxy]-ethylester,
Pyrimidin-4-ylcarbaminsäure 2-[6-(4-tert-butyl-phenylsulfonylamino)-5-(2-methoxy-phenoxy)-2-thiophen-2-yl-pyrimidin-4-yloxy]-ethylester,
Pyridin-4-ylcarbaminsäure 2-[6-(4-tert-butyl-phenylsulfonylamino)-5-(2-methoxy-phenoxy)-2-methyl-pyrimidin-4-yloxy]-ethylester,
Pyridin-4-ylcarbaminsäure 2-[6-(4-tert-butyl-phenylsulfonylamino)-5-(2-methoxyphenoxy)-2,2'-bipyrimidin-4-yloxy]-ethylester,
Pyridin-4-ylcarbaminsäure 2-[6-(2-tert-butyl-phenylsulfonylamino)-5-(2-methoxy-phenoxy)-2-morpholin-4-yl-pyrimidin-4-yloxy]-ethylester,
Pyridin-3-ylcarbaminsäure 2-[6-(2-tert-butyl-phenylsulfonylamino)-5-(2-methoxy-phenoxy)-2-morpholin-4-yl-pyrimidin-4-yloxy]-ethylester,
Pyridin-4-ylcarbaminsäure 2-[6-(4-tert-butyl-phenylsulfonylamino)-5-(3,4-dimethoxy-phenoxy)-pyrimidin-4-yloxy]-ethylester,
Essigsäure 2-[4-5-(2-methoxy-phenoxy)-6-(2-pyridin-4-ylcarbamoyloxy-ethoxy)-pyrimidin-4-ylsulfamoyl]-phenoxy]-ethylester,
Essigsäure 2-[4-[5-(2-methoxy-phenoxy)-6-(2-pyridin-4-ylcarbamoyloxy-ethoxy)-pyrimidin-4-ylsulfamoyl]-phenoxy]-ethylester,
Pyridin-4-ylcarbaminsäure 2-[6-[4-(2-hydroxy-ethoxy)-phenylsulfonylamino]-5-(2-methoxy-phenoxy)-pyrimidin-4-yloxy]-ethylester,
Pyridin-3-ylcarbaminsäure 2-[6-[4-(2-hydroxy-ethoxy)-phenylsulfonylamino]-5-(2-methoxy-phenoxy)-pyrimidin-4-yloxy]-ethylester,
Pyridin-4-ylcarbaminsäure 2-[6-[4-methoxy-3-[2-morpholin-4-yl-2-oxo-ethoxy)-phenylsulfonylamino]-5-(2-methoxy-phenoxy)-pyrimidin-4-yloxy]-ethylester,
Pyridin-4-ylcarbaminsäure 2-[5-(2-chloro-5-methoxy-phenoxy)-6-[4-methoxy-3-(2-morpholin-4-yl-2-oxo-ethoxy)-phenylsulfonylamino]-pyrimidin-4-yloxy]-ethylester,
Pyridin-4-ylcarbaminsäure 2-[5-(2-methoxy-phenoxy)-6-[4-(2-morpholin-4-yl-2-oxo-ethoxy)-phenylsulfonylamino]-pyrimidin-4-yloxy]-ethylester.

**12.** Die Verbindungen gemäss Anspruch 8,

1-Methyl-pyrrol-2-carbamylsäure 2-[6-(1,3-benzodioxol-5-ylsulfonylamino)-5-(2-chlor-5-methoxy-phenoxy)-pyrimidin-4-yloxy]-ethylester,
Thiophen-3-ylcarbaminsäure 2-[6-(4-tert-butyl-phenylsulfonylamino)-5-(2-chlor-5-methoxy-phenoxy)-pyrimidin-4-yloxy]-ethylester,
Thiophen-3-yl-carbamylsäure 2-[6-(1,3-benzodioxol-5-ylsulfonylamino)-5-(2-chlor-5-methoxy-phenoxy)-pyrimidin-4-yloxy]-ethylester,
Thiophen-3-ylcarbaminsäure 2-[6-(1,3-benzodioxol-5-ylsulfonylamino)-5-(2-chlor-5-methoxy-phenoxy)-2-methoxymethyl-pyrimidin-4-yloxy]-ethylester,
Thiophen-2-ylcarbamylsäure 2-[6-(1,3-benzodioxol-5-ylsulfonylamino)-5-(2-chlor-5-methoxy-phenoxy)-pyrimidin-4-yloxy]-ethylester,
Thiophen-3-ylcarbaminsäure 2-[6-(1,3-benzodioxol-5-ylsulfonylamino)-5-(2-chlor-5-methoxy-phenoxy)-2-(2-methoxy-ethyl)-pyrimidin-4-yloxy]-ethylester,
Pyrazin-2-ylcarbaminsäure 2-[6-(4-tert-butylphenylsulfonylamino)-5-(2-chloro-5-methoxy-phenoxy)-pyrimidin-4-yloxy]ethylester,
Chinolin-2-ylcarbaminsäure-2-[6-(4-tert-butyl-phenylsulfonylamino)-5-(3-methoxy-phenoxy)-pyrimidin-4-yloxy]-ethylester,
Furan-3-ylcarbaminsäure 2-[6-(1,3-benzodioxol-5-ylsulfonylamino)-5-(2-chloro-5-methoxy-phenoxy)-pyrimidin-4-yloxy]-ethylester,
Furan-2-ylcarbaminsäure 2-[6-(4-tert-butyl-phenylsulfonylamino)-5-(2-methoxy-phenoxy)-2-morpholin-4-yl-pyrimidin-4-yloxy]-ethylester,
3-Methyl-isoxazol-5-yl-carbaminsäure 2-[6-(4-tert-butyl-phenylsulfonylamino)-5-(2-methoxy-phenoxy)-2-morpholin-4-yl-pyrimidin-4-yloxy]-ethylester,
3-Methyl-isoxazol-5-ylcarbaminsäure 2-[5-(2-methoxy-phenoxy)-6-(4-methylsulfanyl-phenylsulfonylamino)-2,2'-bipyrimidin-4-yloxy-ethylester,
Pyrazin-2-ylcarbaminsäure 2-[6-[4-methoxy-3-[2-morpholin-4-yl-2-oxo-ethoxy)-phenylsulfonylamino]-5-(2-methoxy-phenoxy)-pyrimidin-4-yloxy]-ethylester,
Pyrazin-2-ylcarbaminsäure 2-[5-(2-chloro-5-methoxy-phenoxy)-6-[4-methoxy-3-(2-morpholin-4-yl-2-oxo-ethoxy)-phenylsulfonylamino]-pyrimidin-4-yloxy]-ethylester,
Pyrazin-2-ylcarbaminsäure 2-[5-(2-chloro-5-methoxy-phenoxy)-6-[4-(2-morpholin-4-yl-2-oxo-ethoxy]-phenylsulfonylamino]-pyrimidin-4-yloxy]-ethylester,

Pyrimidin-2-ylcarbaminsäure 2-[5-(2-chloro-5-methoxy-phenoxy)-6-[4-methoxy-3-(3-morpholin-4-yl-3-oxo-propyl)-phenylsulfonylamino]-pyrimidin-4-yloxy]-ethylester.

**13.** Verbindungen gemäss Anspruch 7, in denen $R^{10}$ Aryl oder Cyclonieder-alkyl und $R^{11}$ Wasserstoff ist, und "nieder" Reste mit 1-7 C-Atomen bedeutet.

**14.** Die Verbindungen gemäss Anspruch 13,

1,3-Benzodioxol-5-ylcarbaminsäure 2-[6-(4-tert-butyl-phenylsulfonylamino)-5-(2-chlor-5-methoxy-phenoxy)-pyrimidin-4-yloxy]-ethylester,
3-Fluorophenylcarbaminsäure 2-[6-(4-tert-butylphenylsulfonylamino)-5-(2-chloro-5-methoxy-phenoxy)-pyrimidin-4-yloxy]ethylester,
2-Fluorophenylcarbaminsäure 2-[(6-(4-tert-butyl-phenylsulfonylamino)-5-(2-chloro-5-methoxy-phenoxy)-pyrimidin-4-yloxy]ethylester,
Phenylcarbaminsäure 2-[6-(4-tert-butyl-phenylsulfonylamino)-5-(2-chloro-5-methoxy-phenoxy)pyrimidin-4-yloxy]ethylester,
4-Chloro-phenylcarbaminsäure 2-[6-(4-tert-butyl-phenylsulfanoylamino)-5-(2-chloro-5-methoxy-phenoxy)-pyrimidin-4-yloxy]ethylester,
3-Methoxy-phenylcarbaminsäure 2-[6-(4-tert-butyl-phenylsulfonylamino)-5-(2-chloro-5-methoxy-phenoxy)-pyrimidin-4-yloxy]ethylester,
4-Trifluoromethyl-phenylcarbaminsäure 2-[6-(4-tert-butyl-phenylsulfonylamino)-5-(2-chloro-5-methoxy-phenoxy)-pyrimidin-4-yloxy]-ethylester,
2-[2-[6-(4-tert-Butyl-phenylsulfonylamino)-5-(2-chloro-5-methoxy)-pyrimidin-4-yloxy]-ethoxycarbonylamino]-benzoesäure-methylester,
3-Tolylcarbaminsäure 2-(6-(4-tert-butyl-phenylsulfonylamino)-5-(2-chloro-5-methoxy-phenoxy)-pyrimidin-4-yloxy]ethylester,
2-Methoxy-phenoxycarbaminsäure 2-[6-(4-tert-butylphenylsulfonylamino)-5-(2-chloro-5-methoxy-phenoxy)-pyrimidin-4-yloxy]ethylester,

Essigsäure 2-[2-[(6-tert-butyl-phenylsulfonylamino)-5-(2-chloro-5-methoxy)-pyrimidin-4-yloxy]-ethoxycarbonylamino]-phenylester,
2-Hydroxy-phenylcarbaminsäure 2-[6-(4-tert-butylphenylsulfonylamino)-5-(2-chloro-5-methoxy-phenoxy)-pyrimidin-4-yloxy]ethylester,
Benzylcarbaminsäure 2-[6-(4-tert-butylphenylsulfonylamino)-5-(2-chloro-5-methoxy-phenoxy)pyrimidin-4-yloxy]ethylester,
Phenylcarbaminsäure 2-[6-(4-tert-butyl-phenylsulfonylamino)-5-(3-methoxy-phenoxy)-pyrimidin-4-yloxy]-ethylester,
(R)-1-Phenyl-ethylcarbaminsäure-2-[6-(4-tert-butyl-phenylsulfonylamino)-5-(3-methoxy-phenoxy)-pyrimidin-4-yloxy]-ethylester,
Cyclohexylcarbaminsäure 2-[6-(4-tert-butyl-phenylsulfonylamino)-5-(3-methoxy-phenoxy)-pyrimidin-4-yloxy]-ethylester,
Essigsäure 2-[4-[5-(2-methoxy-phenoxy)-6-(2-phenylcarbamoyloxy-ethoxy)-pyrimidin-4-ylsulfamoyl]-phenoxy]-ethylester,
Essigsäure 2-[4-[6-[2-(2-fluoro-phenylcarbamoyloxy)-ethoxy]-5-(2-methoxy-phenoxy)-pyrimidin-4-ylsulfamoyl]-phenoxy]-ethylester,
Phenylcarbaminsäure 2-[6-[4-(2-hydroxy-ethoxy)-phenylsulfonylamino]-5-(2-methoxy-phenoxy)-pyrimidin-4-yloxy]-ethylester,
2-Fluoro-phenylcarbaminsäure 2-[6-[4-(2-hydroxy-ethoxy)-phenylsulfonylamino]-5-(2-methoxy-phenoxy)-pyrimidin-4-yloxy]-ethylester,
2-Fluoro-phenylcarbaminsäure 2-[6-[4-methoxy-3-(2-morpholin-4-yl-2-oxo-ethoxy)-phenylsulfonylamino]-5-(2-methoxy-phenoxy)-pyrimidin-4-yloxy]-ethylester,
Essigsäure 2-[4-[5-(2-chloro-5-methoxy-phenoxy)-6-[2-(2-fluoro-phenylcarbamoyloxy)-ethoxy]-pyrimidin-4-ylsulfamoyl]-phenoxy]-ethylester,
2-Fluoro-phenylcarbaminsäure 2-[5-(2-chloro-5-methoxy-phenoxy)-6-[4-(2-hydroxy-ethoxy)-phenylsulfonylamino]-pyrimidin-4-yloxy]-ethylester,
2-Fluoro-phenylcarbaminsäure 2-[5-(2-chloro-5-methoxy-phenoxy)-6-[4-methoxy-3-(2-morpholin-4-yl-2-oxo-ethoxy)-phenylsulfonylamino]-pyrimidin-4-yloxy]-ethylester,
Phenylcarbaminsäure 2-[6-[4-methoxy-3-(2-morpholin-4-yl-2-oxo-ethoxy)-phenylsulfonylamino]-5-(3-methoxy-phenoxy)-pyrimidin-4-yloxy]-ethylester,
[5-[5-(2-Chloro-5-methoxy-phenoxy)-6-[2-(2-fluorophenylcarbamoyloxy)-ethoxy]-pyrimidin-4-ylsulfamoyl]-2-methoxy-phenoxy]-essigsäure-ethylester,
5-[5-(2-Chloro-5-methoxy-phenoxy)-6-[2-(2-fluoro-phenylcarbamoyloxy)-ethoxy]-pyrimidin-4-ylsulfamoyl]-2-methoxy-phenoxy]-essigsäure,
2-Fluoro-phenylcarbaminsäure 2-[6-[4-methoxy-3-(2-oxo-2-piperidin-1-yl-ethoxy)-phenylsulfonylamino]-5-(2-methoxy-phenoxy)-pyridin-4-yloxy]-ethylester.

**15.** Die Verbindungen gemäss Anspruch 7,

Isopropylcarbaminsäure 2-[6-(4-tert-butyl-phenylsulfonylamino)-5-(2-chloro-5-methoxy-phenoxy)-pyrimidin-4-yloxy]ethylester,
Ethylcarbaminsäure 2-[6-(4-tert-butyl-phenylsulfonylamino)-5-(2-chloro-5-methoxy-phenoxy)-pyrimidin-4-yloxy]-ethylester,
[2-[6-(4-tert-Butyl-phenylsulfonylamino)-5-(3-methoxy-phenoxy)-pyrimidin-4-yloxy]-ethoxycarbonylamino]-essigsäureethylester,
2-[6-(4-tert-Butyl-phenylsulfonylamino)-5-(3-methoxy-phenoxy)-pyrimidin-4-yloxy]-ethoxycarbonylamino-essigsäure,
2-Hydroxy-ethylcarbaminsäure 2-[6-(4-tert-butyl-phenylsulfonylamino)-5-(3-methoxy-phenoxy)-pyrimidin-4-yloxy]-ethylester,
Morpholin-4-carbonsäure 2-[6-(4-tert-butyl-phenylsulfonylamino)-5-(3-methoxy-phenoxy)-pyrimidin-4-yloxy]-ethylester,
2-Morpholin-4-yl-2-oxo-ethylcarbaminsäure 2-[6-(4-tert-butyl-phenylsulfonylamino)-5-(3-methoxy-phenoxy)-pyrmidin-4-yloxy]-ethylester,
2-Oxo-2-pyrrolidin-1-yl-ethylcarbaminsäure 2-[6-(4-tert-butyl-phenylsulfonylamino)-5-(3-methoxy-phenoxy)-pyrimidin-4-yloxy]ethylester,
Phenylcarbamoylmethylcarbaminsäure 2-[6-(4-tert-butyl-phenylsulfonylamino)-5-(3-methoxy-phenoxy)-pyrimidin-4-yloxy]ethylester,
2-[2-[6-(4-tert-Butyl-phenylsulfonylamino)-5-(3-methoxy-phenoxy)-pyrimidin-4-yloxy]-ethoxycarbonylamino]-4-methyl-pentansäureethylester.

**16.** Verbindungen gemäss den Ansprüchen 1-6, in denen Y $-NHC(O)NR^{10}R^{11}$ ist.

**17.** Die Verbindungen gemäss Anspruch 16,

4-tert-Butyl-N-[5-(2-chloro-5-methoxy-phenoxy)-6-[2-(3-phenyl-ureido)-ethoxy]-pyrimidin-4-yl]-benzolsulfonamid,
4-tert-Butyl-N-[5-(2-chloro-5-methoxy-phenoxy)-6-[2-[3-(2-fluoro-phenyl)-ureido]-ethoxy]-pyrimidin-4-yl]-benzolsulfonamid,
4-tert-Butyl-N-[5-(2-chloro-5-methoxy-phenoxy)-6-[2-(3-pyridin-2-yl-ureido)-ethoxy]-pyrimidin-4-yl]-benzolsulfonamid,
4-tert-Butyl-N-[5-(2-chloro-5-methoxy-phenoxy)-6-[2-(3-pyridin-4-yl-ureido)-ethoxy]-pyrimidin-4-yl[-benzolsulfonamid,
4-tert-Butyl-N-[5-(2-chloro-5-methoxy-phenoxy)-6-[2-(3-pyridin-3-yl-ureido)-ethoxy]-pyrimidin-4-yl]-benzolsulfonamid,
4-tert-Butyl-N-[5-(2-chloro-5-methoxy-phenoxy)-6-[2-(3-1-oxy-pyridin-4-yl)-ureido]-ethoxy]-pyrimidin-4-yl]-benzolsulfonamid,
4-tert-Butyl-N-[5-(2-methoxy-phenoxy)-2-methyl-6-[2-(3-pyridin-2-yl-ureido)-ethoxy]-pyrimidin-4-yl]-benzolsulfonamid.

**18.** Verbindungen gemäss den Ansprüchen 1-6, in denen Y $-OC(O)COR^{10}$ ist.

**19.** Die Verbindungen gemäss Anspruch 18,

Carbonsäure 2-[6-(1,3-benzodioxol-5-ylsulfonylamino)-5-(3,5-dimethoxy-phenoxy)-pyrimidin-4-yloxy]-ethylester-pyridin-3-ylmethylester,
Carbonsäure 2-[6-(1,3-benzodioxol-5-sulfonylamino)-5-(2-chlor-5-methoxy-phenoxy)-pyrimidin-4-yloxy]-ethylester-pyridin-2-ylmethylester,
Carbonsäure 2-[6-(1,3-benzodioxol-5-ylsulfonylamino)-5-(2-chlor-5-methoxy-phenoxy)-pyrimidin-4-yloxy]-ethylester-pyridin-2-ylmethylester,
Carbonsäure 2-[6-(4-tert-butyl-benzolsulfonylamino)-5-(2-chlor-5-methoxy-phenoxy)-pyrimidin-4-yloxy]-ethylester-pyridin-2-ylmethylester,
Carbonsäure 2-[6-(1,3-benzodioxol-5-ylsulfonylamino)-5-(2-chlor-5-methoxy-phenoxy)-pyrimidin-4-yloxy]-ethylester-furan-3-ylmethylester,
Carbonsäure 2-[6-(4-tert-butyl-phenylsulfonylamino)-5-(2-chloro-5-methoxy-phenoxy)-2-methyl-pyrimidin-4-yloxy]-ethylester-pyridin-3-yl-methylester,
Carbonsäure 2-[6-(4-tert-butyl-phenylsulfonylamino)-5-(2-chloro-5-methoxy-phenoxy)-2-methyl-pyrimidin-4-yloxy]-ethylester-pyridin-2-ylmethylester,
Carbonsäure 2-[6-(4-tert-butyl-phenylsulfonylamino)-5-(2-chloro-5-methoxy-phenoxy)-2-methyl-pyrimidin-4-yloxy]-ethylester-pyrimidin-4-ylmethylester,
Carbonsäure 2-[6-(4-tert-Butyl-phenylsulfonylamino)-5-(2-methoxy-phenylsulfanyl)-2,2'-bipyrimidin-4-yloxy]-ethylester-pyridin-3-ylmethylester,
Carbonsäure 2-[6-(4-tert-Butyl-phenylsulfonylamino)-5-(2-methoxy-phenylsulfanyl)-2-methyl-pyrimidin-4-yloxy]-ethylester-pyridin-3-ylmethylester.

**20.** Verbindungen gemäss Anspruch 1, in denen n = 1 und Z = -S- ist.

**21.** Die Verbindungen gemäss Anspruch 20,

Pyridin-2-ylcarbaminsäure 2-[6-(4-tert-butyl-phenylsulfonylamino)-5-(2-methoxy-phenylsulfanyl)-pyrimidin-4-yloxy)-ethylester,
Pyridin-3-ylcarbaminsäure 2-[6-tert-butyl-phenylsulfonylamino)-5-(2-methoxy-phenylsulfanyl)-2,2'-bipyrimidin-4-yloxy]-ethylester,
Pyridin-4-ylcarbaminsäure 2-[6-(4-tert-butyl-phenylsulfonylamino)-5-(2-methoxy-phenylsulfanyl)-2-methyl-pyrimidin-4-yloxy]-ethylester.

**22.** Die Verbindungen der Ansprüche 1-21 zur Anwendung als Heilmittel.

23. Pharmazeutische Präparate, enthaltend eine Verbindung der Ansprüche 1-21 und übliche Träger- und Hilfsstoffe.

24. Verwendung von Verbindungen der Ansprüche 1-21 als Wirkstoffe bei der Herstellung von Heilmitteln zur Behandlung von Erkrankungen, die mit Endothelin-Aktivitäten assoziiert sind, insbesondere Kreislauferkrankungen wie Hypertonie, Ischämie, Vasopasmen und Angina pectoris.

25. Verfahren zur Herstellung von Verbindungen der Ansprüche 1-21, dadurch gekennzeichnet, dass man eine Verbindung der Formel

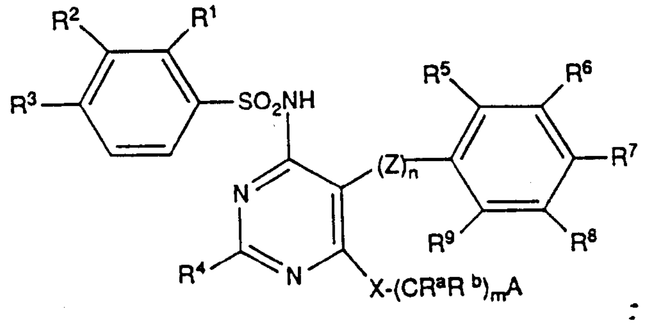

II

worin $R^1$-$R^9$, $R^a$, $R^b$, X, Z, m und n die in Anspruch 1 angegebene Bedeutung haben und A Hydroxy oder Amino ist,

a) mit einem Isocyanat der Formel $R^{10}NCO$ oder einem Carbamoylchlorid der Formel $(R^{10}R^{11})NCOCl$ umsetzt, worin $R^{10}$ und $R^{11}$ der in Anspruch 1 angegebene Bedeutung haben, oder
b) mit Phosgen und danach mit einem Alkohol der Formel $R^{10}OH$; oder mit einem Chlorameisensäureester der Formel $R^{10}OC(O)Cl$ umsetzt,

und gewünschtenfalls in der so erhaltenen Verbindung der Formel I anwesende Substituenten abwandelt und gewünschtenfalls eine Verbindung der Formel I in ein pharmazeutisch anwendbares Salz überführt.

## Claims

1. Compounds of the formula

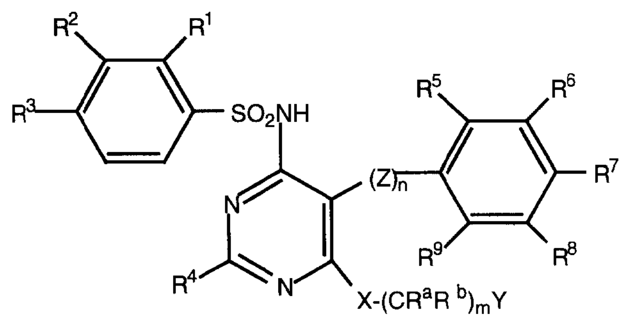

I

wherein

| | |
|---|---|
| $R^1$-$R^3$ | each independently signify hydrogen, lower-alkyl, lower-alkoxy, lower-alkylthio, lower-alkenyl, halogen, trifluoromethyl, hydroxy-lower-alkoxy, halo-lower-alkoxy, cyclo-lower-alkyl, hydroxy-lower-alkyl, amino-lower alkyl, amino-lower alkoxy, alkanoylamino-lower-alkoxy, alkanoylamino-lower-alkyl, carboxy-lower-alkoxy, carboxy-lower-alkyl, lower-alkoxycarbonyl-lower-alkyl, lower-alkoxycarbonyl-lower-alkoxy, alkanoyloxy-lower-alkoxy, alkanoyloxy-lower-alkyl, alkoxycarbonyl, carboxy, amino, mono- or di-(lower-alkyl)amino or a residue $(R^c,R^d)N-C(O)(CH_2)_{0-4}O-$ or $(R^c,R^d)N-C(O)(CH_2)_{0-4}-$; |
| $R^2$ and $R^3$ | together signify butadienyl, methylenedioxy, ethylenedioxy or isopropylidenedioxy; |

| | |
|---|---|
| $R^4$ | signifies hydrogen, lower-alkyl, cyclo-lower alkyl, trifluoromethyl, lower-alkoxy, lower-alkynyloxy, lower-alkylthio, lower-alkylthio-lower-alkyl, lower-alkylthio-lower alkoxy, hydroxy-lower-alkyl, hydroxy-lower-alkoxy, dihydroxy-lower alkoxy, lower-alkoxy-lower-alkyl, hydroxy-lower-alkoxy-lower-alkyl, lower-alkoxy-lower alkoxy, di-(lower-alkoxy)-alkoxy, hydroxy-lower-alkoxy-lower-alkoxy, lower-alkylsulphinyl, lower-alkylsulphinyl-lower-alkoxy, lower-alkylsulphonyl, 2-methoxy-3-hydroxypropoxy, 2-hydroxy-3-phenylpropyl, amino-lower-alkyl, lower-alkylamino-lower-alkyl, di-lower-alkylamino-lower-alkyl, amino, lower-alkylamino, di-lower-alkylamino, arylamino, aryl, arylthio, aryloxy, aryl-lower-alkyl, aryl-lower-alkoxy-lower-alkyl, aryl-lower-alkyl-lower-alkoxy, heterocyclyl, heterocyclyl-lower-alkyl or heterocyclylalkoxy; |
| $R^5$ to $R^9$ | signify hydrogen, halogen, trifluoromethyl, lower-alkyl, lower-alkoxy, lower-alkylthio, lower-alkylsulphinyl or lower-alkylsulphonyl; |
| $R^6$ | and $R^5$ or $R^7$ together signify butadienyl, methylenedioxy, ethylenedioxy or isopropylidenedioxy; |
| $R^{10}$ | signifies lower-alkyl, cyclo-lower-alkyl, hydroxy-lower-alkyl, carboxy-lower-alkyl, lower-alkoxycarbonyl-lower-alkyl, lower-alkanoyloxy-lower-alkyl, aryl, aryl-lower-alkyl, arylcarbamoyl-lower-alkyl, heterocyclyl, heterocyclyl-lower-alkyl or a residue $(R^c,R^d)N-C(O)(CH_2)_{1-4}$-; |
| $R^{11}$ | signifies hydrogen or a residue $R^{10}$; |
| $R^{10}$ and $R^{11}$ | together with the N atom to which they are attached signify a 5-7 membered heterocyclic residue; |
| $R^a$ and $R^b$ | each independently signify hydrogen, lower-alkyl, lower-alkoxy or lower-alkylthio; |
| $R^c$ and $R^d$ | each independently signify hydrogen, lower-alkyl or aryl; or $R^c$ and $R^d$ together with the N atom to which they are attached signify a 5-7-membered heterocyclic residue; |
| Y | signifies a residue $-OC(O)NR^{10}R^{11}$, $-NHC(O)NR^{10}R^{11}$, $-OC(O)OR^{10}$ or $-NHC(O)OR^{10}$; |
| Z | signifies -O-, -S- or $-CH_2$-; |
| X | signifies -O-, -S- or -NH-; |
| n | signifies 0 or 1; |
| m | signifies 1, 2 or 3 and "lower" signifies residues with 1-7 C atoms, |

and pharmaceutically usable salts thereof.

2. Compounds according to claim 1, in which n = 1 and Z = -O-.

3. Compounds according to claim 2, in which X = -O-.

4. Compounds according to claims 1-3, in which $R^5$ is halogen, $R^8$ is lower-alkoxy; and $R^6$, $R^7$ and $R^9$ are hydrogen, and "lower" signifies residues with 1-7 C atoms.

5. Compounds according to claims 1-4, in which $R^3$ is lower-alkyl and $R^1$ and $R^2$ are hydrogen, and "lower" signifies residues with 1-7 C atoms.

6. Compounds according to claims 1-5, in which $R^4$ is hydrogen, lower-alkyl, lower-alkoxy-lower-alkyl, lower-alkylthio, phenyl, lower-alkoxyphenyl, phenyl-lower-alkoxy-lower-alkyl, cyclo-lower-alkyl, pyrimidinyl, morpholino or thienyl, and "lower" signifies residues with 1-7 C atoms.

7. Compounds according to claims 1-6, in which $Y = -OC(O)NR^{10}R^{11}$.

8. Compounds according to claim 7, in which $R^{10}$ is heterocyclyl and $R^{11}$ is hydrogen.

9. Compounds according to claim 8, in which the heterocyclyl residue $R^{10}$ is a pyridyl residue.

10. The compounds according to claim 9,

pyridin-2-ylcarbamic acid 2-[6-(1,3-benzodioxol-5-ylsulphonylamino)-5-(2-chloro-5-methoxy-phenoxy)-pyrimidin-4-yloxy]-ethyl ester,
pyridin-2-ylcarbamic acid 2-[6-(4-tert-butyl-phenylsulphonylamino)-5-(2-chloro-5-methoxy-phenoxy)-2-methoxymethyl-pyrimidin-4-yloxy]-ethyl ester,
pyridin-2-ylcarbamic acid 2-[6-(1,3-benzodioxol-5-ylsulphonylamino)-5-(2-chloro-5-methoxy-phenoxy)-2-methoxymethyl-pyrimidin-4-yloxy]-ethyl ester,
pyridin-2-ylcarbamic acid 2-[6-(1,3-benzodioxol-5-ylsulphonylamino)-5-(2-chloro-5-methoxy-phenoxy)-2-(2-methoxyethyl)-pyrimidin-4-yloxy]-ethyl ester,

pyridin-2-ylcarbamic acid 2-[6-(1,3-benzodioxol-5-ylsulphonylamino)-5-(2-chloro-5-methoxy-phenoxy)-2-methylsulphanyl-pyrimidn-4-yloxy]-ethyl ester,

pyridin-2-ylcarbamic acid 2-[6-(1,3-benzodioxol-5-ylsulphonylamino)-5-(2-methoxy-phenoxy)-2-(4-methoxy-phenyl)-pyrimidin-4-yloxy]-ethyl ester,

pyridin-2-ylcarbamic acid 2-[6-(1,3-benzodioxol-5-ylsulphonylamino)-5-(2-methoxy-phenoxy)-2-phenyl-pyrimidin-4-yloxy]-ethyl ester,

pyridin-2-ylcarbamic acid 2-[6-(1,3-benzodioxol-5-ylsulphonylamino)-5-(3,5-dimethoxy-phenoxy)-pyrimidin-4-yloxy]-ethyl ester,

pyridin-2-ylcarbamic acid 2-[6-(4-tert-butylphenylsulphonylamino)-5-(2-chloro-5-methoxy-phenoxy)-pyrimidin-4-yloxy]ethyl ester,

1-oxy-pyridin-2-ylcarbamic acid 2-[6-(4-tert-butyl-phenylsulphonylamino)-5-(2-chloro-5-methoxy-phenoxy)-pyrimidin-4-yoxy]-ethyl ester,

pyridin-2-ylcarbamic acid 2-[6-(4-tert-butyl-phenylsulphonylamino)-5-(3-methoxy-phenoxy)-pyrimidin-4-yloxy]-ethyl ester,

pyridin-2-ylcarbamic acid 2-[6-(4-tert-butyl-phenylsulphonylamino)-2-isopropyl-5-(2-methoxy-phenoxy)-pyrimidin-4-yloxy]-ethyl ester,

pyridin-2-ylcarbamic acid 2-[6-(4-tert-butyl-phenylsulphonylamino)-5-(2-methoxy-phenoxy)-2-propyl-pyrimidin-4-yloxy]-ethyl ester,

pyridin-2-ylcarbamic acid 2-[2-tert-butyl-6-(4-tert-butyl-phenylsulphonylamino)-5-(2-methoxy-phenoxy)-pyrimidin-4-yloxy]-ethyl ester,

pyridin-2-ylcarbamic acid 2-[6-(4-tert-butyl-phenylsulphonylamino)-2-cyclopropyl-5-(2-methoxy-phenoxy)-pyrimidin-4-yloxy]-ethyl ester,

pyridin-2-ylcarbamic acid 2-[6-(4-tert-butyl-phenylsulphonylamino)-5-(2-methoxy-phenoxy)-2-thiophen-2-yl-pyrimidin-4-yloxy]-ethyl ester,

pyridin-2-ylcarbamic acid 2-[6-(4-tert-butyl-phenylsulphonylamino)-5-(2-methoxy-phenoxy)-2-methyl-pyrimidin-4-yloxy]-ethyl ester,

pyridin-2-ylcarbamic acid 2-[6-(4-tert-butyl-phenylsulphonylamino)-5-(2-methoxy-phenoxy)-2,2'-bipyrimidin-4-yloxy]-ethyl ester,

pyridin-2-ylcarbamic acid 2-[6-(2-tert-butyl-phenylsulphonylamino)-5-(2-methoxyphenoxy)-2-morpholin-4-yl-pyrimidin-4-yloxy]-ethyl ester,

pyridin-2-yl-carbamic acid 2-[6-(4-cyclopropyl-phenylsulphonylamino)-5-(2-methoxy-phenoxy)-2,2'-bipyrimidin-4-yloxy]-ethyl ester,

pyridin-2-ylcarbamic acid 2-[5-(2-methoxy-phenoxy)-6-(4-methylsulphanyl-phenylsulphonylamino)-2,2'-bipyrimidin-4-yloxy]-ethyl ester,

pyridin-2-ylcarbamic acid 2-[5-(2-methoxy-phenoxy)-6-(4-vinylphenylsulphonylamino)-2,2'-bipyrimidin-4-yloxy]-ethyl ester,

pyridyl-2-ylcarbamic acid 2-[5-(2-bromo-5-methoxy-phenoxy)-6-(4-tert-butyl-phenylsulphonylamino)-pyrimidin-4-yloxy]-ethyl ester,

pyridin-2-ylcarbamic acid 2-[6-(4-tert-butyl-phenylsulphonylamino)-5-(3,4-dimethoxy-phenoxy)-pyrimidin-4-yloxy]-ethyl ester,

pyridin-2-ylcarbamic acid 2-[5-(2-chloro-5-methoxy-phenoxy)-6-(3,4-dimethoxy-phenylsulphonylamino)-pyrimidin-4-yloxy]-ethyl ester,

acetic acid 2-[4,5-(2-methoxy-phenoxy)-6-(2-pyridin-2-ylcarbamoyloxyethoxy)-pyrimidin-4-ylsulphamoyl]-phenoxy]-ethyl ester,

pyridin-2-ylcarbamic acid 2-[6-[4-(2-hydroxy-ethoxy)-phenylsulphonylamino]-5-(2-methoxy-phenoxy)-pyrimidin-4-yloxy]-ethyl ester,

pyridin-2-ylcarbamic acid 2-[6-[4-methoxy-3-[2-morpholin-4-yl-2-oxo-ethoxy)-phenylsulphonylamino]-5-(2-methoxy-phenoxy)-pyrimidin-4-yloxy]-ethyl ester,

acetic acid 2-[4-[5-(2-chloro-5-methoxy-phenoxy)-6-(2-pyridin-2-ylcarbamoyloxy-ethoxy)-pyrimidin-4-ylsulphamoyl]-phenoxy]-ethyl ester,

pyridin-2-ylcarbamic acid 2-[5-(2-chloro-5-methoxy-phenoxy)-6-[4-(2-hydroxy-ethoxy)-phenylsulphonylamino]-pyrimidin-4-yloxy]-ethyl ester,

pyridin-2-ylcarbamic acid 2-[5-(2-chloro-5-methoxy-phenoxy)-6-[4-methoxy-3-(2-morpholin-4-yl-2-oxo-ethoxy)-phenylsulphonylamino]-pyrimidin-4-yloxy]-ethyl ester,

pyridin-2-ylcarbamic acid 2-[5-(2-chloro-5-methoxy-phenoxy)-6-[4-(2-morpholin-4-yl-2-oxo-ethoxy]-phenylsulphonylamino]-pyrimidin-4-yloxy]-ethyl ester,

pyridin-2-ylcarbamic acid 2-[5-(2-methoxy-phenoxy)-6-[4-(2-morpholin-4-yl-2-oxo-ethoxy)-phenylsulphonylamino]-pyrimidin-4-yloxy]-ethyl ester,

pyridin-2-ylcarbamic acid 2-[5-(2-methoxy-phenoxy)-6-[4-(3-morpholin-4-yl-3-oxo-propyl)-phenylsulphonylamino]-pyrimidin-4-yloxy]-ethyl ester,
pyridin-2-ylcarbamic acid 2-[5-(2-chloro-5-methoxy-phenoxy)-6-[4-(3-morpholin-4-yl-3-oxo-propyl)-phenylsulphonylamino]-pyrimidin-4-yloxy]-ethyl ester,
pyridin-2-ylcarbamic acid 2-[6-[4-methoxy-3-(3-morpholin-4-yl-3-oxo-propyl)-phenylsulphonylamino]-5-(2-methoxy-phenoxy)-pyrimidin-4-yloxy]-ethyl ester,
pyridin-2-ylcarbamic acid 2-[5-(2-chloro-5-methoxy-phenoxy)-6-[4-methoxy-3-(3-morpholin-4-yl-3-oxo-propyl)-phenylsulphonylamino]-pyrimidin-4-yloxy]-ethyl ester,
pyridin-2-ylcarbamic acid 2-[5-(4-methoxy-phenoxy)-6-[4-methoxy-3-(3-piperidin-1-yl-3-oxo-propyl)-phenylsulphonylamino]-pyrimidin-4-yloxy]-ethyl ester,
pyridin-2-ylcarbamic acid 2-[5-(2-methoxy-phenoxy)-6-[4-methoxy-3-(3-piperidin-1-yl-3-oxo-propyl)-phenylsulphonylamino]-2,2'-bipyrimidin-4-yloxy]-ethyl ester,
pyridin-2-ylcarbamic acid 2-[6-[4-methoxy-2-(3-morpholin-4-yl-3-oxo-propyl)-phenylsulphonylamino]-5-(2-methoxy-phenoxy)-pyrimidin-4-yloxy]-ethyl ester,
pyridin-2-ylcarbamic acid 2-[6-[4-(2-bromo-ethoxy)-phenylsulphonylamino]-5-(2-methoxy-phenoxy)-pyrimidin-4-yloxy]-ethyl ester,
acetic acid 3-[4-[5-(2-methoxy-phenoxy)-6-(2-pyridin-2-ylcarbamoyloxyethoxy)-pyrimidin-4-ylsulphamoyl]-phenoxy]-propyl ester,
pyridin-2-ylcarbamic acid 2-[6-[4-(3-hydroxy-propoxy)-phenylsulphonylamino]-5-(2-methoxy-phenoxy)-pyrimidin-4-yloxy]-ethyl ester,
pyridin-2-ylcarbamic acid 2-[6-[4-methoxy-3-(2-morpholin-4-yl-2-oxo-ethyl)-phenylsulphonylamino]-5-(2-methoxy-phenoxy)-pyrimidin-4-yloxy]-ethyl ester,
pyridin-2-ylcarbamic acid 2-[6-(1,3-benzodioxol-5-ylsulphonylamino)-2-(2-benzyloxy-ethyl]-5-(2-chloro-5-methoxy-phenoxy)-pyrimidin-4-yloxy]-ethyl ester,
pyridin-2-ylcarbamic acid 2-[5-(2-chloro-5-methoxy-phenoxy)-6-(3-isopropyl-4-methoxy-phenylsulphonylamino)-pyrimidin-4-yloxy]-ethyl ester,
pyridin-2-ylcarbamic acid 2-[6-(1,3-benzodioxol-5-ylsulphonylamino)-5-(2-chloro-5-methoxy-phenoxy)-2-(3-methoxy-propyl)-pyrimidin-4-yloxy]-ethyl ester,
pyridin-2-ylcarbamic acid 2-[6-(4-dimethylaminophenylsulphonylamino)-5-(2-methoxy-phenoxy)-2-morpholin-4-yl-pyrimidin-4-yloxy]ethyl ester.

**11.** The compounds according to claim 9,

pyridin-3-ylcarbamic acid 2-[5-(2-chloro-5-methoxy-phenoxy]-6-(2,3-dihydro-1,4-benzodioxin-6-ylsulphonylamino)-pyrimidin-4-yloxy]-ethyl ester,
pyridin-4-ylcarbamic acid 2-[6-(1,3-benzodioxol-5-ylsulphonylamino)-5-(2-chloro-5-methoxy-phenoxy)-pyrimidin-4-yloxy]-ethyl ester,
pyridin-3-ylcarbamic acid 2-[6-(1,3-benzodioxol-5-ylsulphonylamino)-5-(2-chloro-5-methoxy-phenoxy)-pyrimidin-4-yloxy)-ethyl ester,
pyridin-3-ylcarbamic acid 2-[6-(4-tert-butyl-phenylsulphonylamino)-5-(2-chloro-5-methoxy-phenoxy)-2-methoxymethyl-pyrimidin-4-yloxy]-ethyl ester,
pyridin-3-ylcarbamic acid 2-[6-(4-tert-butylphenylsulphonylamino)-5-(2-chloro-5-methoxy-phenoxy)-pyrimidin-4-yloxy]ethyl ester,
pyridin-4-ylcarbamic acid 2-[6-(4-tert-butylphenylsulphonylamino)-5-(2-chloro-5-methoxy-phenoxy)-pyrimidin-4-yloxy]ethyl ester,
1-oxy-pyridin-4-ylcarbamic acid 2-[6-(4-tert-butyl-phenylsulphonylamino)-5-(2-chloro-5-methoxyphenoxy)-pyrimidin-4-yloxy]-ethyl ester,
3-[2-[5-(2-chloro-5-methoxy-phenoxy)-6-(2,3-dihydro-1,4-benzodioxin-6-ylsulphonylamino)-pyrimidin-4-yloxy]-ethoxycarbonylamino]-1-methylpyridinium iodide,
N-[5-(2-chloro-5-methoxy-phenoxy)-6-[2-(1-methyl-pyridin-3-yl-carbamoyloxy)-ethoxy]-pyrimidin-4-yl]-1,3-benzodioxol-5-sulphonamide,
pyridin-4-ylcarbamic acid 2-[6-(4-tert-butyl-phenylsulphonylamino)-5-(3-methoxy-phenoxy)-pyrimidin-4-yloxy]-ethyl ester,
4-[2-[6-(4-tert-butyl-phenylsulphonylamino)-5-(2-chloro-5-methoxy-phenoxy-pyrimidin-4-yloxy]-ethoxycarbonylamino]-1-methyl-pyridinium iodide,
pyridin-4-ylcarbamic acid 2-[6-(4-tert-butyl-phenylsulphonylamino)-2-isopropyl-5-(2-methoxy-phenoxy)-pyrimidin-4-yloxy]-ethyl ester,
pyridin-4-ylcarbamic acid 2-[6-(4-tert-butyl-phenylsulphonylamino)-5-(2-methoxy-phenoxy)-2-propyl-pyrimidin-4-yloxy]-ethyl ester,

pyridin-4-ylcarbamic acid 2-[2-tert-butyl-6-(4-tert-butylphenylsulphonylamino)-5-(2-methoxy-phenoxy)-pyrimidin-4-yloxy]-ethyl ester,

pyridin-4-ylcarbamic acid 2-[6-(4-tert-butyl-phenylsulphonylamino)-2-cyclopropyl-5-(2-methoxy-phenoxy)-pyrimidin-4-yloxy]-ethyl ester,

pyrimidin-4-ylcarbamic acid 2-[6-(4-tert-butyl-phenylsulphonylamino)-5-(2-methoy-phenoxy)-2-thiophen-2-yl-pyrimidin-4-yloxy]-ethyl ester,

pyridin-4-ylcarbamic acid 2-[6-(4-tert-butyl-phenylsulphonylamino)-5-(2-methoxy-phenoxy)-2-methyl-pyrimidin-4-yloxy]-ethyl ester,

pyridin-4-ylcarbamic acid 2-[6-(4-tert-butyl-phenylsulphonylamino)-5-(2-methoxy-phenoxy)-2,2'-bipyrimidin-4-yloxy]-ethyl ester,

pyridin-4-ylcarbamic acid 2-[6-(2-tert-butyl-phenylsulphonylamino)-5-(2-methoxy-phenoxy)-2-morpholin-4-yl-pyrimidin-4-yloxy]-ethyl ester,

pyridin-3-ylcarbamic acid 2-[6-(2-tert-butyl-phenylsulphonylamino)-5-(2-methoxy-phenoxy)-2-morpholin-4-yl-pyrimidin-4-yloxy]-ethyl ester,

pyridin-4-ylcarbamic acid 2-[6-(4-tert-butyl-phenylsulphonylamino)-5-(3,4-dimethoxy-phenoxy)-pyrimidin-4-yloxy]-ethyl ester,

acetic acid 2-[4-5-(2-methoxy-phenoxy)-6-(2-pyridin-4-ylcarbamoyloxyethoxy)-pyrimidin-4-ylsulphamoyl]-phenoxy]-ethyl ester,

acetic acid 2-[4-[5-(2-methoxy-phenoxy)-6-(2-pyridin-4-ylcarbamoyloxyethoxy)-pyrimidin-4-ylsulphamoyl]-phenoxy]-ethyl ester,

pyridin-4-ylcarbamic acid 2-[6-[4-(2-hydroxy-ethoxy)-phenylsulphonylamino]-5-(2-methoxy-phenoxy)-pyrimidin-4-yloxy]-ethyl ester,

pyridin-3-ylcarbamic acid 2-[6-[4-(2-hydroxy-ethoxy)-phenylsulphonylamino]-5-(2-methoxy-phenoxy)-pyrimidin-4-yloxy]-ethyl ester,

pyridin-4-ylcarbamic acid 2-[6-[4-methoxy-3-[2-morpholin-4-yl-2-oxo-ethoxy)-phenylsulphonylamino]-5-(2-methoxy-phenoxy)-pyrimidin-4-yloxy]-ethyl ester,

pyridin-4-ylcarbamic acid 2-[5-(2-chloro-5-methoxy-phenoxy)-6-[4-methoxy-3-(2-morpholin-4-yl-2-oxo-ethoxy)-phenylsulphonylamino]-pyrimidin-4-yloxy]-ethyl ester,

pyridin-4-ylcarbamic acid 2-[5-(2-methoxy-phenoxy)-6-[4-(2-morpholin-4-yl-2-oxo-ethoxy)-phenylsulphonylamino]-pyrimidin-4-yloxy]-ethyl ester.

**12.** The compounds according to claim 8,

1-methyl-pyrrol-2-ylcarbamic acid 2-[6-(1,3-benzodioxol-5-yl-sulphonylamino)-5-(2-chloro-5-methoxy-phenoxy)-pyrimidin-4-yloxy]-ethyl ester,

thiophen-3-ylcarbamic acid 2-[6-(4-tert-butyl-phenylsulphonylamino)-5-(2-chloro-5-methoxy-phenoxy)-pyrimidin-4-yloxy]-ethyl ester,

thiophen-3-ylcarbamic acid 2-[6-(1,3-benzodioxol-5-ylsulphonylamino)-5-(2-chloro-5-methoxy-phenoxy)-pyrimidin-4-yloxy]-ethyl ester,

thiophen-3-ylcarbamic acid 2-[6-(1,3-benzodioxol-5-ylsulphonylamino)-5-(2-chlor-5-methoxy-phenoxy)-2-methoxymethyl-pyrimidin-4-yloxy]-ethyl ester,

thiophen-2-ylcarbamic acid 2-[6-(1,3-benzodioxol-5-ylsulphonylamino)-5-(2-chloro-5-methoxy-phenoxy)-pyrimidin-4-yloxy]-ethyl ester,

thiophen-3-ylcarbamic acid 2-[6-(1,3-benzodioxol-5-ylsulphonylamino)-5-(2-chloro-5-methoxy-phenoxy)-2-(2-methoxy-ethyl)-pyrimidin-4-yloxy]-ethyl ester,

pyrazin-2-ylcarbamic acid 2-[6-(4-tert-butylphenylsulphonylamino)-5-(2-chloro-5-methoxy-phenoxy)-pyrimidin-4-yloxy]ethyl ester,

quinolin-2-ylcarbamic acid 2-[6-(4-tert-butyl-phenylsulphonylamino)-5-(3-methoxy-phenoxy)-pyrimidin-4-yloxy]-ethyl ester,

furan-3-ylcarbamic acid 2-[6-(1,3-benzodioxol-5-ylsulphonylamino)-5-(2-chloro-5-methoxy-phenoxy)-pyrimidin-4-yloxy]-ethyl ester,

furan-2-ylcarbamic acid 2-[6-(4-tert-butyl-phenylsulphonylamino)-5-(2-methoxy-phenoxy)-2-morpholin-4-yl-pyrimidin-4-yloxy]-ethyl ester,

3-methyl-isoxazol-5-ylcarbamic acid 2-[6-(4-tert-butyl-phenylsulphonylamino)-5-(2-methoxy-phenoxy)-2-morpholin-4-yl-pyrimidin-4-yloxy]-ethyl ester,

3-methyl-isoxazol-5-ylcarbamic acid 2-[5-(2-methoxy-phenoxy)-6-(4-methylsulphanyl-phenylsulphonylamino)-2,2'-bipyrimidin-4-yloxy-ethyl ester,

pyrazin-2-ylcarbamic acid 2-[6-[4-methoxy-3-[2-morpholin-4-yl-2-oxo-ethoxy)-phenylsulphonylamino]-5-(2-methoxy-phenoxy)-pyrimidin-4-yloxy]-ethyl ester,
pyrazin-2-ylcarbamic acid 2-[5-(2-chloro-5-methoxy-phenoxy)-6-[4-methoxy-3-(2-morpholin-4-yl-2-oxo-ethoxy)-phenylsulphonylamino]-pyrimidin-4-yloxy]-ethyl ester,
pyrazin-2-ylcarbamic acid 2-[5-(2-chloro-5-methoxy-phenoxy)-6-[4-(2-morpholin-4-yl-2-oxo-ethoxy]-phenylsulphonylamino]-pyrimidin-4-yloxy]-ethyl ester,
pyrimidin-2-ylcarbamic acid 2-[5-(2-chloro-5-methoxy-phenoxy)-6-[4-methoxy-3-(3-morpholin-4-yl-3-oxo-propyl)-phenylsulphonylamino]-pyrimidin-4-yloxy]-ethyl ester.

**13.** Compounds according to claim 7, in which $R^{10}$ is aryl or cyclo-lower-alkyl and $R^{11}$ is hydrogen, and "lower" signifies residues with 1-7 C atoms.

**14.** The compounds according to claim 13,

1,3-benzodioxol-5-ylcarbamic acid 2-[6-(4-tert-butyl-phenylsulphonylamino)-5-(2-chloro-5-methoxy-phenoxy)-pyrimidin-4-yloxy]-ethyl ester,
3-fluorophenylcarbamic acid 2-[6-(4-tert-butylphenylsulphonylamino)-5-(2-chloro-5-methoxy-phenoxy)-pyrimidin-4-yloxy]-ethyl ester,
2-fluorophenylcarbamic acid 2-[(6-(4-tert-butyl-phenylsulphonylamino)-5-(2-chloro-5-methoxy-phenoxy)-pyrimidin-4-yloxy]-ethyl ester,
phenylcarbamic acid 2-[6-(4-tert-butyl-phenylsulphonylamino)-5-(2-chloro-5-methoxy-phenoxy)pyrimidin-4-yloxy]-ethyl ester,
4-chloro-phenylcarbamic acid 2-[6-(4-tert-butyl-phenylsulphanoylamino)-5-(2-chloro-5-methoxy-phenoxy)-pyrimidin-4-yloxy]-ethyl ester,
3-methoxy-phenylcarbamic acid 2-[6-(4-tert-butyl-phenylsulphonylamino)-5-(2-chloro-5-methoxy-phenoxy)-pyrimidin-4-yloxy]-ethyl ester,
4-trifluoromethyl-phenylcarbamic acid 2-[6-(4-tert-butyl-phenylsulphonylamino)-5-(2-chloro-5-methoxy-phenoxy)-pyrimidin-4-yloxy]-ethyl ester,
2-[2-[6-(4-tert-butyl-phenylsulphonylamino)-5-(2-chloro-5-methoxy)-pyrimidin-4-yloxy]-ethoxycarbonylamino]-benzoic acid methyl ester,
3-tolylcarbamic acid 2-(6-(4-tert-butyl-phenylsulphonylamino)-5-(2-chloro-5-methoxy-phenoxy)-pyrimidin-4-yloxy]-ethyl ester,
2-methoxy-phenoxycarbamic acid 2-[6-(4-tert-butylphenylsulphonylamino)-5-(2-chloro-5-methoxy-phenoxy)-pyrimidin-4-yloxy]-ethyl ester,
acetic acid 2-[2-[(6-tert-butyl-phenylsulphonylamino)-5-(2-chloro-5-methoxy)-pyrimidin-4-yloxy]-ethoxycarbonylamino]-phenyl ester,
2-hydroxy-phenylcarbamic acid 2-[6-(4-tert-butylphenylsulphonylamino)-5-(2-chloro-5-methoxy-phenoxy)-pyrimidin-4-yloxy]-ethyl ester,
benzylcarbamic acid 2-[6-(4-tert-butylphenylsulphonylamino)-5-(2-chloro-5-methoxy-phenoxy)pyrimidin-4-yloxy]-ethyl ester,
phenylcarbamic acid 2-[6-(4-tert-butyl-phenylsulphonylamino)-5-(3-methoxy-phenoxy)-pyrimidin-4-yloxy]-ethyl ester,
(R)-1-phenyl-ethylcarbamic acid 2-[6-(4-tert-butyl-phenylsulphonylamino)-5-(3-methoxy-phenoxy)-pyrimidin-4-yloxy]-ethyl ester,
cyclohexylcarbamic acid 2-[6-(4-tert-butyl-phenylsulphonylamino)-5-(3-methoxy-phenoxy)-pyrimidin-4-yloxy]-ethyl ester,
acetic acid 2-[4-[5-(2-methoxy-phenoxy)-6-(2-phenylcarbamoyloxyethoxy)-pyrimidin-4-ylsulphamoyl]-phenoxy]-ethyl ester,
acetic acid 2-[4-[6-[2-(2-fluoro-phenylcarbamoyloxy)-ethoxy]-5-(2-methoxy-phenoxy)-pyrimidin-4-ylsulphamoyl]-phenoxy]-ethyl ester,
phenylcarbamic acid 2-[6-[4-(2-hydroxy-ethoxy)-phenylsulphonylamino]-5-(2-methoxy-phenoxy)-pyrimidin-4-yloxy]-ethyl ester,
2-fluoro-phenylcarbamic acid 2-[6-[4-(2-hydroxy-ethoxy)-phenylsulphonylamino]-5-(2-methoxy-phenoxy)-pyrimidin-4-yloxy]-ethyl ester,
2-fluoro-phenylcarbamic acid 2-[6-[4-methoxy-3-(2-morpholin-4-yl-2-oxo-ethoxy)-phenylsulphonylamino]-5-(2-methoxy-phenoxy)-pyrimidin-4-yloxy]-ethyl ester,
acetic acid 2-[4-[5-(2-chloro-5-methoxy-phenoxy)-6-[2-(2-fluorophenylcarbamoyloxy)-ethoxy]-pyrimidin-4-ylsulphamoyl]-phenoxy]-ethyl ester,

2-fluoro-phenylcarbamic acid 2-[5-(2-chloro-5-methoxy-phenoxy)-6-[4-(2-hydroxy-ethoxy)-phenylsulphonylamino]-pyrimidin-4-yloxy]-ethyl ester,
2-fluoro-phenylcarbamic acid 2-[5-(2-chloro-5-methoxy-phenoxy)-6-[4-methoxy-3-(2-morpholin-4-yl-2-oxo-ethoxy)-phenylsulphonylamino]-pyrimidin-4-yloxy]-ethyl ester,
phenylcarbamic acid 2-[6-[4-methoxy-3-(2-morpholin-4-yl-2-oxo-ethoxy)-phenylsulphonylamino]-5-(3-methoxy-phenoxy)-pyrimidin-4-yloxy]-ethyl ester,
[5-[5-(2-chloro-5-methoxy-phenoxy)-6-[2-(2-fluorophenylcarbamoyloxy)-ethoxy]-pyrimidin-4-ylsulphamoyl]-2-methoxy-phenoxy]-acetic acid ethyl ester,
5-[5-(2-chloro-5-methoxy-phenoxy)-6-[2-(2-fluoro-phenylcarbamoyloxy)-ethoxy]-pyrimidin-4-ylsulphamoyl]-2-methoxy-phenoxy]-acetic acid,
2-fluoro-phenylcarbamic acid 2-[6-[4-methoxy-3-(2-oxo-2-piperidin-1-yl-ethoxy)-phenylsulphonylamino]-5-(2-methoxy-phenoxy)-pyridin-4-yloxy]-ethyl ester.

**15.** The compounds according to claim 7,

isopropylcarbamic acid 2-[6-(4-tert-butyl-phenylsulphonylamino)-5-(2-chloro-5-methoxy-phenoxy)-pyrimidin-4-yloxy]-ethyl ester,
ethylcarbamic acid 2-[6-(4-tert-butyl-phenylsulphonylamino)-5-(2-chloro-5-methoxy-phenoxy)-pyrimidin-4-yloxy]-ethyl ester,
[2-[6-(4-tert-butyl-phenylsulphonylamino)-5-(3-methoxy-phenoxy)-pyrimidin-4-yloxy]-ethoxycarbonylamino]-acetic acid ethyl ester,
2-[6-(4-tert-butyl-phenylsulphonylamino)-5-(3-methoxy-phenoxy)-pyrimidin-4-yloxy]-ethoxycarbonylaminoacetic acid,
2-hydroxy-ethylcarbamic acid 2-[6-(4-tert-butyl-phenylsulphonylamino)-5-(3-methoxy-phenoxy)-pyrimidin-4-yloxy]-ethyl ester,
morpholine-4-carboxylic acid 2-[6-(4-tert-butyl-phenylsulphonylamino)-5-(3-methoxy-phenoxy)-pyrimidin-4-yloxy]-ethyl ester,
2-morpholin-4-yl-2-oxo-ethylcarbamic acid 2-[6-(4-tert-butyl-phenylsulphonylamino)-5-(3-methoxy-phenoxy)-pyrimidin-4-yloxy]-ethyl ester,
2-oxo-2-pyrrolidin-1-yl-ethylcarbamic acid 2-[6-(4-tert-butyl-phenylsulphonylamino)-5-(3-methoxy-phenoxy)-pyrimidin-4-yloxy]-ethyl ester,
phenylcarbamoylmethylcarbamic acid 2-[6-(4-tert-butyl-phenylsulphonylamino)-5-(3-methoxy-phenoxy)-pyrimidin-4-yloxy]ethyl ester,
2-[2-[6-(4-tert-butyl-phenylsulphonylamino)-5-(3-methoxy-phenoxy)-pyrimidin-4-yloxy]-ethoxycarbonylamino]-4-methyl-pentanoic acid ethyl ester.

**16.** Compounds according to claim 1-6 in which Y is $-NHC(O)NR^{10}R^{11}$.

**17.** The compounds according to claim 16,

4-tert-butyl-N-[5-(2-chloro-5-methoxy-phenoxy)-6-[2-(3-phenyl-ureido)-ethoxy]-pyrimidin-4-yl]-benzenesulphonamide,
4-tert-butyl-N-[5-(2-chloro-5-methoxy-phenoxy)-6-[2-[3-(2-fluorophenyl)-ureido]-ethoxy]-pyrimidin-4-yl]-benzenesulphonamide,
4-tert-butyl-N-[5-(2-chloro-5-methoxy-phenoxy)-6-[2-(3-pyridin-2-yl-ureido)-ethoxy]-pyrimidin-4-yl]-benzenesulphonamide
4-tert-butyl-N-[5-(2-chloro-5-methoxy-phenoxy)-6-[2-(3-pyridin-4-yl-ureido)-ethoxy]-pyrimidin-4-yl]-benzenesulphonamide,
4-tert-butyl-N-[5-(2-chloro-5-methoxy-phenoxy)-6-[2-(3-pyridin-3-yl-ureido)-ethoxy]-pyrimidin-4-yl]-benzenesulphonamide,
4-tert-butyl-N-[5-(2-chloro-5-methoxy-phenoxy)-6-[2-(3-1-oxy-pyridin-4-yl)-ureido]-ethoxy]-pyrimidin-4-yl]-benzenesulphonamide,
4-tert-butyl-N-[5-(2-methoxy-phenoxy)-2-methyl-6-[2-(3-pyridin-2-yl-ureido)-ethoxy]-pyrimidin-4-yl]-benzenesulphonamide.

**18.** Compounds according to claims 1-6, in which Y is $-OC(O)COR^{10}$.

**19.** The compounds according to claim 18,

carboxylic acid 2-[6-(1,3-benzodioxol-5-ylsulphonylamino)-5-(3,5-dimethoxy-phenoxy)-pyrimidin-4-yloxy]-ethyl ester pyridin-3-ylmethyl ester,
carboxylic acid 2-[6-(1,3-benzodioxol-5-sulphonylamino)-5-(2-chloro-5-methoxy-phenoxy)-pyrimidin-4-yloxy]-ethyl ester pyridin-2-ylmethyl ester,
carboxylic acid 2-[6-(1,3-benzodioxol-5-ylsulphonylamino)-5-(2-chloro-5-methoxy-phenoxy)-pyrimidin-4-yloxy]-ethyl ester pyridin-2-ylmethyl ester,
carboxylic acid 2-[6-(4-tert-butyl-benzenesulphonylamino)-5-(2-chloro-5-methoxy-phenoxy)-pyrimidin-4-yloxy]-ethyl ester pyridin-2-ylmethyl ester,
carboxylic acid 2-[6-(1,3-benzodioxol-5-ylsulphonylamino)-5-(2-chloro-5-methoxy-phenoxy)-pyrimidin-4-yloxy]-ethyl ester furan-3-ylmethyl ester,
carboxylic acid 2-[6-(4-tert-butyl-phenylsulphonylamino)-5-(2-chloro-5-methoxy-phenoxy)-2-methyl-pyrimidin-4-yloxy]-ethyl ester pyridin-3-ylmethyl ester,
carboxylic acid 2-[6-(4-tert-butyl-phenylsulphonylamino)-5-(2-chloro-5-methoxy-phenoxy)-2-methyl-pyrimidin-4-yloxy]-ethyl ester pyridin-2-ylmethyl ester,
carboxylic acid 2-[6-(4-tert-butyl-phenylsulphonylamino)-5-(2-chloro-5-methoxy-phenoxy)-2-methyl-pyrimidin-4-yloxy]-ethyl ester pyrimidin-4-ylmethyl ester,
carboxylic acid 2-[6-(4-tert-butyl-phenylsulphonylamino)-5-(2-methoxyphenylsulphanyl)-2,2'-bipyrimidin-4-yloxy]-ethyl ester-pyridin-3-ylmethyl ester,
carboxylic acid 2-[6-(4-tert-butyl-phenylsulphonylamino)-5-(2-methoxyphenylsulphanyl)-2-methyl-pyrimidin-4-yloxy]-ethyl ester pyridin-3-ylmethyl ester.

**20.** Compounds according to claim 1, in which n = 1 and Z = -S-.

**21.** The compounds according to claim 20,

pyridin-2-ylcarbamic acid 2-[6-(4-tert-butyl-phenylsulphonylamino)-5-(2-methoxy-phenylsulphanyl)-pyrimidin-4-yloxy)-ethyl ester,
pyridin-3-ylcarbamic acid 2-[6-tert-butyl-phenylsulphonylamino)-5-(2-methoxy-phenylsulphanyl)-2,2'-bipyrimidin-4-yloxy]-ethyl ester,
pyridin-4-ylcarbamic acid 2-[6-(4-tert-butyl-phenylsulphonylamino)-5-(2-methoxy-phenylsulphanyl)-2-methyl-pyrimidin-4-yloxy]-ethyl ester.

**22.** The compounds of claims 1-21 for use as medicaments.

**23.** Pharmaceutical preparations, containing a compound of claims 1-21 and conventional carriers and adjuvants.

**24.** The use of compounds of claims 1-21 as active ingredients in the production of medicaments for the treatment of disorders which are associated with endothelin activities, especially circulatory disorders such as hypertension, ischaemia, vasospasms and angina pectoris.

**25.** A process for the manufacture of compounds of claims 1-21, characterized by reacting a compound of the formula

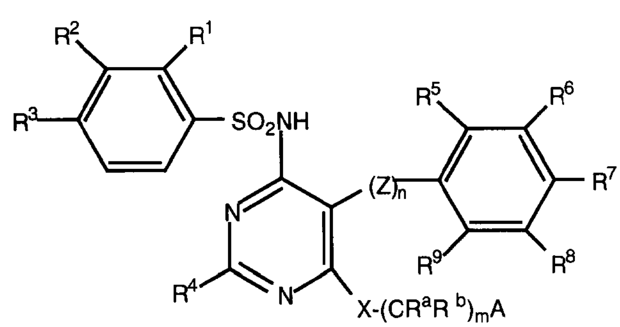

II

wherein $R^1$-$R^9$, $R^a$, $R^b$, X, Z, m and n have the significance given in claim 1 and A is hydroxy or amino,

a) with an isocyanate of the formula $R^{10}NCO$ or a carbamoyl chloride of the formula $(R^{10}R^{11})NCOCl$, wherein

$R^{10}$ and $R^{11}$ have the significance given in claim 1, or

b) with phosgene and thereafter with an alcohol of the formula $R^{10}OH$; or with a chloroformate of the formula $R^{10}OC(O)Cl$,

and, if desired, modifying substituents present in the thus-obtained compound of formula I and, if desired, converting a compound of formula I into a pharmaceutically usable salt.

**Revendications**

1. Composés de formule

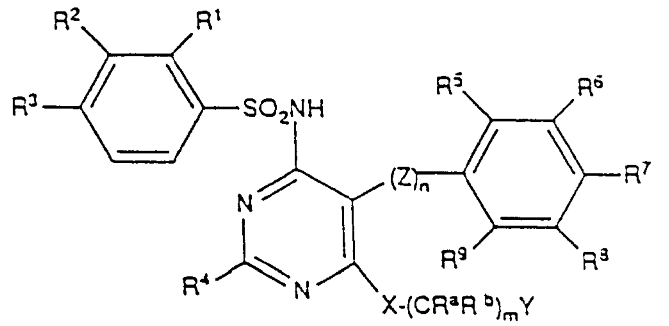

I

dans laquelle

$R^1$-$R^3$ sont chacun indépendamment des autres un atome d'hydrogène, un groupe alkyle inférieur, alcoxy inférieur, alkylthio inférieur, alcényle inférieur, halogéno, trifluorométhyle, hydroxyalcoxy inférieur, halogènalcoxy inférieur, cycloalkyle inférieur, hydroxyalkyle inférieur, aminoalkyle inférieur, aminoalcoxy inférieur, alcanoylamino(alcoxy inférieur), alcanoylamino(alkyle inférieur), carboxy(alcoxy inférieur), carboxy(alkyle inférieur), (alcoxy inférieur)carbonyl(alkyle inférieur), (alcoxy inférieur)carbonyl(alcoxy inférieur), alcanoyloxy(alcoxy inférieur), alcanoyloxy(alkyle inférieur), alcoxycarbonyle, carboxy, amino, mono- ou di-(alkyle inférieur)-amino, ou un radical $(R^c,R^d)N-C(O)(CH_2)_{0-4}O-$ ou $(R^c,R^d)N-C(O)(CH_2)_{0-4}-$ ;
$R^2$ et $R^3$ forment ensemble un radical butadiényle, méthylènedioxy, éthylènedioxy ou isopropylidènedioxy ;
$R^4$ est un atome d'hydrogène ou un groupe alkyle inférieur, cycloalkyle inférieur, trifluorométhyle, alcoxy inférieur, alcynyloxy inférieur, alkylthio inférieur, (alkyle inférieur)thio(alkyle inférieur), (alkyle inférieur)thio(alcoxy inférieur), hydroxyalkyle inférieur, hydroxyalcoxy inférieur, dihydroxyalcoxy inférieur, (alcoxy inférieur)(alkyle inférieur), hydroxy(alcoxy inférieur)(alkyle inférieur), (alcoxy inférieur)(alcoxy inférieur), di-(alcoxy inférieur)-alcoxy, hydroxy(alcoxy inférieur)(alcoxy inférieur), alkylsulfinyle inférieur, (alkyle inférieur)sulfinyl(alcoxy inférieur), alkylsulfonyle inférieur, 2-méthoxy-3-hydroxypropoxy, 2-hydroxy-3-phénylpropyle, amino(alkyle inférieur), (alkyle inférieur)amino(alkyle inférieur), di(alkyle inférieur)-amino(alkyle inférieur), amino, alkylamino inférieur, di-(alkyle inférieur)amino, arylamino, aryle, arylthio, aryloxy, aryl(alkyle inférieur), aryl(alcoxy inférieur)-(alkyle inférieur), aryl(alkyle inférieur)(alcoxy inférieur), hétérocyclyle, hétérocyclyl(alkyle inférieur) ou hétérocyclylalcoxy ;
$R^5$ à $R^9$ représentent chacun un atome d'hydrogène, un groupe halogéno, trifluorométhyle, alkyle inférieur, alcoxy inférieur, alkylthio inférieur, alkylsulfinyle inférieur ou alkylsulfonyle inférieur ;
$R^6$, avec $R^5$ ou $R^7$, forment le radical butadiényle, méthylènedioxy, éthylènedioxy ou isopropylidènedioxy ;
$R^{10}$ est un groupe alkyle inférieur, cycloalkyle inférieur, hydroxyalkyle inférieur, carboxy(alkyle inférieur), (alcoxy inférieur)carbonyl(alkyle inférieur), (alcanoyloxy inférieur)(alkyle inférieur), aryle, aryl(alkyle inférieur), arylcarbamoyl(alkyle inférieur), hétérocyclyle, hétérocyclyl(alkyle inférieur), ou un radical $(R^c,R^d)N-C(O)(CH_2)_{1-4}$ ;
$R^{11}$ est un atome dh'ydrogène ou un radical $R^{10}$ ;
$R^{10}$ et $R^{11}$, avec l'atome d'azote auquel ils sont liés, forment un radical hétérocyclique à 5-7 chaînons ;
$R^a$ et $R^b$ représentent chacun indépendamment de l'autre un atome d'hydrogène, un groupe alkyle inférieur, alcoxy inférieur ou alkylthio inférieur ;
$R^c$ et $R^d$ représentent chacun indépendamment de l'autre un atome d'hydrogène, un groupe alkyle inférieur ou aryle ; ou bien $R^c$ et $R^d$, avec l'atome d'azote auquel ils sont liés, forment un radical hétérocyclique à 5-7 chaînons ;
Y est un radical $-OC(O)NR^{10}R^{11}$, $-NHC(O)NR^{10}R^{11}$, $-OC(O)OR^{10}$ ou $-NHC(O)OR^{10}$ ;
Z est -O-, -S- ou $-CH_2-$ ;
X est -O-, -S- ou -NH- ;
n vaut 0 ou 1 ;
m vaut 1, 2 ou 3, et

"inférieur" désigne des radicaux ayant de 1 à 7 atomes de carbone, et leurs sels acceptables d'un point de vue pharmaceutique.

2. Composés selon la revendication 1, dans lesquels n = 1 et Z = -O-.

3. Composés selon la revendication 2, dans lesquels X = -O-.

4. Composés selon les revendications 1 à 3, dans lesquels $R^5$ est un halogène, $R^8$ est un groupe alcoxy inférieur et $R^6$, $R^7$ et $R^9$ sont des atomes d'hydrogène, et "inférieur" désigne des radicaux ayant de 1 à 7 atomes de carbone.

5. Composés selon les revendications 1 à 4, dans lesquels $R^3$ est un groupe alkyle inférieur, et $R^1$ et $R^2$ sont des atomes d'hydrogène, et "inférieur" désigne des radicaux ayant de 1 à 7 atomes de carbone.

6. Composés selon les revendications 1 à 5, dans lesquels $R^4$ est un atome d'hydrogène, un groupe alkyle inférieur, (alcoxy inférieur)(alkyle inférieur), alkylthio inférieur, phényle, (alcoxy inférieur)phényle, phényl(alcoxy inférieur)(alkyle inférieur), cycloalkyle inférieur, pyrimidinyle, morpholino ou thiényle, et "inférieur" désigne des radicaux ayant de 1 à 7 atomes de carbone.

7. Composés selon les revendications 1 à 6, dans lesquels Y est $-OC(O)NR^{10}R^{11}$.

8. Composés selon la revendication 7, dans lesquels $R^{10}$ est un radical hétérocyclyle et $R^{11}$ est un atome d'hydrogène.

9. Composés selon la revendication 8, dans lesquels le radical hétérocyclyle $R^{10}$ est un radical pyridyle.

10. Les composés selon la revendication 9 :

Ester 2-[6-(1,3-benzodioxole-5-ylsulfonylamino)-5-(2-chloro-5-méthoxy-phénoxy)-pyrimidine-4-yloxy]-éthylique de l'acide pyridine-2-ylcarbamique,
Ester 2-[6-(4-tert-butyl-phénylsulfonylamino)-5-(2-chloro-5-méthoxy-phénoxy)-pyrimidine-4-yloxy]-éthylique de l'acide pyridine-2-ylcarbamique,
Ester 2-[6-(1,3-benzodioxole-5-ylsulfonylamino)-5-(2-chloro-5-méthoxy-phénoxy)-2-méthoxyméthyl-pyrimidine-4-yloxy]-éthylique de l'acide pyridine-2-ylcarbamique,
Ester 2-[6-(1,3-benzodioxole-5-ylsulfonylamino)-5-(2-chloro-5-méthoxy-phénoxy)-2-(2-méthoxyéthyl)-pyrimidine-4-yloxy]-éthylique de l'acide pyridine-2-ylcarbamique,
Ester 2-[6-(1,3-benzodioxole-5-ylsulfonylamino)-5-(2-chloro-5-méthoxy-phénoxy)-2-méthylsulfanyl-pyrimidine-4-yloxy]-éthylique de l'acide pyridine-2-ylcarbamique,
Ester 2-[6-(1,3-benzodioxole-5-ylsulfonylamino)-5-(2-chloro-5-méthoxy-phénoxy)-2-(4-méthoxyphényl)-pyrimidine-4-yloxy]-éthylique de l'acide pyridine-2-ylcarbamique,
Ester 2-[6-(1,3-benzodioxole-5-ylsulfonylamino)-5-(2-chloro-5-méthoxy-phénoxy)-2-phényl-pyrimidine-4-yloxy]-éthylique de l'acide pyridine-2-ylcarbamique,
Ester 2-[6-(1,3-benzodioxole-5-ylsulfonylamino)-5-(3,5-diméthoxy-phénoxy)-pyrimidine-4-yloxy]-éthylique de l'acide pyridine-2-ylcarbamique,
Ester 2-[6-(4-tert-butyl-phénylsulfonylamino)-5-(2-chloro-5-méthoxy-phénoxy)-pyrimidine-4-yloxy]-éthylique de l'acide pyridine-2-ylcarbamique,
Ester 2-[6-(4-tert-butylphénylsulfonylamino)-5-(2-chloro-5-méthoxy-phénoxy)-pyrimidine-4-yloxy]-éthylique de l'acide 1-oxypyridine,
Ester 2-[6-(4-tert-butyl-phénylsulfonylamino)-5-(3-méthoxy-phénoxy)-pyrimidine-4-yloxy]-éthylique de l'acide pyridine-2-ylcarbamique,
Ester 2-[6-(4-tert-butyl-phénylsulfonylamino)-2-isopropyl-5-(2-méthoxy-phénoxy)-pyrimidine-4-yloxy]-éthylique de l'acide pyridine-2-ylcarbamique,

Ester 2-[6-(4-tert-butyl-phénylsulfonylamino)-5-(2-méthoxy-phénoxy)-2-propyl-pyrimidine-4-yloxy]-éthylique de l'acide pyridine-2-ylcarbamique,

Ester 2-[2-tert-butyl-6-(4-tert-butyl-phénylsulfonylamino)-5-(2-méthoxy-phénoxy)-pyrimidine-4-yloxy]-éthylique de l'acide pyridine-2-ylcarbamique,

Ester 2-[6-(4-tert-butyl-phénylsulfonylamino)-2-cyclopropyl-5-(2-méthoxy-phénoxy)-pyrimidine-4-yloxy]-éthylique de l'acide pyridine-2-ylcarbamique,

Ester 2-[6-(4-tert-butyl-phénylsulfonylamino)-5-(2-méthoxy-phénoxy)-2-thiophène-2-yl-pyrimidine-4-yloxy]-éthylique de l'acide pyridine-2-ylcarbamique,

Ester 2-[6-(4-tert-butyl-phénylsulfonylamino)-5-(2-méthoxy-phénoxy)-2-méthyl-pyrimidine-4-yloxy]-éthylique de l'acide pyridine-2-ylcarbamique,

Ester 2-[6-(4-tert-butyl-phénylsulfonylamino)-5-(2-méthoxy-phénoxy)-2,2'-bipyrimidine-4-yloxy]-éthylique de l'acide pyridine-2-ylcarbamique,

Ester 2-[6-(2-tert-butyl-phénylsulfonylamino)-5-(2-méthoxy-phénoxy)-2-morpholine-4-yloxy]-éthylique de l'acide pyridine-2-ylcarbamique,

Ester 2-[6-(4-cyclopropyl-phénylsulfonylamino)-5-(2-méthoxy-phénoxy)-2,2'-pyrimidine-4-yloxy]-éthylique de l'acide pyridine-2-ylcarbamique,

Este 2-[5-(2-méthoxy-phénoxy)-6-(4-méthylsulsulfanylphénylsulfonylamino)-2,2'-bipyrimidine-4-yloxy]-éthylique de l'acide pyridine-2-ylcarbamique,

Ester 2-[5-(2-méthoxy-phénoxy)-6-(4-vinylphénylsulfonylamino)-2,2'bipyrimidine-4-yloxy]-éthylique de l'acide pyridine-2-ylcarbamique,

Ester 2-[5-(2-bromo-5-méthoxy-phénoxy)-6-(4-tert-butyl-phénylsulfonylamino)-pyrimidine-4-yloxy]-éthylique de l'acide pyridine-2-ylcarbamique,

Ester 2-[6-(4-tert-butyl-phénylsulfonylamino)-5-(3,4-diméthoxy-phénoxy)-pyrimidine-4-yloxy]-éthylique de l'acide pyridine-2-ylcarbamique,

Ester 2-[5-(2-chloro-5-méthoxy-phénoxy)-6-(3,4-diméthoxy-phénylsulfonylamino)-pyrimidine-4-yloxy]-éthylique de l'acide pyridine-2-ylcarbamique,

Ester 2-[4-5-(2-méthoxy-phénoxy)-6-(2-pyridine-2-ylcarbamoyloxy-éthoxy)-pyrimidine-4-ylsulfamoyl]-phénoxy éthylique de l'acide acétique,

Ester 2-[6-[4-(2-hydroxy-éthoxy)-phényl-sulfonyl-amino]-5-(2-méthoxy-phénoxy)-pyrimidine-4-yloxy]-éthylique de l'acide pyridine-2-ylcarbamique,

Ester 2-[6-[4-méthoxy-3-[2-morpholin-4-yl-2-oxo-éthoxy)-phénylsulfonylamino]-5-(2-méthoxy-phénoxy)-pyrimidine-4-yloxy]-éthylique de l'acide pyridine-2-ylcarbamique,

Ester 2-[4-[5-(2-chloro-5-méthoxy-phénoxy)-6-(2-pyridine-2-ylcarbamoyloxy-éthoxy)-pyrimidine-4-ylsulfamoyl]-phénoxy]-éthylique de l'acide acétique,

Ester 2-[5-(2-chloro-5-méthoxy-phénoxy)-6-[4-(2-hydroxy-éthoxy)-phénylsulfonylamino]-pyrimidine-4-yloxy]-éthylique de l'acide pyridine-2-ylcarbamique,

Ester 2-[5-(2-chloro-5-méthoxy-phénoxy)-6-[4-méthoxy-3-(2-morpholine-4-yl-2-oxo-éthoxy)-phénylsulfonylamino]-pyrimidine-4-yloxy]-éthylique de l'acide pyridine-2-ylcarbamique,

Ester 2-[5-(2-chloro-5-méthoxy-phénoxy)-6-[4-(2-morpholine-4-yl-2-oxo-éthoxy]-phénylsulfonylamino]-pyrimidine-4-yloxy]-éthylique de l'acide pyridine-2-ylcarbamique,

Ester 2-[5-(2-méthoxy-phénoxy)-6-[4-(2-morpholine-4-yl-2-oxo-éthoxy)-phénylsulfonylamino]-pyrimidine-4-yloxy]-éthylique de l'acide pyridine-2-ylcarbamique,

Ester 2-[5-(2-méthoxy-phénoxy)-6-[4-(3-morpholine-4-yl-3-oxo-propyl)-phénylsulfonylamino]-pyrimidine-4-yloxy]-éthylique de l'acide pyridine-2-ylcarbamique,

Ester 2-[5-(2-chloro-5-méthoxy-phénoxy)-6-[4-(3-morpholine-4-yl-3-oxo-propyl)-phénylsulfonylamino]-pyrimidine-4-yloxy]-éthylique de l'acide pyridine-2-ylcarbamique,

Ester 2-[6-[4-méthoxy-3-(3-morpholino-4-yl-3-oxo-propyl)-phénylsulfonylamino]-5-(2-méthoxy-phénoxy)-pyrimidine-4-yloxy]-éthylique de l'acide pyridine-2-ylcarbamique,

Ester 2-[5-(2-chloro-5-méthoxy-phénoxy)-6-[4-méthoxy-3-(3-morpholino-4-yl-3-oxo-propyl)-phényl-sulfonylamino]-pyrimidine-4-yloxy]-éthylique de l'acide pyridine-2-ylcarbamique,

Ester 2-[5-(4-méthoxy-phénoxy)-6-[4-méthoxy-3-(3-pipéridine-1-yl-3-oxo-propyl)-phénylsulfonylamino]-pyrimidine-4-yloxy]-éthylique de l'acide pyridine-2-ylcarbamique,

Ester 2-[5-(2-méthoxy-phénoxy)-6-[4-méthoxy-3-(3-pipéridine-1-yl-3-oxo-propyl)-phénylsulfonylamino]-2,2'-bipyrimidine-4-yloxy]-éthylique de l'acide pyridine-2-ylcarbamique,

Ester 2-[6-[4-méthoxy-2-(3-morpholine-4-yl-3-oxo-propyl)-phénylsulfonylamino]-5-(2-méthoxy-phénoxy)-pyrimidine-4-yloxy]-éthylique de l'acide pyridine-2-ylcarbamique,

Ester 2-[6-[4-(2-bromo-éthoxy)-phénylsulfonylamino]-5-(2-méthoxy-phénoxy)-pyrimidine-4-yloxy]-éthylique de l'acide pyridine-2-ylcarbamique,

Ester 3-[4-[5-(2-méthoxy-phénoxy)-6-(2-pyridine-2-ylcarbamoyloxy-éthoxy)-pyrimidine-4-ylsulfamoyl]- phénoxy]-propylique de l'acide acétique,
Ester 2-[6-[4-(3-hydroxy-propoxy)-phénylsulfonyl-amino]-5-(2-méthoxy-phénoxy)-pyrimidine-4-yloxy]-éthylique de l'acide pyridine-2-ylcarbamique,
Ester 2-[6-[4-méthoxy-3-(2-morpholine-4-yl-2-oxo-éthyl)-phénylsulfonylamino]-5-(2-méthoxy-phénoxy)-pyrimidine-4-yloxy]-éthylique de l'acide pyridine-2-ylcarbamique,
Ester 2-[6-(1,3-benzodioxol-5-ylsulfonylamino)-2-(2-benzyloxy-éthyl]-5-(2-chloro-5-méthoxy-phénoxy)-pyrimidine-4-yloxy]-éthylique de l'acide pyridine-2-ylcarbamique,
Ester 2-[5-(2-chloro-5-méthoxy-phénoxy)-6-(3-isopropyl-4-méthoxy-phénylsulfonylamino)-pyrimidine-4-yloxy]-éthylique de l'acide pyridine-2-ylcarbamique,
Ester 2-[6-(1,3-benzodioxol-5-ylsulfonylamino)-5-(2-chloro-5-méthoxy-phénoxy)-(2-(3-méthoxy-propyl)-pyrimidine-4-yloxy]-éthylique de l'acide pyridine-2-ylcarbamique,
Ester 2-[6-(4-diméthylaminophénylsulfonylamino)-5-(2-méthoxy-phénoxy)-2-morpholine-4-yl-pyrimidine-4-yloxy]-éthylique de l'acide pyridine-2-ylcarbamique,

**11.** Les composés selon la revendication 9 :

Ester 2-[5-(2-chloro-5-méthoxy-phénoxy)-6-(2,3-dihydro-1,4-benzodioxine-6-ylsulfonylamino)-pyrimidine-4-yloxy]-éthylique de l'acide pyridine-3-ylcarbamique,
Ester 2-[6-(1,3-benzodioxol-5-ylsulfonylamino)-5-(2-chloro-5-méthoxy-phénoxy)-pyrimidine-4-yloxy]-éthylique de l'acide pyridine-4-ylcarbamique,
Ester 2-[6-(1,3-benzodioxol-5-ylsulfonylamino)-5-(2-chloro-5-méthoxy-phénoxy)-pyrimidine-4-yloxy]-éthylique de l'acide pyridine-3-ylcarbamique,
Ester 2-[6-(4-tert-butyl-phénylsulfonylamino)-5-(2-chloro-5-méthoxy-phénoxy)-2-méthoxyméthyl-pyrimidine-4-yloxy]-éthylique de l'acide pyridine-3-ylcarbamique,
Ester 2-[6-(4-tert-butylphénylsulfonylamino)-5-(2-chloro-5-méthoxy-phénoxy)-pyrimidine-4-yloxy]-éthylique de l'acide pyridine-3-ylcarbamique,
Ester 2-[6-(4-tert-butylphénylsulfonylamino)-5-(2-chloro-5-méthoxy-phénoxy)-pyrimidine-4-yloxy]-éthylique de l'acide pyridine-4-ylcarbamique,
Ester 2-[6-(4-tert-butyl-phénylsulfonylamino)-5-(2-chloro-5-méthoxy-phénoxy)-pyrimidine-4-yloxy]-éthylique de l'acide 1-oxy-pyridine-4-ylcarbamique,
Iodure de 3-[2-[5-(2-chloro-5-méthoxy-phénoxy)-6-2,3-dihydro-1,4-benzodioxine-6-ylsulfonylamino)-pyrimidine-4-yloxy]-éthoxycarbonylamino]-1-méthyl-pyridinium,
N-[5-(2-chloro-5-méthoxy-phénoxy)-6-[2-(1-méthyl-pyridine-3-yliocarbamoyloxy)-éthoxy]-pyrimidine-4-yl]-1,3-benzodioxole-5-sulfonamide,
Ester 2-[6-(4-tert-butyl-phénylsulfonylamino)-5-(3-méthoxy-phénoxy)-pyrimidine-4-yloxy]-éthylique de l'acide pyridine-4-ylcarbamique,
Iodure de 4-[2-[6-(4-tert-butyl-phénylsulfonylamino)-5-(2-chloro-5-méthoxy-phénoxy-pyrimidine-4-yloxy]-éthoxy-carbonylamino]-1-méthyl-pyridinium,
Ester 2-[6-(4-tert-butyl-phénylsulfonylamino)-2-isopropyl-5-(2-méthoxy-phénoxy)-pyrimidine-4-yloxy]-éthylique de l'acide pyridine-4-ylcarbamique,
Ester 2-[6-(4-tert-butyl-phénylsulfonylamino)-5- (2-méthoxy-phénoxy)-2-propyl-pyrimidine-4-yloxy]-éthylique de l'acide pyridine-4-ylcarbamique,
Ester 2-[2-tert-butyl-6-(4-tert-butylphénylsulfonylamino)-5-(2-méthoxy-phénoxy)-pyrimidine-4-yloxy]-éthylique de l'acide pyridine-4-ylcarbamique,
Ester 2-[6-(4-tert-butyl-phénylsulfonylamino)-2-cyclopropyl-5-(2-méthoxy-phénoxy)-pyrimidine-4-yloxy]-éthylique de l'acide pyridine-4-ylcarbamique,
Ester 2-[6-(4-tert-butyl-phénylsulfonylamino)-5-(2-méthoxy-phénoxy)-2-thiophène-2-yl-pyrimidine-4-yloxy]-éthylique de l'acide pyridine-4-ylcarbamique,
Ester 2-[6-(4-tert-butyl-phénylsulfonylamino)-5-(2-méthoxy-phénoxy)-2-méthyl-pyrimidine-4-yloxy]-éthylique de l'acide pyridine-4-ylcarbamique,
Ester 2-[6-(4-tert-butyl-phénylsulfonylamino)-5-(2-méthoxy-phénoxy)-2,2'-bipyrimidine-4-yloxy]-éthylique de l'acide pyridine-4-ylcarbamique,
Ester 2-[6-(2-tert-butyl-phénylsulfonylamino)-5-(2-méthoxy-phénoxy)-2-morpholine-4-yl-pyrimidine-4-yloxy]-éthylique de l'acide pyridine-4-ylcarbamique,
Ester 2-[6-(2-tert-butyl-phénylsulfonylamino)-5-(2-méthoxy-phénoxy)-2-morpholine-4-yl-pyrimidine-4-yloxy]-éthylique de l'acide pyridine-3-ylcarbamique,
Ester 2-[6-(4-tert-butyl-phénylsulfonylamino)-5-(3,4-diméthoxy-phénoxy)-pyrimidine-4-yloxy]-éthylique de

l'acide pyridine-4-ylcarbamique,
Ester 2-[4-5-(2-méthoxy-phénoxy)-6-(2-pyridine-4-ylcarbamoyloxyéthoxy)-pyrimidine-4-ylsulfamoyl]-phénoxy]-éthylique de l'acide acétique,
Ester 2-[4-[5-(2-méthoxy-phénoxy)-6-(2-pyridine-4-ylcarbamoyloxyéthoxy)-pyrimidine-4-ylsulfamoyl]-phénoxy]-éthylique de l'acide acétique,
Ester 2-[6-[4-(2-hydroxy-éthoxy)-phénylsulfonylamino]-5-(2-méthoxy-phénoxy)-pyrimidine-4-yloxy]-éthylique de l'acide pyridine-4-ylcarbamique,
Ester 2-[6-[4-(2-hydroxy-éthoxy)-phénylsulfonylamino]-5-(2-méthoxy-phénoxy)-pyrimidine-4-yloxy]-éthylique de l'acide pyridine-3-ylcarbamique,
Ester 2-[6-[4-méthoxy-3-[2-morpholine-4-yl-2-oxo-éthoxy)-phénylsulfonylamino]-5-(2-méthoxy-phénoxy)-pyrimidine-4-yloxy]-éthylique de l'acide pyridine-4-ylcarbamique,
Ester 2-[5-(2-chloro-5-méthoxy-phénoxy)-6-[4-méthoxy-3-(2-morpholine-4-yl-2-oxo-éthoxy)-phényl sulfonylamino]-pyrimidine-4-yloxy]-éthylique de l'acide pyridine-4-ylcarbamique,
Ester 2-[5-(2-méthoxy-phénoxy)-6-[4-(2-morpholine-4-yl-2-oxo-éthoxy)-phénylsulfonylamino]-pyrimidine-4-yloxy]-éthylique de l'acide pyridine-4-ylcarbamique.

**12.** Les composés selon la revendication 8 :

Ester 2-[6-(1,3-benzodioxol-5-ylsulfonyl-amino)-5-(2-chloro-5-méthoxy-phénoxy)-pyrimidine-4-yloxy]-éthylique de l'acide 1-méthyl-pyrrole-2-carbamique,
Ester 2-[6-(4-tert-butyl-phénylsulfonylamino)-5-(2-chloro-5-méthoxy-phénoxy)-pyrimidine-4-yloxy]-éthylique de l'acide thiophène-3-ylcarbamique,
Ester 2-[6-(1,3-benzodioxol-5-ylsulfonylamino)-5-(2-chloro-5-méthoxy-phénoxy)-pyrimidine-4-yloxy]-éthylique de l'acide thiophène-3-ylcarbamique,
Ester 2-[6-(1,3-benzodioxol-5-ylsulfonylamino)-5-(2-chloro-5-méthoxy-phénoxy)-2-méthoxyméthyl--pyrimidine-4-yloxy]-éthylique de l'acide thiophène-3-ylcarbamique,
Ester 2-[6-(1,3-benzodioxol-5-ylsulfonylamino)-5-(2-chloro-5-méthoxy-phénoxy)-pyrimidine-4-yloxy]-éthylique de l'acide thiophène-2-ylcarbamique,
Ester 2-[6-(1,3-benzodioxol-5-ylsulfonylamino)-5-(2-chloro-5-méthoxy-phénoxy)-2-(2-méthoxy-éthyl)-pyrimidine-4-yloxy]-éthylique de l'acide thiophène-3-ylcarbamique,
Ester 2-[6-(4-tert-butylphénylsulfonylamino)-5-(2-chloro-5-méthoxy-phénoxy)-pyrimidine-4-yloxy]-éthylique de l'acide pyrazine-2-ylcarbamique,
Ester 2-[6-(4-tert-butyl-phénylsulfonylamino)-5-(3-méthoxy-phénoxy)-pyrimidine-4-yloxy]-éthylique de l'acide quinoléine-2-ylcarbamique,
Ester 2-[6-(1,3-benzodioxol-5-ylsulfonylamino)-5-(2-chloro-5-méthoxy-phénoxy)-pyrimidine-4-yloxy]-éthylique de l'acide furanne-3-ylcarbamique,
Ester 2-[6-(4-tert-butylphénylsulfonylamino)-5-(2-méthoxy-phénoxy)-2-morpholine-4-yl-pyrimidine-4-yloxy]-éthylique de l'acide furanne-2-ylcarbamique,
Ester 2-[6-(4-tert-butyl-phénylsulfonylamino)-5-(2-méthoxy-phénoxy)-2-morpholine-4-yl-pyrimidine-4-yloxy]-éthylique de l'acide méthyl-isoxazole-5-ylcarbamique,
Ester 2-[5-(2-méthoxy-phénoxy)-6-(4-méthylsulfanyl-phénylsulfonylamino)-2,2'-bipyrimidine-4-yloxy-éthylique de l'acide méthyl-isoxazole-5-ylcarbamique,
Ester 2-[6-[4-méthoxy-3-[2-morpholine-4-yl-2-oxo-éthoxy)-phénylsulfonylamino]5-(2-méthoxy-phénoxy)-pyrimidine-4-yloxy]-éthylique de l'acide pyrazine-2-ylcarbamique,
Ester 2-[5-(2-chloro-5-méthoxy-phénoxy)-6-[4-méthoxy-3-(2-morpholine-4-yl-2-oxo-éthoxy)-phénylsulfonylamino]-pyrimidine-4-yloxy]-éthylique de l'acide pyrazine-2-ylcarbamique,
Ester 2-[5-(2-chloro-5-méthoxy-phénoxy)-6-[4-(2-morpholine-4-yl-2-oxo-éthoxy]-phénylsulfonylamino]-pyrimidine-4-yloxy]-éthylique de l'acide pyrazine-2-ylcarbamique,
Ester 2-[5-(2-chloro-5-méthoxy-phénoxy)-6-[4-méthoxy-3-(3-morpholine-4-yl-3-oxo-propyl]-phénylsulfonylamino]-pyrimidine-4-yloxy]-éthylique de l'acide pyrimidine-2-ylcarbamique.

**13.** Composés selon la revendication 7, dans lesquels $R^{10}$ est un groupe aryle ou cycloalkyle inférieur, et $R^{11}$ est un atome d'hydrogène, et "inférieur" désigne des radicaux ayant de 1 à 7 atomes de carbone.

**14.** Les composés selon la revendication 13 :

Ester 2-[6-(4-tert-butyl-phénylsulfonylamino)-5-(2-chloro-5-méthoxy-phénoxy)-pyrimidine-4-yloxy]-éthylique de l'acide benzodioxol-5-ylcarbamique,

Ester 2-[6-(4-tert-butylphénylsulfonylamino)-5-(2-chloro-5-méthoxy-phénoxy)-pyrimidine-4-yloxy]-éthylique de l'acide 3-fluorophénylcarbamique,
Ester 2-[(6-(4-tert-butyl-phénylsulfonylamino)-5-(2-chloro-5-méthoxy-phénoxy)-pyrimidine-4-yloxy]-éthylique de l'acide 2-fluorophénylcarbamique,
Ester 2-[6-(4-tert-butyl-phénylsulfonylamino)-5-(2-chloro-5-méthoxy-phénoxy)-pyrimidine-4-yloxy]-éthylique de l'acide phénylcarbamique,
Ester 2-[6-(4-tert-butyl-phénylsulfonylamino)-5-(2-chloro-5-méthoxy-phénoxy)-pyrimidine-4-yloxy]-éthylique de l'acide 4-chloro-phénylcarbamique,
Ester 2-[6-(4-tert-butyl-phénylsulfonylamino)-5-(2-chloro-5-méthoxy-phénoxy)-pyrimidine-4-yloxy]-éthylique de l'acide 3-méthoxy-carbamique,
Ester 2-[6-(4-tert-butyl-phénylsulfonylamino)-5-(2-chloro-5-méthoxy-phénoxy)-pyrimidine-4-yloxy]-éthylique de l'acide 4-trifluorométhyl-phénylcarbamique,
Ester 2-[2-[6-(4-tert-butyl-phénylsulfonylamino)-5-(2-chloro-5-méthoxy-phénoxy)-pyrimidine-4-yloxy]-éthoxy-carbonylamino]-méthylique de l'acide benzoïque,
Ester 2-(6-(4-tert-butyl-phénylsulfonylamino)-5-(2-chloro-5-méthoxy-phénoxy)-pyrimidine-4-yloxy]-éthylique de l'acide 3-tolylcarbamique,
Ester 2-[6-(4-tert-butylphénylsulfonylamino)-5-(2-chloro-5-méthoxy-phénoxy)-pyrimidine-4-yloxy]-éthylique de l'acide 2-méthoxy-phénoxycarbamique,
Ester 2-[2-[((6-tert-butyl-phénylsulfonylamino)-5-(2-chloro-5-méthoxy-phénoxy)-pyrimidine-4-yloxy]-éthoxy-carbonylamino]-phénylique de l'acide acétique,
Ester 2-[6-(4-tert-butylphénylsulfonylamino)-5-(2-chloro-5-méthoxy-phénoxy)-pyrimidine-4-yloxy]-éthylique de l'acide 2-hydroxy-phénylcarbamique,
Ester 2-[6-(4-tert-butylphénylsulfonylamino)-5-(2-chloro-5-méthoxy-phénoxy)-pyrimidine-4-yloxy]-éthylique de l'acide benzylcarbamique,
Ester 2-[6-(4-tert-butylphénylsulfonylamino)-5-(3-méthoxy-phénoxy)-pyrimidine-4-yloxy]-éthylique de l'acide phénylcarbamique,
Ester 2-[6-(4-tert-butyl-phénylsulfonylamino)-5-(3-méthoxy-phénoxy)-pyrimidine-4-yloxy]-éthylique de l'acide (R)-1-phényl-éthylcarbamique,
Ester 2-[6-(4-tert-butyl-phénylsulfonylamino)-5-(3-méthoxy-phénoxy)-pyrimidine-4-yloxy]-éthylique de l'acide cyclohexylcarbamique,
Ester 2-[4-[5-(2-méthoxy-phénoxy)-6-(2-phénylcarbamoyloxyéthoxy)-pyrimidine-4-ylsulfamoyl]-phénoxy]-éthylique de l'acide acétique,
Ester 2-[4-[6-(2-fluoro-phénylcarbamoyloxyéthoxy)-éthoxy]-5-(2-méthoxy-phénoxy)-pyrimidine-4-ylsulfamoyl]-phénoxy]-éthylique de l'acide acétique,
Ester 2-[6-[4-(2-hydroxy-éthoxy)-phénylsulfonylamino]-5-(2-méthoxy-phénoxy)-pyrimidine-4-yloxy]-éthylique de l'acide phénylcarbamique,
Ester 2-[6-[4-(2-hydroxy-éthoxy)-phénylsulfonylamino]-5-(2-méthoxy-phénoxy)-pyrimidine-4-yloxy]-éthylique de l'acide 2-fluoro-phénylcarbamique,
Ester 2-[6-[4-méthoxy-3-(2-morpholine-4-yl-2-oxo-éthoxy)-phénylsulfonylamino]-5-(2-méthoxy-phénoxy)-pyrimidine-4-yloxy]-éthylique de l'acide 2-fluoro-phénylcarbamique,
Ester 2-[4-[5-(2-chloro-5-méthoxy-phénoxy)-6-[2-(2-fluoro-phénylcarbamoyloxy)-éthoxy]-pyrimidine-4-ylsulfamoyl]-phénoxy]-éthylique de l'acide acétique,
Ester 2-[4-[5-(2-chloro-5-méthoxy-phénoxy)-6-[4-(2-hydroxy-éthoxy)-phénylsulfonylamino]-pyrimidine-4-yloxy]-éthylique de l'acide 2-fluoro-phénylcarbamique,
Ester 2-[5-(2-chloro-5-méthoxy-phénoxy)-6-[4-méthoxy-3-(2-morpholine-4-yl-2-oxo-éthoxy)-phénylsulfonylamino]-pyrimidine-4-yloxy]-éthylique de l'acide 2-fluoro-phénylcarbamique,
Ester 2-[6-[4-méthoxy-3-(2-morpholine-4-yl-2-oxo-éthoxy)-phénylsulfonylamino]-5-(3-méthoxy-phénoxy)- pyrimidine-4-yloxy]-éthylique de l'acide phénylcarbamique,
Ester éthylique de l'acide [5-[5-(2-chloro-5-méthoxy-phénoxy)-6-[2-(2-fluorophénylcarbamoyloxy)-éthoxy]-pyrimidine-4-ylsulfamoyl]-2-méthoxy-phénoxy]-acétique,
Acide [5-[5-(2-chloro-5-méthoxy-phénoxy)-6-[2-(2-fluoro-phénylcarbamoyloxy)-éthoxy]-pyrimidine-4-ylsulfamoyl]-2-méthoxy-phénoxy]-acétique,
Ester 2-[6-[4-méthoxy-3-(2-oxo-2-pipéridine-1-yl-éthoxy)-phénylsulfonylamino]-5-(2-méthoxy-phénoxy)-pyridine-4-yloxy]-éthylique de l'acide 2-fluoro-phénylcarbamique.

**15.** Les composés selon la revendication 7 :

Ester 2-[6-(4-tert-butyl-phénylsulfonylamino)-5-(2-chloro-5-méthoxy-phénoxy)-pyrmidine-4-yloxy]-éthylique de

l'acide isopropylcarbamique,
Ester 2-[6-(4-tert-butyl-phénylsulfonylamino)-5-(2-chloro-5-méthoxy-phénoxy)-pyrmidine-4-yloxy]-éthylique de l'acide éthylcarbamique,
Ester éthylique de l'acide [2-[6-(4-tert-butyl-phénylsulfonylamino)-5-(3-méthoxy-phénoxy)-pyrimidine-4-yloxy]-éthoxycarbonylamino]-acétique,
Acide 2-[6-(4-tert-butyl-phénylsulfonylamino)-5-(3-méthoxy-phénoxy)-pyrimidine-4-yloxy]-éthoxycarbonylamino]-acétique,
Ester 2-[6-(4-tert-butyl-phénylsulfonylamino)-5-(3-méthoxy-phénoxy)-pyrimidine-4-yloxy]-éthylique de l'acide 2-hydroxy-éthylcarbamique,
Ester 2-[6-(4-tert-butyl-phénylsulfonylamino)-5-(3-méthoxy-phénoxy)-pyrimidine-4-yloxy]-éthylique de l'acide morpholine-4-carbonique,
Ester 2-[6-(4-tert-butyl-phénylsulfonylamino)-5-(3-méthoxy-phénoxy)-pyrimidine-4-yloxy]-éthylique de l'acide morpholine-4-yl-2-oxo-éthylcarbamique,
Ester 2-[6-(4-tert-butyl-phénylsulfonylamino)-5-(3-méthoxy-phénoxy)-pyrimidine-4-yloxy]-éthylique de l'acide 2-oxo-2-pyrrolidine-1-yl-éthylcarbamique,
Ester 2-[6-(4-tert-butyl-phénylsulfonylamino)-5-(3-méthoxy-phénoxy)-pyrimidine-4-yloxy]-éthylique de l'acide phénylcarbamoylméthylcarbamique,
Ester éthylique de l'acide 2-[2-[6-(4-tert-phénylsulfonylamino)-5-(3-méthoxy-phénoxy)-pyrimidine-4-yloxy]-éthoxycarbonylamino]-4-méthyl-pentanoïque.

**16.** Composés selon les revendications 1 à 6, dans lesquels Y est $-NHC(O)NR^{10}R^{11}$.

**17.** Les composés selon la revendication 16 :

4-tert-butyl-N-[5-(2-chloro-5-méthoxy-phénoxy)-6-[2-(3-phényl-uréido)-éthoxy]-pyrimidine-4-yl]-benzènesulfonamide,
4-tert-butyl-N-[5-(2-chloro-5-méthoxy-phénoxy)-6-[2-[3-(2fluoro-phényl)-uréido]-éthoxy]-pyrimidine-4-yl]-benzènesulfonamide,
4-tert-butyl-N-[5-(2-chloro-5-méthoxy-phénoxy)-6-[2-(3-pyridine-2-yl-uréido)-éthoxy]-pyrimidine-4-yl]-benzènesulfonamide,
4-tert-butyl-N-[5-(2-chloro-5-méthoxy-phénoxy)-6-[2-(3-pyridine-4-yl-uréido)-éthoxy]-pyrimidine-4-yl]-benzènesulfonamide,
4-tert-butyl-N-[5-(2-chloro-5-méthoxy-phénoxy)-6-[2-(3-pyridine-3-yl-uréido)-éthoxy]-pyrimidine-4-yl]-benzènesulfonamide,
4-tert-butyl-N-[5-(2-chloro-5-méthoxy-phénoxy)-6-[2-(3-1-oxy-pyridine-4-yl)-uréido)-éthoxy]-pyrimidine-4-yl]-benzènesulfonamide,
4-tert-butyl-N-[5-(2-méthoxy-phénoxy)-6-[2-(3-pyridine-2-yl-uréido)-éthoxy]-pyrimidine-4-yl]-benzènesulfonamide.

**18.** Composés selon les revendications 1 à 6, dans lesquels Y est $-OC(O)COR^{10}$.

**19.** Les composés selon la revendication 18 :

Carbonate de 2-[6-(1,3-benzodioxol-5-ylsulfonylamino)-5-(3,5-diméthoxy-phénoxy)-pyrimidine-4-yloxy]-éthyle et de pyridine-3-ylméthyle,
Carbonate de 2-[6-(1,3-benzodioxol-5-sulfonylamino)-5-(2-chloro-5-méthoxy-phénoxy)-pyrimidine-4-yloxy]-éthyle et de pyridine-2-ylméthyle,
Carbonate de 2-[6-(1,3-benzodioxol-5-ylsulfonylamino)-5-(2-chloro-5-méthoxy-phénoxy)-pyrimidine-4-yloxy]-éthyle et de pyridine-2-ylméthyle,
Carbonate de 2-[6-(4-tert-butyl-benzènesulfonylamino)-5-(2-chloro-5-méthoxy-phénoxy)-pyrimidine-4-yloxy]-éthyle et de pyridine-2-ylméthyle,
Carbonate de 2-[6-(1,3-benzodioxol-5-ylsulfonylamino)-5-(2-chloro-5-méthoxy-phénoxy)-pyrimidine-4-yloxy]-éthyle et de furanne-3-ylméthyle,
Carbonate de 2-[6-(4-tert-butyl-phénylsulfonylamino)-5-(2-chloro-5-méthoxy-phénoxy)-2-méthyl-pyrimidine-4-yloxy]-éthyle et de pyridine-3-ylméthyle,
Carbonate de 2-[6-(4-tert-butyl-phénylsulfonylamino)-5-(2-chloro-5-méthoxy-phénoxy)-2-méthyl-pyrimidine-4-yloxy]-éthyle et de pyridine-2-ylméthyle,
Carbonate de 2-[6-(4-tert-butyl-phénylsulfonylamino)-5-(2-chloro-5-méthoxy-phénoxy)-2-méthyl-pyrimidine-4-yloxy]-éthyle et de pyrimidine-4-ylméthyle,
Carbonate de 2-[6-(4-tert-butyl-phénylsulfonylamino)-5-(2-méthoxy-phénylsulfanyl)-2,2'-bipyrimidine-4-yloxy]-éthyle et de pyridine-3-ylméthyle,
Carbonate de 2-[6-(4-tert-butyl-phénylsulfonylamino)-5-(2-méthoxy-phénylsulfanyl)-2-méthyl-pyrimidine-4-yloxy]-éthyle et de pyridine-3-ylméthyle.

**20.** Composés selon la revendication 1, dans lesquels n = 1 et Z = -S-.

**21.** Les composés selon la revendication 20 :

Ester 2-[6-(4-tert-butyl-phénylsulfonylamino)-5-(2-méthoxy-phénylsulfanyl)-pyrimidine-4-yloxy]-éthylique de l'acide pyridine-2-ylcarbamique,
Ester 2-[6-tert-butyl-phénylsulfonylamino)-5-(2-méthoxy-phénylsulfanyl)-2,2'-pyrimidine-4-yloxy]-éthylique de l'acide pyridine-3-ylcarbamique,
Ester 2-[6-(4-tert-butyl-phénylsulfonylamino)-5-(2-méthoxy-phénylsulfanyl)-2-méthyl-pyrimidine-4-yloxy]-éthylique de l'acide pyridine-4-ylcarbamique.

**22.** Composés selon les revendications 1 à 21, pour utilisation en tant que médicaments.

**23.** Préparations pharmaceutiques contenant un composé selon les revendications 1 à 21 et des excipients et adjuvants usuels.

**24.** Utilisation de composés selon les revendications 1 à 21 en tant que principes actifs lors de la fabrication de médicaments destinés au traitement de maladies associées à l'activité d'endothéline, en particulier les maladies circulatoires telles que l'hypertonie, l'ischémie, les angiospasmes et l'angine de poitrine.

**25.** Procédé de préparation de composés selon les revendications 1 à 21, caractérisé en ce qu'on fait réagir un composé de formule

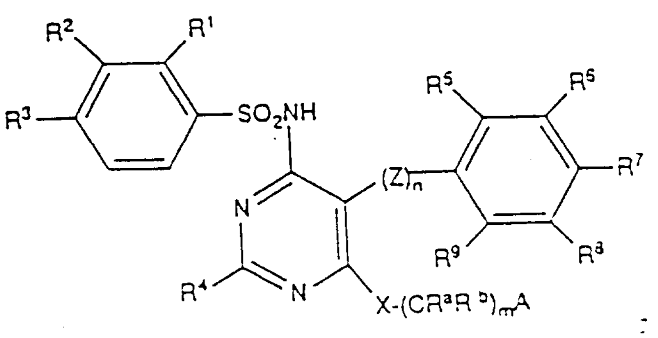

II

dans laquelle $R^1$-$R^9$, $R^a$, $R^b$, X, Z, m et n ont les significations données dans la revendication 1, et A est un groupe hydroxy ou amino,

a) avec un isocyanate de formule $R^{10}NCO$ et un chlo-rure de carbamoyle de formule $(R^{10}R^{11})NCOCl$, où $R^{10}$ et $R^{11}$ ont les significations données dans la revendication 1, ou bien
b) avec du phosgène, puis avec un alcool de formule $R^{10}OH$; ou encore avec un ester de l'acide chloroformique de formule $R^{10}OC(O)Cl$,

et éventuellement en modifie les substituants pré-sents dans le composé ainsi obtenu de formule I et, si on le souhaite, on convertit un composé de formule I en un sel acceptable d'un point de vue pharmaceutique.